# EUROPÄISCHE PATENTSCHRIFT

(11) **EP 0 627 411 B1**
(45) Veröffentlichungstag und Bekanntmachung des Hinweises auf die Patenterteilung: **13.08.1997**
(21) Anmeldenummer: 94108065.7
(22) Anmeldetag: 26.05.1994
(51) Int. Cl.: C07C 251/88, A01N 37/50, A01N 33/26

(54) **Azin-substituierte Phenylessigsäurederivate und diese enthaltende Fungizide**
Azine-substituted phenylacetic acid derivatives and fungicides containing them
Dérivés azine de l'acide phénylacétique et fongicides les contenant

(30) Priorität: 03.06.1993 DE 4318397
(43) Veröffentlichungstag der Anmeldung: 07.12.1994
(73) Patentinhaber: BASF Aktiengesellschaft, 67063 Ludwigshafen (DE)
(72) Erfinder: Doetzer, Reinhard, Dr., D-69469 Weinheim (DE); Sauter, Hubert, Dr., D-68167 Mannheim (DE); Bayer, Herbert, Dr., D-68159 Mannheim (DE); Roehl, Franz, Dr., D-67105 Schifferstadt (DE); Lorenz, Gisela, Dr., D-67434 Neustadt (DE); Ammermann, Eberhard, Dr., D-64646 Heppenheim (DE); Wingert, Horst, Dr., D-68159 Mannheim (DE)

(56) Entgegenhaltungen:
- EP-A- 0 463 488
- EP-A- 0 499 823
- US-A- 3 829 492

## Beschreibung

Die vorliegende Erfindung betrifft Verbindungen der Formel I, 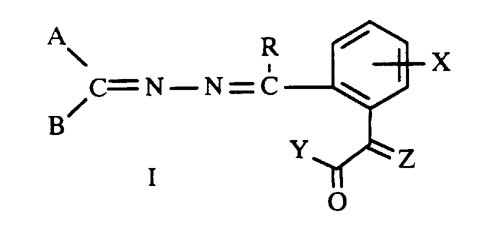 in der die Substituenten die folgende Bedeutung haben
- X: Wasserstoff, Cyano, Nitro, Trifluormethyl, Halogen, C₁-C₆-Alkyl oder C₁-C₆-Alkoxy;
- Y: Methoxy, Amino, Methylamino oder Dimethylamino;
- Z: CHCH₃, CHOCH₃ oder NOCH₃;
- R: Wasserstoff oder C₁-C₆-Alkyl;
- A: Wasserstoff, C₁-C₆-Alkyl, C₁-C₆-Haloalkyl oder C₃-C₇-Cycloalkyl:
- B: gegebenenfalls substituiertes C₁-C₆-Alkyl, C₂-C₆-Alkenyl, C₂-C₆-Alkinyl, C₃-C₇-Cycloalkyl, C₅-C₇-Cycloalkenyl;
gegebenenfalls substituiertes gesättigtes oder partiell ungesättigtes Heterocyclyl;
gegebenenfalls substituiertes, gegebenenfalls kondensiertes Aryl;
gegebenenfalls substituiertes, gegebenenfalls benzo- oder heterokondensiertes 5- oder 6-gliedriges Heteroaryl;
gegebenenfalls substituiertes Aryl-C₁-C₆-Alkyl oder Heteroaryl-C₁-C₆-Alkyl, oder
C(=O)W;
wobei
- W: Wasserstoff,
gegebenenfalls substituiertes C₁-C₆-Alkyl,
C₁-C₆-Alkoxy, C₁-C₆-Alkylthio, C₁-C₆-Alkylamino oder Di-C₁-C₆-alkylamino,
gegebenenfalls substituiertes Aryl, Heteroaryl, Aryloxy, Arylthio, Arylamino, Aryl-N-(C₁-C₆-Alkyl)-amino, Heteroaryloxy, Heteroarylthio, Heteroarylamino oder Heteroaryl-N-(C₁-C₆-Alkyl)amino;
oder
- A und B: zusammen mit dem C-Atom, dessen Substituenten sie sind, einen gegebenenfalls substituierten, gegebenenfalls annellierten, gesättigten oder ungesättigten 3- bis 7-gliedrigen isocyclischen oder 5-bis 7-gliedrigen heterocyclischen Ring, der auch chinonoid sein kann.

Außerdem betrifft die Erfindung Verfahren zur Herstellung dieser Verbindungen, sie enthaltende Mittel, sowie ihre Verwendung zur Herstellung derartiger Mittel und zur Bekämpfung von Schädlingen und Schadpilzen.

Aus EP-A-499823 ist die Verwendung von hydrazon-substituierten Phenylessigsäurederivaten als agrochemische Fungizide bekannt. Ihre Wirkung ist jedoch unbefriedigend.

Der vorliegenden Erfindung lagen neue Verbindungen mit verbesserten Eigenschaften als Aufgabe zugrunde.

Demgemäß wurden die eingangs definierten Verbindungen, Verfahren zu ihrer Herstellung, sie enthaltende Mittel und ihre Verwendung gefunden.

Die Synthese der Wirkstoffe kann nach allgemeinen Methoden zur Darstellung unsymmetrischer Azine erfolgen, wie sie z.B. in Houben/Weyl, "Methoden der präparativen organischen Chemie Bd. 10/2, S.104-111 und Bd. E 14 b, Teil 1, S. 655-673, beschrieben sind.

So können beispielsweise (s. Schema 1) Azine der Formel **I** durch Umsetzung von zwei Carbonylverbindungen **II** und **III** mit Hydrazin, einem seiner Säureadditionssalze oder seinem Hydrat in einem geeigneten Lösungsmittel und gegebenenfalls in Anwesenheit einer katalytisch aktiven Verbindung, z.B. Essigsäure, dargestellt werden und das unsymmetrische Azin **I** von den mit entstehenden symmetrischen Azinen **VI** und **VII** durch Anwendung einer geeigneten Trennmethode wie z.B. Säulenchromatographie, fraktionierte Kristallisation oder Destillation isoliert werden. Die Verbindungen **II** sind nach allgemein bekannten Methoden der organischen Synthese zugänglich bzw. kommerziell erhältlich, die Herstellung von Verbindungen **III** ist in EP 499823 und dort zitierter Literatur beschrieben.

Ebenso ist es möglich, **II** und eine Carbonylverbindung **VIII** mit Hydrazin, einem seiner Säureadditionssalze oder seinem Hydrat zur Reaktion zu bringen, wobei ein unsymmetrisches Azin **IX** im Gemisch mit den symmetrischen Azinen **VI** und **X** entsteht und nach den üblichen Trennmethoden isoliert werden kann. Die Variable V in den Formeln **VIII** und **IX** bedeutet eine Gruppe, die nach Standardmethoden der organischen Chemie in die Gruppe Y-C(=O)-C=Z überführt werden kann, beispielsweise wie in EP 463488, S.7-8 für ähnliche Verbindungen beschrieben. Es kann vorteilhaft sein, zuerst die Azine **IX** aufzubauen und danach durch Umwandlung der Gruppe V in die Gruppierung Y-C(=O)-C=Z den Wirkstoff **I** darzustellen. Die Gruppe V wird vorteilhaft so ausgewählt, daß sie unter den Bedingungen der Azinsynthese unverändert bleibt. Auch im Edukt der Formel **II** kann es von Vorteil sein, einen funktionellen Substituenten in der Gruppe B geschützt einzusetzen und nach der Azinbildung zu entschützen. Die Herstellung der Carbonylverbindungen **VIII** kann nach den für **III** genannten oder allgemein bekannten Methoden der organischen Synthese erfolgen.

Weiterhin können Azine der Formel **I** oder **IX** dargestellt werden, indem man nach bekannten Methoden eine Carbonylverbindung **II** in ein Hydrazon **IV** überführt und dieses in einem geeigneten Lösungsmittel und gegebenenfalls in Gegenwart einer katalytisch wirksamen Spezies mit einer Carbonylverbindung **III** oder **VIII** umsetzt [siehe z.B. Liebigs Ann. Chem. **640** (1961),37]. Auch hierbei können mit dem unsymmetrischen Azin **I** bzw. **IX** als Nebenprodukte die symmetrischen Azine **VI** und **VII** bzw. **X** entstehen, die abgetrennt werden müssen. Anstelle der Carbonylverbindungen kann man auch deren (nach allgemein bekannten Methoden herstellbare) Schiffsche Basen einsetzen [Liebigs Ann.Chem. **657**(1962),39]; dies kann in Bezug auf die Ausbeute und Selektivität der Reaktion von Vorteil sein. **IX** kann nach Standardmethoden in **I** überführt werden (s. oben).

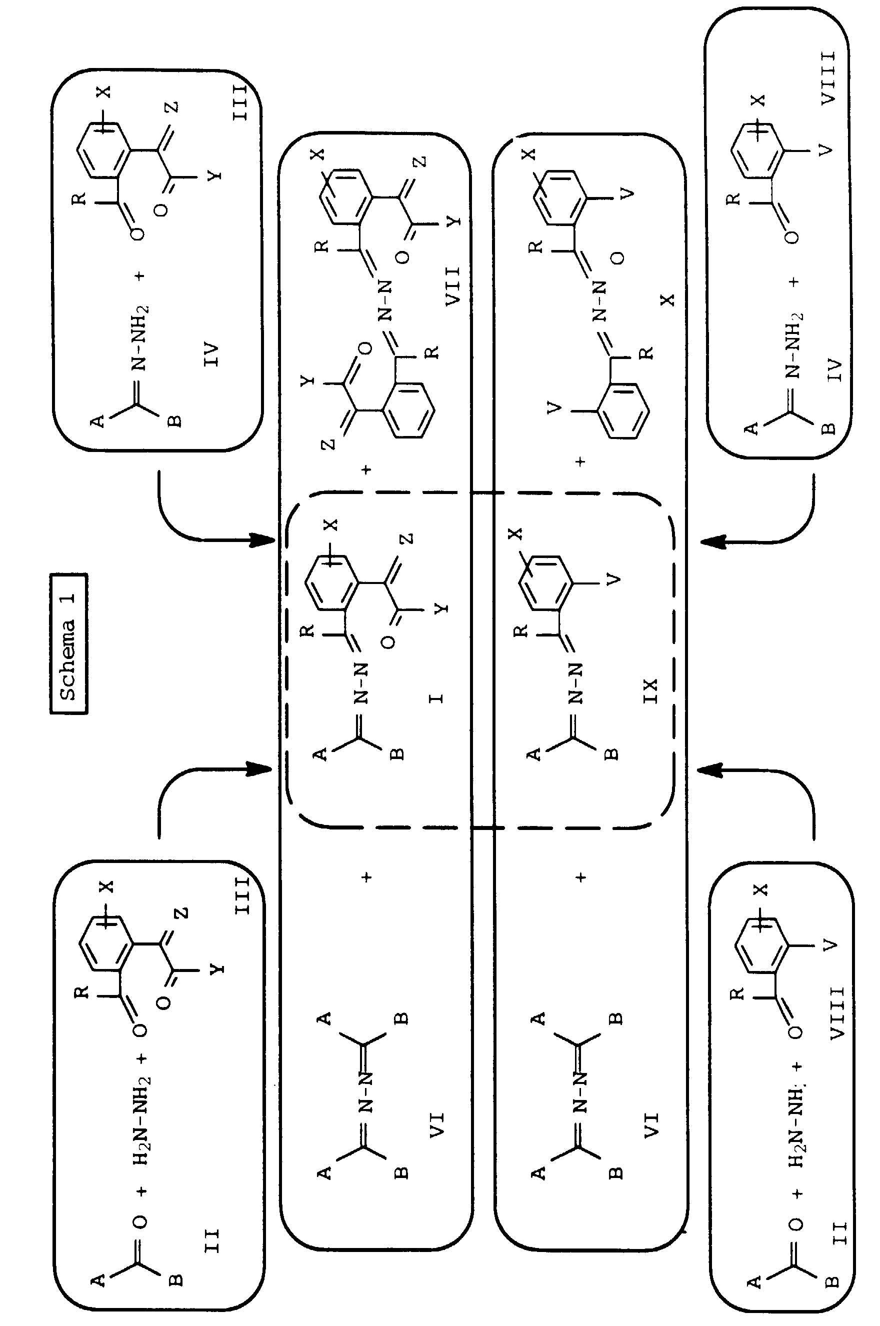

Es ist auch möglich [Sulfur Lett. **9**(1989),227], Hydrazone **IV** durch Thermolyse von o-Ethylsulfamoylbenzoesäurehydrazoniden **XII** (zugänglich aus N-Ethylsaccharin **XI**, Herstellung s. z.B. J.Pharm.Soc.Jap. **75**(1955),153) in Gegenwart einer Carbonylverbindung **III** bzw. **VIII** zu erzeugen und in situ zum Azin **I** bzw. **IX** umzusetzen (Schema 2); die Methode kann für die selektive Darstellung der unsymmetrischen Azine vorteilhaft sein.

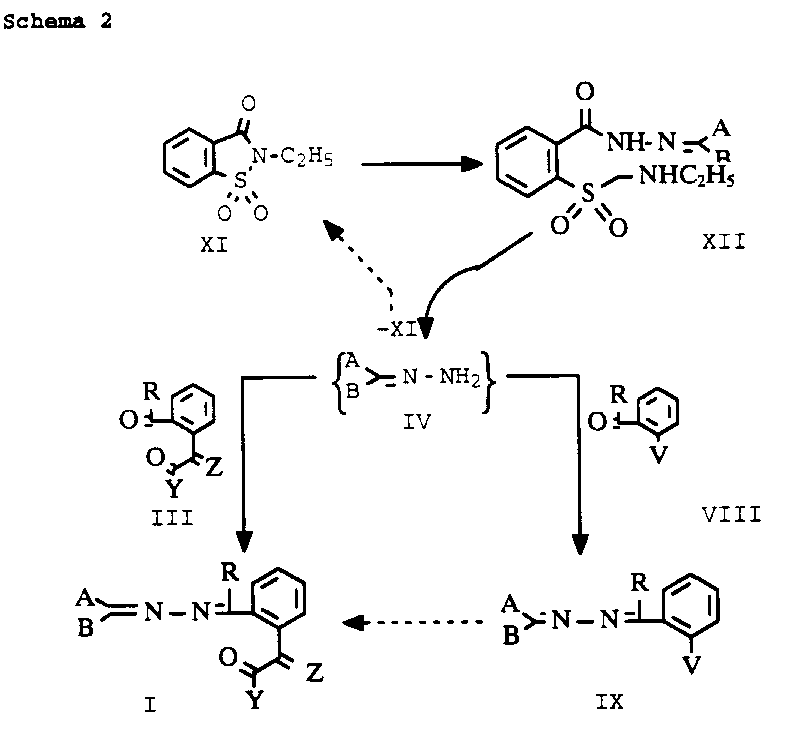

Ferner können die Verbindungen der Formel **I** oder **IX** durch Umsetzung von Carbonylverbindungen **III** bzw. **VIII**, bevorzugt Aldehyden (R = H), in einer aza-analogen Wittig- [Chem.Ber. **94**(1961),2477] bzw. Horner-Emmons-Reaktion [Synthesis **1986**, 298] mit Phosphazinen **XIII** bzw. Metallsalzen der Phosphonohydrazonide **XIV** dargestellt werden (Schema 3). Die Synthese der Edukte **XIII** und **XIV** ist in obiger Literatur beschrieben bzw. zitiert.

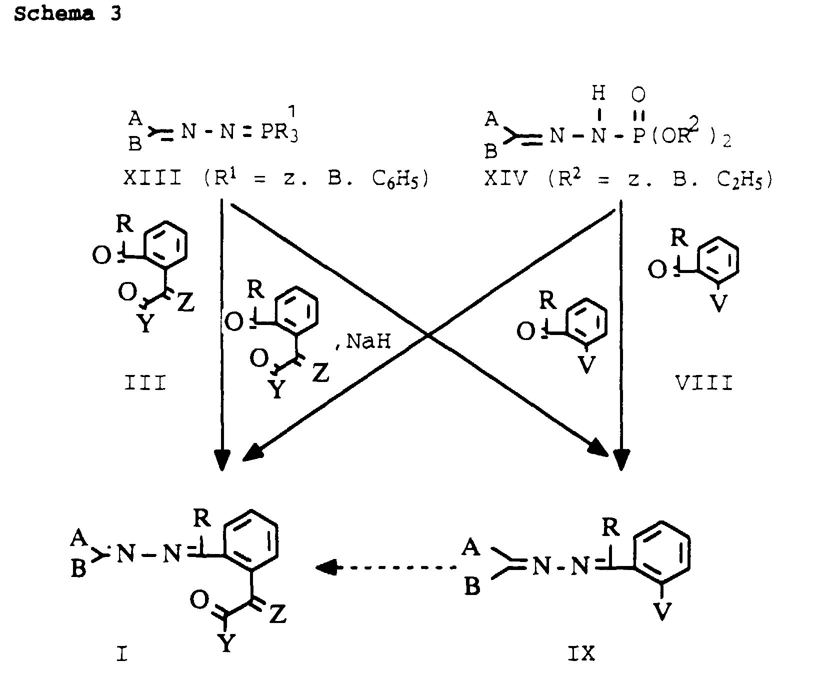

Azine **XVI**, entsprechend Verbindungen der Formel **I**,in denen Z eine Gruppe NOCH₃ und Y eine Gruppe NHCH₃ bedeutet, können dargestellt werden, indem man entweder eine Carbonylverbindung **II** und Hydrazin, eines seiner Säureadditionssalze oder sein Hydrat oder anstelle dieser Eduktkombination ein Hydrazon **IV** mit einem Aldehyd- bzw. Ketonäquivalent **V** umsetzt (Schema 4). Die cyclischen Halbaminale **V** sind durch Behandeln der Carbonylverbindungen **XV** (entsprechend Verbindungen, in denen R Alkyl, bevorzugt Methyl bedeutet) mit Methylamin in einem geeigneten Lösungsmittel, z.B. Wasser, Methanol oder Tetrahydrofuran bzw. einem Gemisch solcher Lösungsmittel, zugänglich. Ebenso kann **V** nach den in den Schemata 2 und 3 beschriebenen Methoden anstelle von **III** mit **XII**, **XIII** oder **XIV** zu Verbindungen der Formel **XVI** umgesetzt werden.

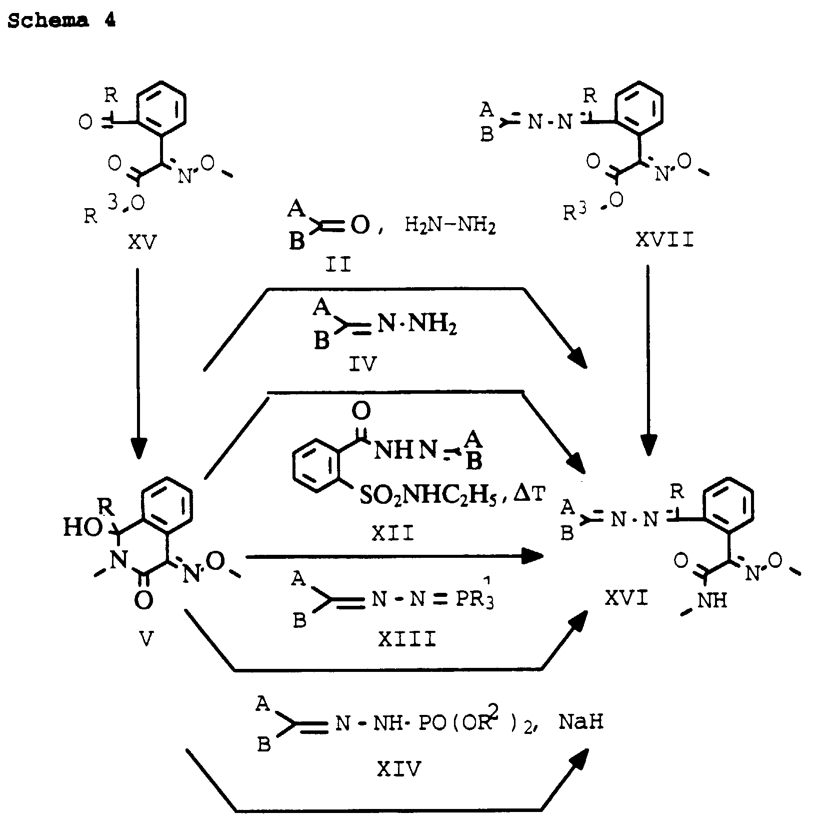

Verbindungen der Formel **XVI** können auch dargestellt werden, indem man Azine der Formel **XVII** (entsprechend Formel **I**, mit Z = NOCH₃ und Y = OR³, wobei R³ kurzkettiges Alkyl, bevorzugt Methyl bedeutet) wie oben beschrieben mit Methylamin umsetzt. Die Edukte **XVII** sind z.B. nach den obigen Methoden (siehe Schemata 1-3) zugänglich.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Jod;
Alkyl: gesättigte, geradkettige oder verzweigte Kohlenwasserstoffreste mit 1 bis 6 Kohlenstoffatomen, wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
Alkoxy: geradkettige oder verzweigte Alkylgruppen mit 1 bis 6 Kohlenstoffatomen (wie vorstehend genannt), welche über ein Sauerstoffatom (-O-) an das Gerüst gebunden sind;
Alkylthio: geradkettige oder verzweigte Alkylgruppen mit 1 bis 6 Kohlenstoffatomen (wie vorstehend genannt), welche über ein Schwefelatom (-S-) an das Gerüst gebunden sind;
Alkylamino: eine geradkettige oder verzweigte Alkylgruppe mit 1 bis 6 Kohlenstoffatomen (wie vorstehend genannt), welche über eine Aminogruppe (-NH-) an das Gerüst gebunden ist;
Dialkylamino: eine Aminogruppe, welche zwei voneinander unabhängige, geradkettige oder verzweigte Alkylgruppen mit jeweils 1 bis 6 Kohlenstoffatomen (wie vorstehend genannt) trägt;
Halogenalkyl: geradkettige oder verzweigte Alkylgruppen mit 1 bis 6, vorzugesweise 1 bis 4, insbesondere 1 bis 2 Kohlenstoffatomen (wie vorstehend genannt), wobei diese in Gruppen teilweise oder vollständig die Wasserstoffatome durch Halogenatome wie vorstehend genannt ersetzt sein können, z.B. Fluormethyl, Difluormethyl, Trifluormethyl, 1-Fluorethyl, 2-Fluorethyl, 1,1-Difluorethyl, 1,2-Difluorethyl, 2,2-Difluorethyl, 2,2,2-Trifluorethyl, 1,1,2,2-Tetrafluorethyl, Pentafluorethyl, Fluorpropyl, Fluorbutyl, Fluorpentyl, Fluorhexyl, Heptafluorpropyl, Nonafluorbutyl, Chlormethyl, 2,2,2-Triethyl, Trichlormethyl, 1-Chlorethyl, 2-Chlorethyl, 2,2,2-Trichlorethyl, Chlorpropyl, Chlorbutyl, Chlorpentyl, Chlorhexyl, Brommethyl, Bromethyl, Dibromethyl und Iodmethyl;
Alkenyl: ungesättigte, geradkettige oder verzweigte Kohlenwasserstoffreste mit 2 bis 6 Kohlenstoffatomen und einer Doppelbindung in einer beliebigen Position, z.B. Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1pentenyl, 1-Methyl-2-pentenyl, 2-Methyl-2-pentenyl, 3-Methyl-2pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4pentenyl, 2-Methyl-4-pentenyl, 3-Methyl-4-pentenyl, 4-Methyl-4pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
Alkinyl: geradkettige oder verzweigte Kohlenwasserstoffgruppen mit 2 bis 6 Kohlenstoffatomen und einer Dreifachbindung in einer beliebigen Position, z.B. C2-C6-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
Cycloalkyl: monocyclische oder bicyclische Alkylgruppen mit 3 bis 7 Kohlenstoffringgliedern, z.B. Cyclopropyl, Cyclobutyl, Cyclopentyl, Cyclohexyl, Cycloheptyl und Norbornyl;
Cycloalkenyl: monocyclische oder bicyclische Alkylgruppen mit 5 bis 7 Kohlenstoffringgliedern, z.B. Cyclopentenyl, Cyclohexenyl, Cycloheptenyl und Norbornenyl;
Heterocyclyl: gesättigte oder teiweise ungesättigte Ringsysteme, die neben Kohlenstoffringgliedern auch ein bis drei Heteroatome aus der Gruppe Sauerstoff, Schwefel und Stickstoff enthalten können, z.B. Oxiranyl, Oxetanyl, Tetrahydrofur-2-yl, Tetrahydrofur-3-yl, Tetrahydrothien-2-yl, Tetrahydrothien-3-yl, Pyrrolidin-2-yl, Pyrrolidin-3-yl, Isoxazolidin-3-yl, Isoxazolidin-4-yl, Isoxazolidin-5-yl, Isothiazolidin-3-yl, Isothiazolidin-4-yl, Isothiazolidin-5-yl, Pyrazolidin-3-yl, Pyrazolidin-4-yl, Pyrazolidin-5-yl, Oxazolidin-2-yl, Oxazolidin-4-yl, Oxazolidin-5-yl, Thiazolidin-2-yl, Thiazolidin-4-yl, Thiazolidin-5-yl, Imidazolidin-2-yl, Imidazolidin-4-yl, 1,2,4-Oxadiazolidinyl, 1,3,4-Oxazolidinyl, 1,2,4-Thiazolidinyl, 1,2,4-Triazolidinyl, 1,2,5-Triazolidinyl, 2,3-Dihydrofuryl, 2,5-Dihydrofuryl, 2,3-Dihydrothienyl, 2,5-Dihydrothienyl, 2,3-Dihydropyrrolyl, 2,5-Dihydropyrrolyl, 2,3-Dihydroisoxazolyl, 2,5-Dihydroisoxazolyl, 4,5-Dihydroisoxazolyl, 2,3-Dihydroisothiazolyl, 2,5-Di-hydroisothiazolyl, 4,5-Dihydroisothiazolyl, 2,3-Dihydropyrazol, 2,5-Dihydropyrazolyl, 4,5-Dihydropyrazolyl, 2,5-Dihydrooxazolyl, 3,4-Dihydrooxazolyl, 2,5-Dihydrothiazolyl, 3,4-Dihydrothiazolyl, 2,5-Dihydroimidazolyl, 3,4-Dihydroimidazolyl, Piperidin-2-yl, Piperidin-3-yl, Piperidin-4-yl, Tetrahydropyridazin-3-yl, Tetrahydropyridazin-4-yl, Tetrahydropyrimidin-2-yl, Tetrahydropyrimidin-4-yl, Tetrahydropyrimidin-5-yl, Tetrahydropyrazin-2-yl, Tetrahydro-1,3,5-triazinyl, Tetrahydro-1,2,4-triazinyl, Dihydro-1,3-oxazinyl, 1,3-Dithian-2-yl, Tetrahydropyranyl, 1,3-Dioxolan-2-yl, 4H-1,3-Thiazin-2-yl, 4H-3,1-Benzothiazin-2-yl, 2H-1,4-Benzothiazin-3-yl, 2H-1,4-Benzoxazin-3-yl, 1,1-Dioxo-Tetrahydrothienyl, Dihydro-1,3-oxazin-2-yl, Morpholinyl oder Dihydrochinazolinyl.

Aryl, Aryloxy, Arylthio und Arylamino: aromatische, mono- oder poycyclische Kohlenwasserstoffe, welche direkt bzw. über ein Sauerstoffatom (-oxy), ein Schwefelatom (-thio) oder eine Aminogruppe (-amino) an das Gerüst gebunden sind, z.B. Phenyl, Naphthyl, Anthryl und Phenanthryl bzw. die entsprechenden Oxy-, Thio- oder Aminogruppen;
Arylalkyl: geradkettige oder verzweigte Alkylgruppen mit 1 bis 6 Kohlenstoffatomen (wie vorstehend genannt), welche in einer beliebigen Position einen der vorstehenden Arylreste tragen können;
Arylalkylamino: Alkylaminogruppen mit 1 bis 6, vorzugsweise 1 bis 4, insbesondere 1 bis 2 Kohlenstoffatomen (wie vorstehend genannt), welche am Stickstoffatom einen der vorstehend genannten Arylreste tragen;
Heteroaryl, Heteroaryloxy, -thio und -amino: aromatische, mono- oder polycyclische Systeme, welche neben Kohlenstoffringgliedern noch Sauerstoff-, Schwefel- und/oder Stickstoffatome als Ringglieder enthalten können und welche direkt bzw. über ein exocyclisches Sauerstoffatom (-oxy), Schwefelatom (-thio) oder eine Aminogruppe (-amino) an das Gerüst gebunden sind, z.B.
   - 5-gliedriges Heteroaryl, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können;
   - benzokondensiertes 5-gliedriges Heteroaryl, enthaltend ein bis drei Stickstoffatome oder ein Stickstoffatom und ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
   - über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, wobei diese Ringe über eines der Stickstoffringglieder an das Gerüst gebunden sind;
   - 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können;
   - benzokondensiertes 6-gliedriges Heteroaryl, enthaltend ein bis drei Stickstoffatome: 6-Ring Heteroarylgruppen, welche neben Kohlenstoffatomen ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
z.B. Fur-2-yl, Fur-3-yl, Thien-2-yl, Thien-3-yl, Pyrrol-1-yl, Pyrrol-2-yl, Pyrrol-3-yl, Isoxazol-3-yl, Isoxazol-4-yl, Isoxazol-5-yl, Isothiazol-3-yl, Isothiazol-4-yl, Isothiazol-5-yl, Pyrazol-1-yl, Pyrazol-3-yl, Pyrazol-4-yl, Pyrazol-5-yl, Oxazol-2-yl, Oxazol-4-yl, Oxazol-5-yl, Thiazol-2-yl, Thiazol-4-yl, Thiazol-5-yl, Imidazol-1-yl, Imidazol-2-yl, Imidazol-4-yl, Imidazol-5-yl, 1,2,4-Thiadiazolyl, 1,3,4-Thiadiazolyl, 1,2,4-Oxadiazolyl, 1,3,4-Oxadiazolyl, 1,2,4-Triazolyl, Tetrazolyl, Pyrid-2-yl, Pyrid-3-yl, Pyrid-4-yl, Pyridazin-3-yl, Pyridazin-4-yl, Pyrimidin-2-yl, Pyrimidin-4-yl, Pyrimidin-5-yl, Pyrazin-2-yl, 1,2,4-Triazinyl, 1,3,5-Triazinyl sowie 1,2,4,5-Tetrazinyl; dabei können benachbarte Substituenten des Heteroaromaten zu einem aromatischen oder heteroaromatischen Ring kondensiert sein, so daß Heteroaryl auch kondensierte Ringsysteme bedeutet wie z.B. Benzofuryl, Isobenzofuryl, Benzothien-2-yl, Benzothien-3-yl, Benzothien-4-yl, Benzothien-5-yl, Benzothien-6-yl, Benzothien-7-yl, Indol-1-yl, Indol-2-yl, Indol-3-yl, Isoindolyl, Benzisoxazolyl, Benzisothiazolyl, Benzthiazol-2-yl, Benzthiazol-4-yl, Benzthiazol-5-yl, Benzthiazol-6-yl, Benzthiazol-7-yl, Indazolyl, Benzimidazolyl, Benztriazolyl, Dibenzofuryl, Dibenzothienyl, Carbazolyl, Acridinyl, Phenanthridinyl, Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Phthalazinyl, Chinazolinyl, Chinoxalinyl, Cinnolinyl, 1,5-Naphthyridinyl, 1,6-Naphthyridinyl, 1,7-Naphthyridinyl, 1,8-Naphthyridinyl, Pteridinyl, Pyrrolopyridinyl, Pyrrolypyridazinyl, Pyrrolopyrimidinyl, Pyrrolopyrazinyl, Pyrrolo-1,2,4-triazinyl, Furopyridinyl, Furopyridazinyl, Furopyrimidinyl, Furopyrazinyl, Thienopyridinyl, Thienopyridazinyl, Thienopyrimidinyl, Thienopyrazinyl, Imidazopyridinyl, Imidazolopyridazinyl, Imidazopyrimidinyl, Purinyl, Imidazopyrazinyl, zolopyridinyl, Pyrazolopyridazinyl, Pyrazolopyrimidinyl, Pyrazolopyrazinyl, Isoxazolopyrazinyl, Oxazolopyridinyl, Oxazolopyrimidinyl, Isothiazolopyrimidinyl und Triazolopyrimidinyl bzw. die entsprechenden Oxy-, Thio und Aminogruppen;
Hetarylalkyl: geradkettige oder verzweigte Alkylgruppen mit 1 bis 6 Kohlenstoffatomen (wie vorstehend genannt), welche in einer beliebigen Position einen der vorstehenden Heteroarylreste tragen;
Hetarylalkylamino: Alkylaminogruppen mit 1 bis 6 Kohlenstoffatomen (wie vorstehend genannt), welche am Stickstoffatom einen der vorstehend genannten Heteroarylreste tragen;
A und B gemeinsam mit dem C-Atom, an das sie gebunden sind, ein ggf. annelierter, gesättigter oder ungesättigter, 3- bis 7-gliedriger isocyclischer oder 5- bis 7-gliedriger heterocyclischer Ring, z.B. Cyclopropyliden, Cyclobutyliden, Cyclopentyliden, Cyclopentenyliden, Cyclohexyliden, Cyclohexenyliden, Cycloheptyliden, Cycloheptenyliden, 4-Oxohexa-2,5-dien-1-yliden, Tetrahydrothien-3-yliden, Tetrahydropyran-4-yliden, Dihydropyran-4-yliden, Tetrahydrothiopyran-4-yliden oder Piperidin-4-yliden.

Bei der Definition der Verbindungen I wurden außerdem einzelne der vorstehend erläuterten Substituenten als "gegebenenfalls substituierte" bezeichnet. Darunter ist zu verstehen, das in diesen Resten Wasserstoffatome durch die im folgenden näher bezeichneten Gruppen ersetzt sein können:
"gegebenenfalls substituiert" in Bezug auf Alkyl, Akenyl und Alkinyl: die Substituenten können partiell oder vollständig halogeniert sein und/oder beispielsweise ein bis vorzugsweise eine der folgenden Gruppen tragen:
- Cyano, Nitro, Hydroxy, Merkapto, Amino, Formyl, Carboxyl, Formyloxy, Aminocarbonyl, Hydroxyaminocarbonyl, Formamido, Aminothiocarbonyl, Hydroxysulfinyl, Hydroxysulfonyl,
- des weiteren Alkyl-, Alkenyl- oder Alkinylgruppen welche über Sauerstoff (-O-), Schwefel (-S-), Amino (-NH-), Alkylamino [-N(Alkyl)-], Carbonyl (-CO-), Carbonyloxy (-CO-O-), Oxycarbonyl (-O-CO-), Aminocarbonyl (-NH-CO-), Oxyaminocarbonyl (-O-NH-CO-), Carbonylamino (-CO-NH-), Carbonylalkylamino [-CO-N(Alkyl)-], Sulfoxyl, Sulfonyl, Sulfinyl, Sulfinyloxy oder Sulfonyloxy an den Substituenten gebunden sind,
- des weiteren Cycloalkyl-, Cycloalkenyl-, Heterocyclyl-, Aryl- oder Heteroarylgruppen, welche direkt oder über Sauerstoff (-O-), Schwefel (-S-), Amino (-NH-), Alkylamino [-N(Alkyl)-], Carbonyl (-CO-), Carbonyloxy (-CO-O-), Oxycarbonyl (-O-CO-), Aminocarbonyl (-NH-CO-), Oxyaminocarbonyl (-O-NH-CO-), Carbonylamino (-CO-NH-), Carbonylalkylamino [-CO-N(Alkyl)-], Sulfoxyl, Sulfonyl, Sulfinyl, Sulfinyloxy oder Sulfonyloxy an den Substituenten gebunden sind,
- des weiteren Trialkylsilyl (Silylgruppe mit drei voneinander unabhängigen C₁-C₄-Alkylresten), Dialkoxyphosphinyl (Phosphinylgruppe mit zwei voneinander unabhängigen C₁-C₄-Alkoxyresten) oder Bis-(Aryloxy)-phosphinyl,
- des weiteren eine Gruppe -ON=CR'R'', wobei R' Wasserstoff, Alkyl, Akenyl oder Alkinyl bedeutet und R'' Wasserstoff, Alkyl, Alkoxy, Akenyl oder Alkinyl bedeutet;
"gegebenenfalls substituiert" in Bezug auf Cycloalkyl, Cycloalkenyl und Heterocyclyl: die Substituenten können partiell oder vollständig halogeniert sein und/oder beispielsweise ein bis drei, vorzugsweise eine der folgenden Gruppen tragen:
- Cyano, Nitro, Hydroxy, Merkapto, Amino, Formyl, Carboxyl, Formyloxy, Aminocarbonyl, Hydroxyaminocarbonyl, Formamido, Aminothiocarbonyl, Hydroxysulfinyl, Hydroxysulfonyl,
- des weiteren Alkyl-, Alkenyl- oder Alkinylgruppen welche direkt oder über Sauerstoff (-O-), Schwefel (-S-), Amino (-NH-), Alkylamino [-N(Alkyl)-], Carbonyl (-CO-), Carbonyloxy (-CO-O-), Oxycarbonyl (-O-CO-), Aminocarbonyl (-NH-CO-), Oxyaminocarbonyl (-O-NH-CO-), Carbonylamino (-CO-NH-), Carbonylalkylamino [-CO-N(Alkyl)-], Sulfoxyl, Sulfonyl, Sulfinyl, Sulfinyloxy oder Sulfonyloxy an den Substituenten gebunden sind,
- des weiteren Cycloalkyl-, Cycloalkenyl-, Heterocyclyl-, Aryl- oder Heteroarylgruppen, welche direkt oder über Sauerstoff (-O-), Schwefel (-S-), Amino (-NH-), Alkylamino [-N(Alkyl)-], Carbonyl (-CO-), Carbonyloxy (-CO-O-), Oxycarbonyl (-O-CO-), Aminocarbonyl (-NH-CO-), Oxyaminocarbonyl (-O-NH-CO-), Carbonylamino (-CO-NH-), Carbonylalkylamino [-CO-N(Alkyl)-] Sulfoxyl, Sulfonyl, Sulfinyl, Sulfinyloxy oder Sulfonyloxy an den Substituenten gebunden sind,
- des weiteren Trialkylsilyl (Silylgruppe mit drei voneinander unabhängigen C₁-C₄-Alkylresten), Dialkoxyphosphinyl (Phosphinylgruppe mit zwei voneinander unabhängigen C₁-C₄-Alkoxyresten) oder Bis-(Aryloxy)-phosphinyl,
- des weiteren eine Gruppe -ON=CR'R'', wobei R' Wasserstoff, Alkyl, Akenyl oder Alkinyl bedeutet und R'' Wasserstoff, Alkyl, Alkoxy, Akenyl oder Alkinyl bedeutet;
"gegebenenfalls substituiert" in Bezug auf Aryl und Heteroaryl: die Substituenten können partiell oder vollständig halogeniert sein und/oder beispielsweise ein bis drei der folgenden Gruppen tragen:
- Cyano, Nitro, Hydroxy, Merkapto, Amino, Formyl, Carboxyl, Formyloxy, Aminocarbonyl, Hydroxyaminocarbonyl, Formamido, Aminothiocarbonyl, Hydroxysulfinyl, Hydroxysulfonyl,
- des weiteren Alkyl-, Alkenyl- oder Alkinylgruppen welche direkt oder über Sauerstoff (-O-), Schwefel (-S-), Amino (-NH-), Alkylamino [-N(Alkyl)-], Carbonyl (-CO-), Carbonyloxy (-CO-O-), Oxycarbonyl (-O-CO-), Aminocarbonyl (-NH-CO-), Oxyaminocarbonyl (-O-NH-CO-), Carbonylamino (-CO-NH-), Carbonylalkylamino [-CO-N(Alkyl)-], Sulfoxyl, Sulfonyl, Sulfinyl, Sulfinyloxy oder Sulfonyloxy an den Substituenten gebunden sind,
- des weiteren Cycloalkyl-, Cycloalkenyl-, Heterocyclyl-, Aryl- oder Heteroarylgruppen, welche direkt oder über Sauerstoff (-O-), Schwefel (-S-), Amino (-NH-), Alkylamino [-N(Alkyl)-], Carbonyl (-CO-), Carbonyloxy (-CO-O-), Oxycarbonyl (-O-CO-), Aminocarbonyl (-NH-CO-), Oxyaminocarbonyl (-O-NH-CO-), Carbonylamino (-CO-NH-), Carbonylalkylamino [-CO-N(Alkyl)-], Sulfoxyl, Sulfonyl, Sulfinyl, Sulfinyloxy oder Sulfonyloxy an den Substituenten gebunden sind,
- des weiteren Trialkylsilyl (Silylgruppe mit drei voneinander unabhängigen C₁-C₄-Alkylresten), Dialkoxyphosphinyl (Phosphinylgruppe mit zwei voneinander unabhängigen C₁-C₄-Alkoxyresten) oder Bis-(Aryloxy)-phosphinyl;
- des weiteren eine Gruppe -ON=CR'R'', wobei R' Wasserstoff, Alkyl, Akenyl oder Alkinyl bedeutet und R'' Wasserstoff, Alkyl, Alkoxy, Akenyl oder Alkinyl bedeutet.

Der Ausdruck "partiell oder vollständig halogeniert" soll zum Ausdruck bringen, daß in den entsprechenden Resten die an C-Atome gebundenen Wasserstoffatome teilweise oder vollständig durch gleiche oder verschiedene Halogenatome ersetzt sind.

In den oben aufgeführten Substituenten haben Halogen und die organischen Teilstrukturen jeweils die vorstehend genannten Bedeutungen. Diese Substituenten können ihrerseits wieder mit einem oder mehreren der oben aufgeführten Reste substituiert sein.

Die erfindungsgemäßen Verbindungen können bei der Herstellung als Stereoisomere (z.B. Z/E-Isomere an C=C- oder C=N-Doppelbindungen, Enantio- oder Diastereomere) anfallen, die nach den üblichen Methoden (Chromatographie, Kristallisation, Destillation) getrennt werden können. Sowohl die einzelnen Isomeren als auch ihre Gemische eignen sich als Fungizide und werden von der Erfindung erfaßt.

Im Hinblick auf ihre biologische Wirkung werden Verbindungen I bevorzugt, in denen X Wasserstoff, Chlor, Brom, Methyl oder Methoxy bedeutet.

Daneben werden Verbindungen I bevorzugt, in denen Y Methoxy und Z Methoxymethylen bedeutet.

Außerdem werden Verbindungen I bevorzugt, in denen Y Methoxy und Z Methoxyimino bedeutet.

des weiteren werden Verbindungen I bevorzugt, in denen Y Methoxy und Z Methylmethylen bedeutet.

Außerdem werden Verbindungen I bevorzugt, in denen Y Methoxy oder Methylamino bedeutet.

Daneben werden Verbindungen I bevorzugt, in denen Y Methylamino und Z Methoxyimino bedeutet.

Daneben werden Verbindungen I bevorzugt, in denen Y Methylamino und Z Methoxymethylen bedeutet.

des weiteren werden Verbindungen I bevorzugt, in denen Y Methylamino und Z Methylmethylen bedeutet.

des weiteren werden Verbindungen I bevorzugt, in denen R Wasserstoff oder Methyl bedeutet.

Daneben werden Verbindungen I bevorzugt, in denen A Wasserstoff, Cyclopropyl, C₁-C₃-Alkyl oder C₁-C₂-Halogenalkyl, insbesondere Wasserstoff, Cyclopropyl, Methyl, Ethyl, Isopropyl oder Trifluormethyl bedeutet.

Außerdem werden Verbindungen I bevorzugt, in denen B für ggf. subst. Alkyl oder Cycloalkyl steht.

Insbesondere werden Verbindungen I bevorzugt, in denen B für C₁-C₁₂-Alkyl oder C₃-C₇-Cycloalkyl steht.

des weiteren werden Verbindungen I bevorzugt, in denen B für ggf. subst. Aryl steht.

Insbesondere werden Verbindungen I bevorzugt, in denen B für Aryl (besonders Phenyl oder Naphthyl) steht, welches partiell oder vollständig halogeniert ist und/oder eine Gruppe -CR^{a}=NR^{b} (R^{a} = Wasserstoff, Amino, C₁-C₄-Alkyl, C₁-C₄-Alkoxy, C₁-C₄-Alkylthio und C₁-C₄-Alkylamino; R^{b} = C₁-C₄-Alkyl und C₁-C₄-Alkoxy), und/oder ein bis drei der folgenden Gruppen trägt: Cyano, Nitro, Hydroxy, Mercapto, Formyl, Carboxyl, Aminocarbonyl, Cyanomethyl, C₁-C₆-Alkyl, C₃-C₆-Cycloalkyl, C₁-C₄-Halogenalkyl, C₂-C₄-Alkenyl, C₂-C₄-Alkinyl, C₁-C₆-Alkoxy, C₃-C₆-Cycloalkoxy, C₁-C₄-Halogenalkoxy, Mono- oder Di-C₁-C₄-Alkoxy-C₁-C₂-alkyl, C₁-C₄-Alkylthio, C₃-C₆-Cycloalkylthio, C₁-C₆-Alkylcarbonyl, C₁-C₆-Alkoxycarbonyl, C₁-C₆-Alkylcarbonyloxy, C₁-C₄-Alkylaminocarbonyl, Di-C₁-C₄-Alkylaminocarbonyl, C₁-C₄-Alkylcarbonylamino, C₁-C₄-Alkylcarbonyl-C₁-C₄-amino, C₁-C₄-Alkylsulfonyl, C₁-C₄-Alkylsulfoxyl, Phenyl oder Phenoxy, wobei die Phenylringe ihrerseits partiell oder vollständig halogeniert sein können und/oder ein bis drei der folgenden Gruppen tragen können: Cyano, Nitro, C₁-C₄-Alkyl, C₃-C₆-Cycloalkyl, C₁-C₄-Halogenalkyl, C₁-C₄-Alkoxy, C₃-C₆-Cycloalkoxy und C₁-C₄-Alkylthio.

Daneben werden Verbindungen I bevorzugt, in denen B für ggf. subst. Heteroaryl steht.

Besonders bevorzugt werden Verbindungen I, in denen B für ggf. subst. Pyridyl, Pyridazinyl, Pyrimidinyl, Pyrazinyl, Chinolinyl, Isochinolinyl, Cinnolinyl, Chinazolinyl, Chinoxalinyl, Pteridinyl, Furyl, Thienyl, Pyrryl, Oxazolyl, Thiazolyl, Imidazolyl, Isoxazolyl, Isothiazolyl, Pyrazolyl, Oxadiazolyl, Thiadiazolyl, Triazolyl, Triazinyl, Tetrazinyl, Benzofuryl, Thionaphthenyl, Indolyl, Benzoxazolyl, Benzothiazolyl, Benzimidazolyl, Benzisoxazolyl, Benzoisothiazolyl, Benzopyrazolyl oder Purinyl steht.

Insbesondere werden Verbindungen I bevorzugt, in denen B für Heteroaryl steht, welches partiell halogeniert sein kann und/oder einen bei drei (vorzugsweise einen oder zwei) der folgenden Reste tragen kann: Nitro, Cyanomethyl, C₁-C₄-Alkyl, C₃-C₆-Cycloalkyl, C₁-C₄-Halogenalkyl, C₁-C₄-Alkoxy, C₃-C₆-Cycloalkoxy, C₁-C₄-Halogenalkoxy, Phenyl oder Phenoxy, wobei die Phenylringe ihrerseits partiell oder vollständig halogeniert sein können und/oder ein bis drei der folgenden Gruppen tragen können: Cyano, Nitro, C₁-C₄-Alkyl, C₃-C₆-Cycloalkyl, C₁-C₄-Halogenalkyl, C₁-C₄-Alkoxy, C₃-C₆-Cycloalkoxy und C₁-C₄-Alkylthio.

Außerdem werden Verbindungen I bevorzugt, in denen B für eine Gruppe -CO-W steht, wobei W Wasserstoff, C₁-C₁₂-Alkyl, C₁-C₆-Alkoxy, C₃-C₇-Cycloalkyl, Phenyl, Benzyl, Phenoxy, Benzyloxy, Pyridyl, Furyl, Thiazolyl oder Chinolinyl steht.

des weiteren werden Verbindungen I bevorzugt, in denen A und B gemeinsam mit dem C-Atom an das sie gebunden sind für eine der folgenden Gruppen stehen: C₅-C₆-Cycloalkyl, C₅-C₆-Cycloalkenyl, Tetrahydronaphthyl oder Tetrahydrobenzopyranyl.

Insbesondere bevorzugte Verbindungen der Formel I sind in den folgenden Tabellen 1 bis 16 zusammengestellt.

### Tabelle 1

Verbindungen der allgemeinen Formel I.1, in denen Y Methoxy und Z CHCH₃ bedeutet und die Kombination der Substituenten A, R und Qₘ für eine Verbindung jeweils einer Zeile der Tabelle A entspricht

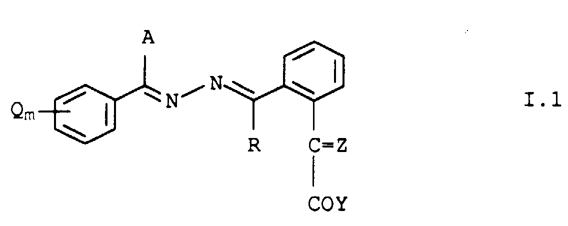

### Tabelle 2

Verbindungen der allgemeinen Formel I.1, in denen Y Methoxy und Z CHOCH₃ bedeutet und die Kombination der Substituenten A, R und Qₘ für eine Verbindung jeweils einer Zeile der Tabelle A entspricht

### Tabelle 3

Verbindungen der allgemeinen Formel I.1, in denen Y Methoxy und Z NOCH₃ bedeutet und die Kombination der Substituenten A, R und Qₘ für eine Verbindung jeweils einer Zeile der Tabelle A entspricht

### Tabelle 4

Verbindungen der allgemeinen Formel I.1, in denen Y Methylamino und Z CHCH₃ bedeutet und die Kombination der Substituenten A, R und Qₘ für eine Verbindung jeweils einer Zeile der Tabelle A entspricht

### Tabelle 5

Verbindungen der allgemeinen Formel I.1, in denen Y Methylamino und Z CHOCH₃ bedeutet und die Kombination der Substituenten A, R und Qₘ für eine Verbindung jeweils einer Zeile der Tabelle A entspricht

### Tabelle 6

Verbindungen der allgemeinen Formel I.1, in denen Y Methylamino und Z NOCH₃ bedeutet und die Kombination der Substituenten A, R und Qₘ für eine Verbindung jeweils einer Zeile der Tabelle A entspricht

### Tabelle 7

Verbindungen der allgemeinen Formel I.1, in denen Y Amino und Z CHOCH₃ bedeutet und die Kombination der Substituenten A, R und Qₘ für eine Verbindung jeweils einer Zeile der Tabelle A entspricht

### Tabelle 8

Verbindungen der allgemeinen Formel I.1, in denen Y Dimethylamino und Z CHOCH₃ bedeutet und die Kombination der Substituenten A, R und Qₘ für eine Verbindung jeweils einer Zeile der Tabelle A entspricht

### Tabelle 9

Verbindungen der allgemeinen Formel I.2, in denen Y Methoxy und Z CHCH₃ bedeutet und die Kombination der Substituenten A, B und R für eine Verbindung jeweils einer Zeile der Tabelle B entspricht

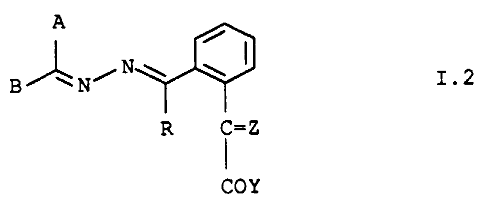

### Tabelle 10

Verbindungen der allgemeinen Formel I.2, in denen Y Methoxy und Z CHOCH₃ bedeutet und die Kombination der Substituenten A, B und R für eine Verbindung jeweils einer Zeile der Tabelle B entspricht

### Tabelle 11

Verbindungen der allgemeinen Formel I.2, in denen Y Methoxy und Z NOCH₃ bedeutet und die Kombination der Substituenten A, B und R für eine Verbindung jeweils einer Zeile der Tabelle B entspricht

### Tabelle 12

Verbindungen der allgemeinen Formel I.2, in denen Y Methylamino und Z CHCH₃ bedeutet und die Kombination der Substituenten A, B und R für eine Verbindung jeweils einer Zeile der Tabelle B entspricht

### Tabelle 13

Verbindungen der allgemeinen Formel I.2, in denen Y Methylamino und Z CHOCH₃ bedeutet und die Kombination der Substituenten A, B und R für eine Verbindung jeweils einer Zeile der Tabelle B entspricht

### Tabelle 14

Verbindungen der allgemeinen Formel I.2, in denen Y Methylamino und Z NOCH₃ bedeutet und die Kombination der Substituenten A, B und R für eine Verbindung jeweils einer Zeile der Tabelle B entspricht

### Tabelle 15

Verbindungen der allgemeinen Formel I.2, in denen Y Amino und Z CHOCH₃ bedeutet und die Kombination der Substituenten A, B und R für eine Verbindung jeweils einer Zeile der Tabelle B entspricht

### Tabelle 16

Verbindungen der allgemeinen Formel I.2, in denen Y Dimethylamino und Z CHOCH₃ bedeutet und die Kombination der Substituenten A, B und R für eine Verbindung jeweils einer Zeile der Tabelle B entspricht

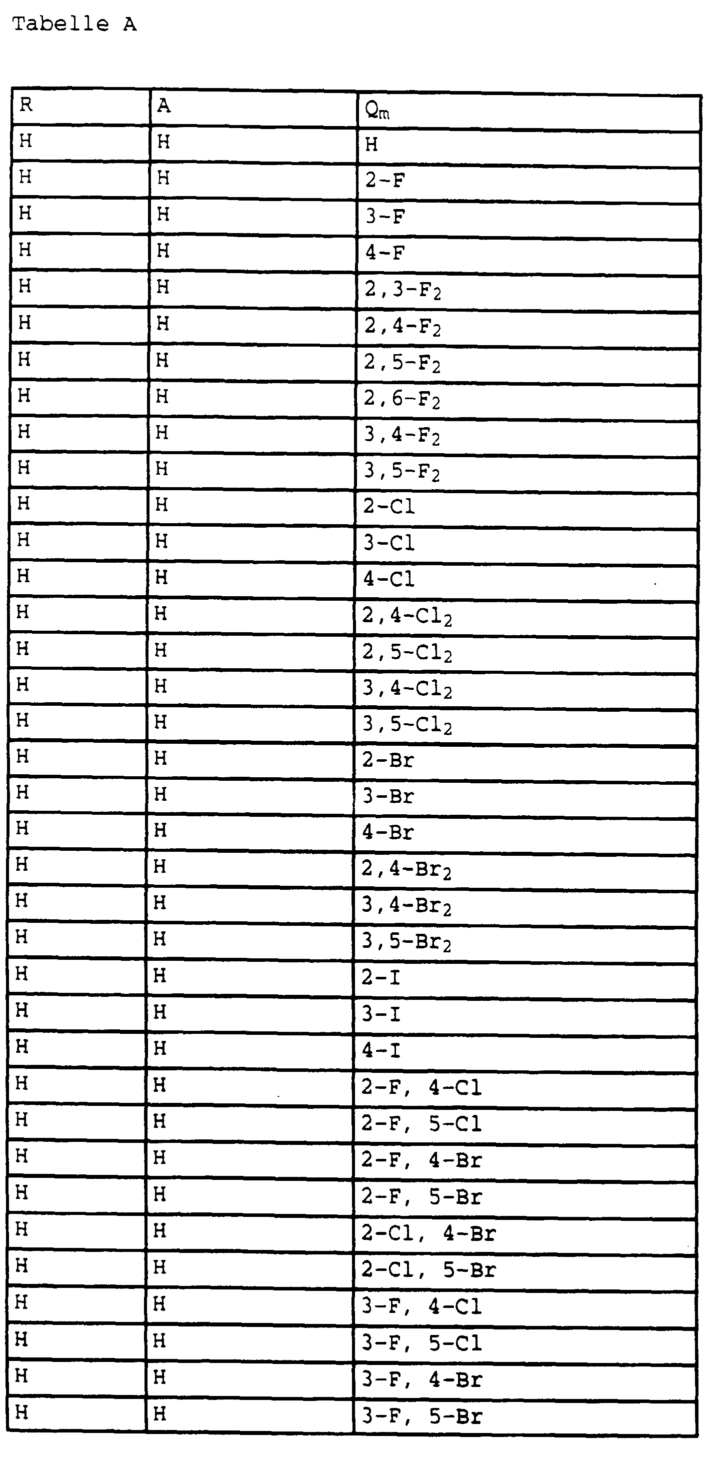

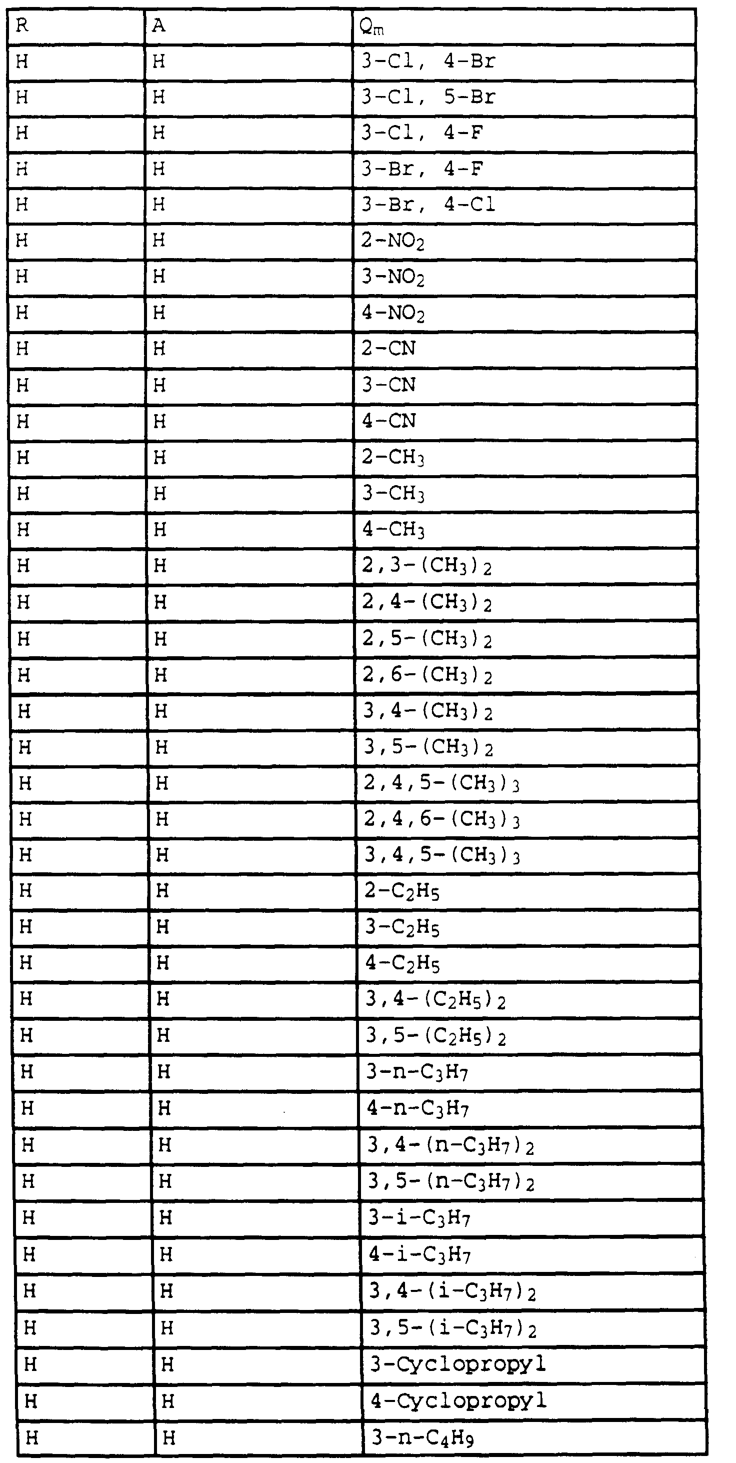

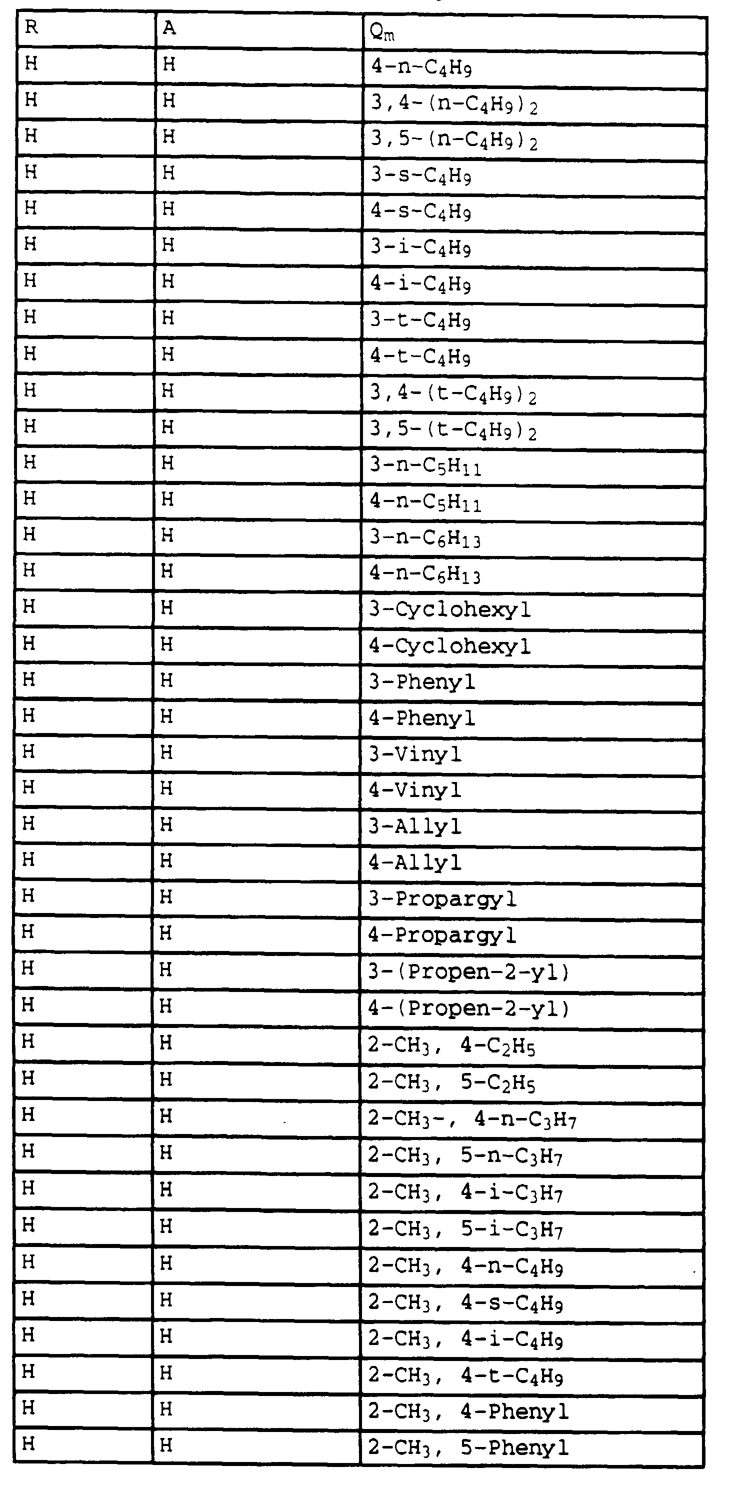

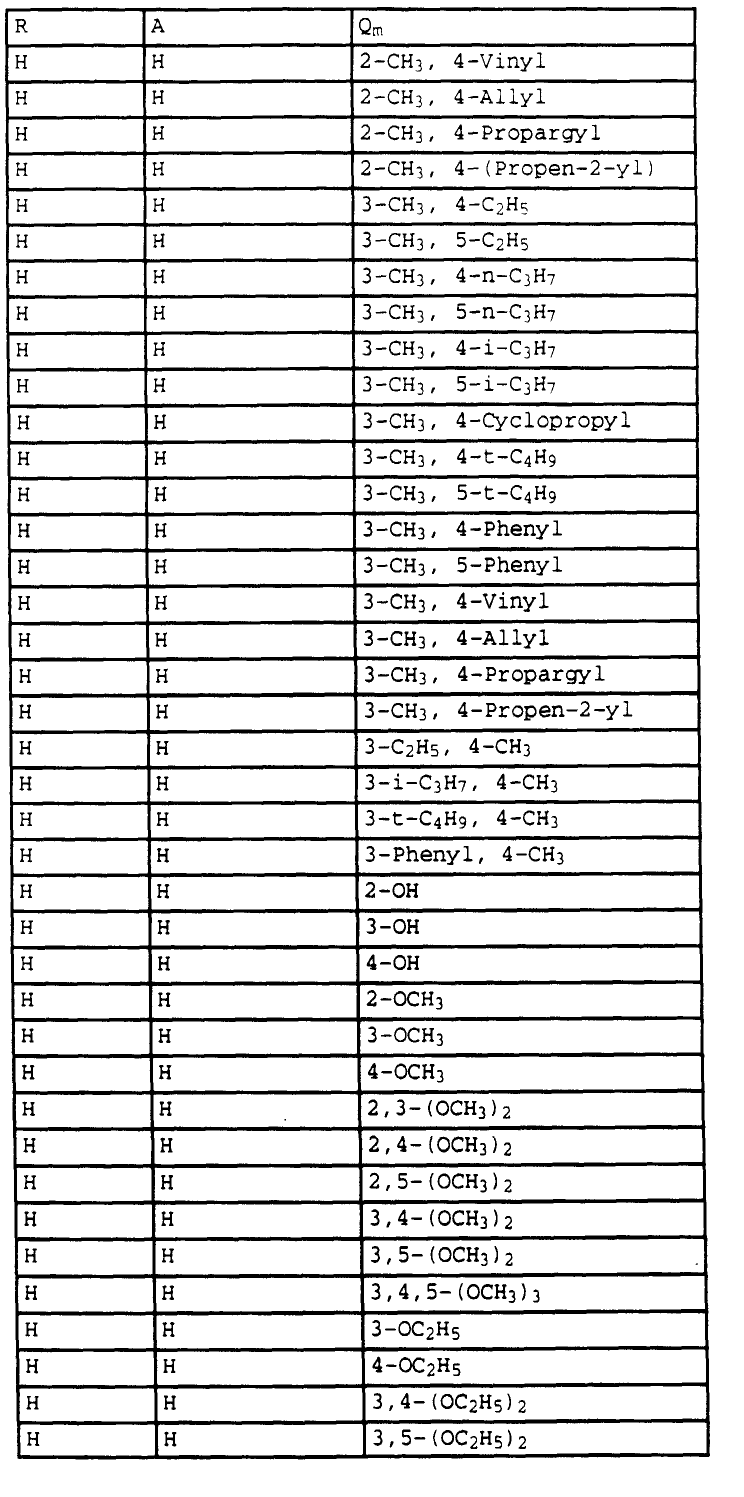

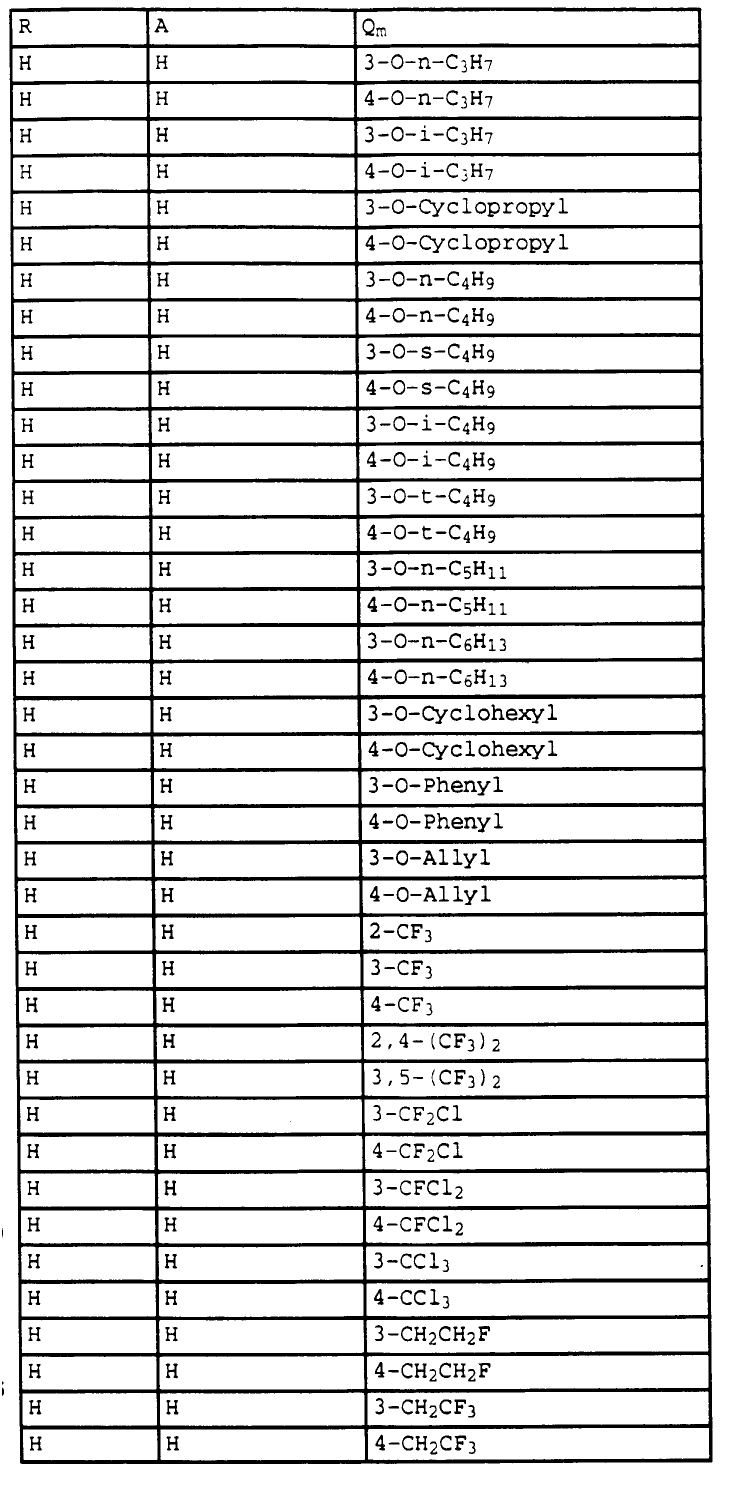

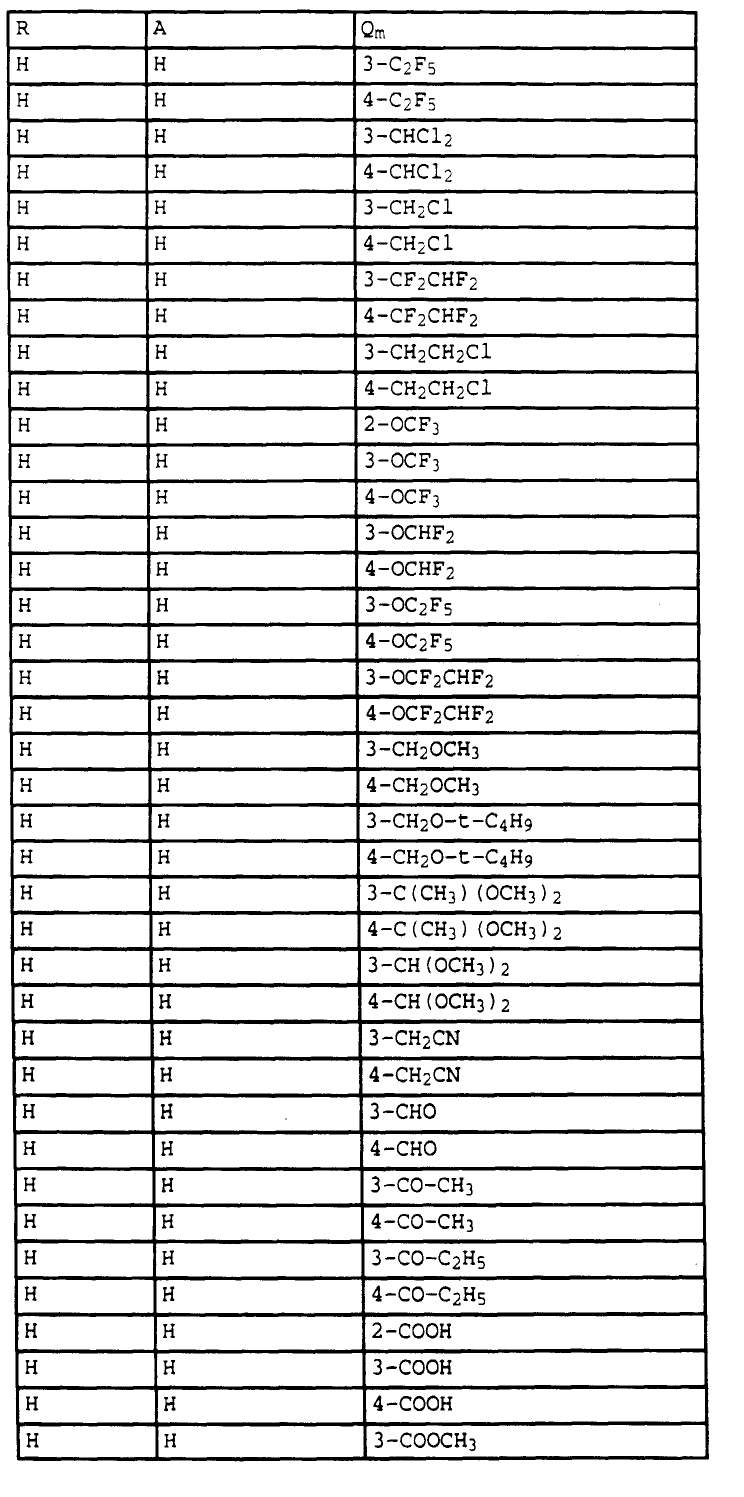

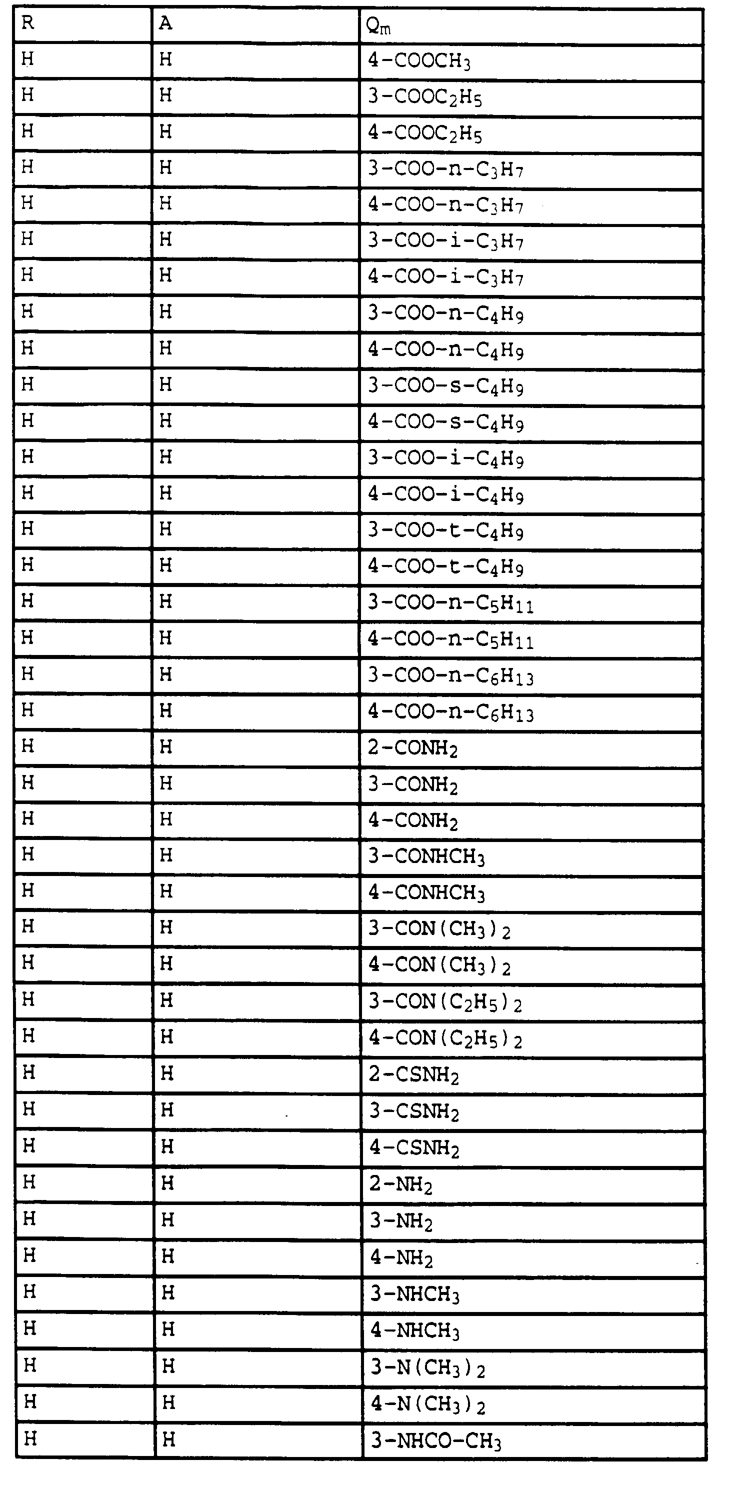

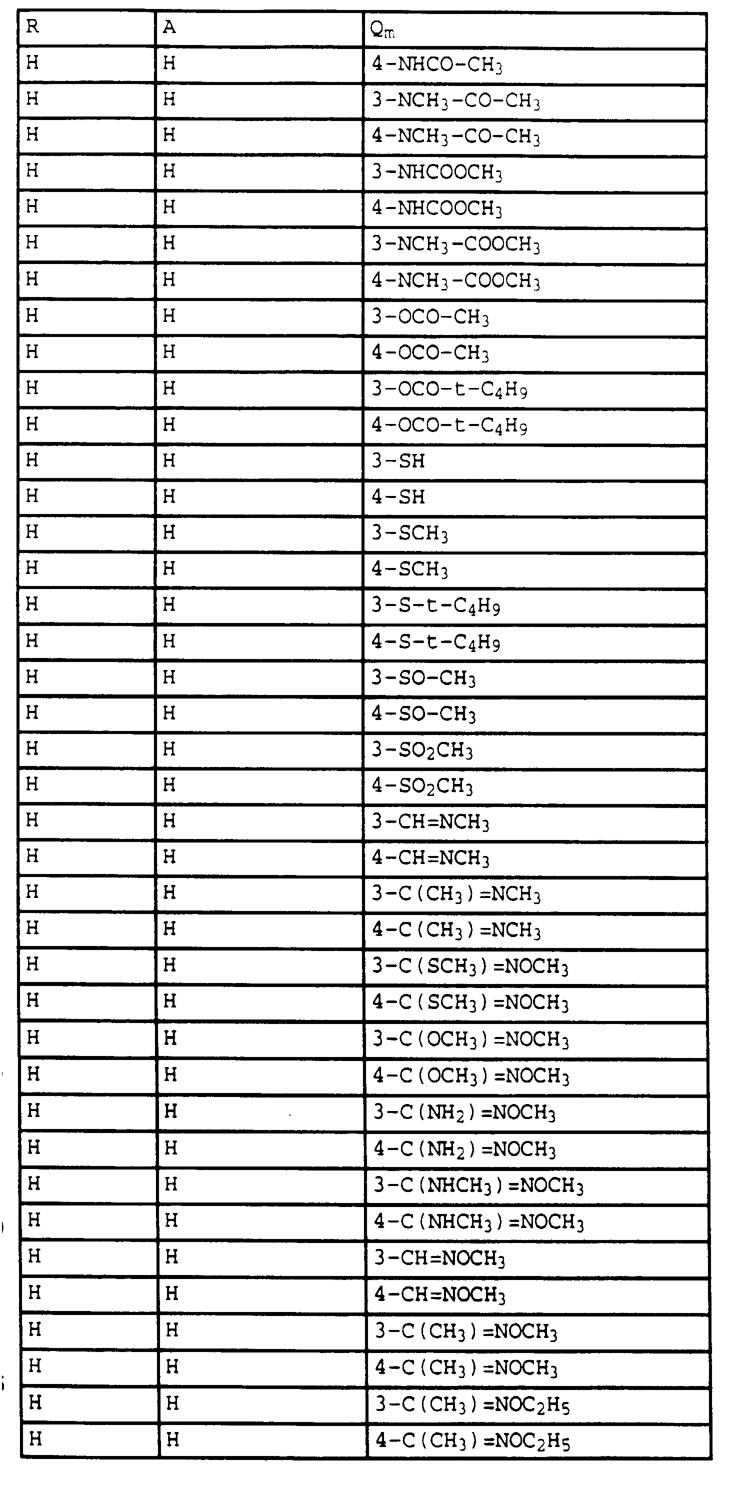

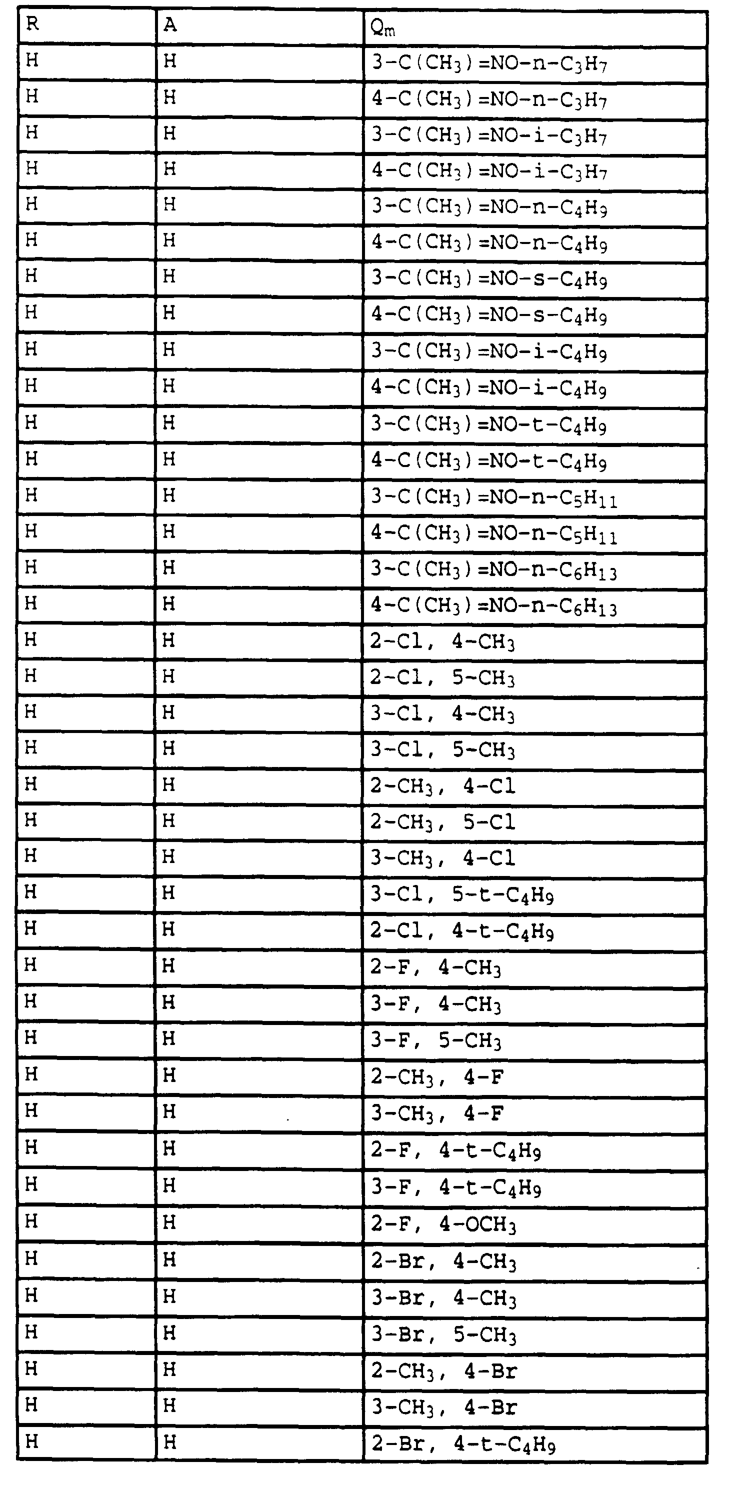

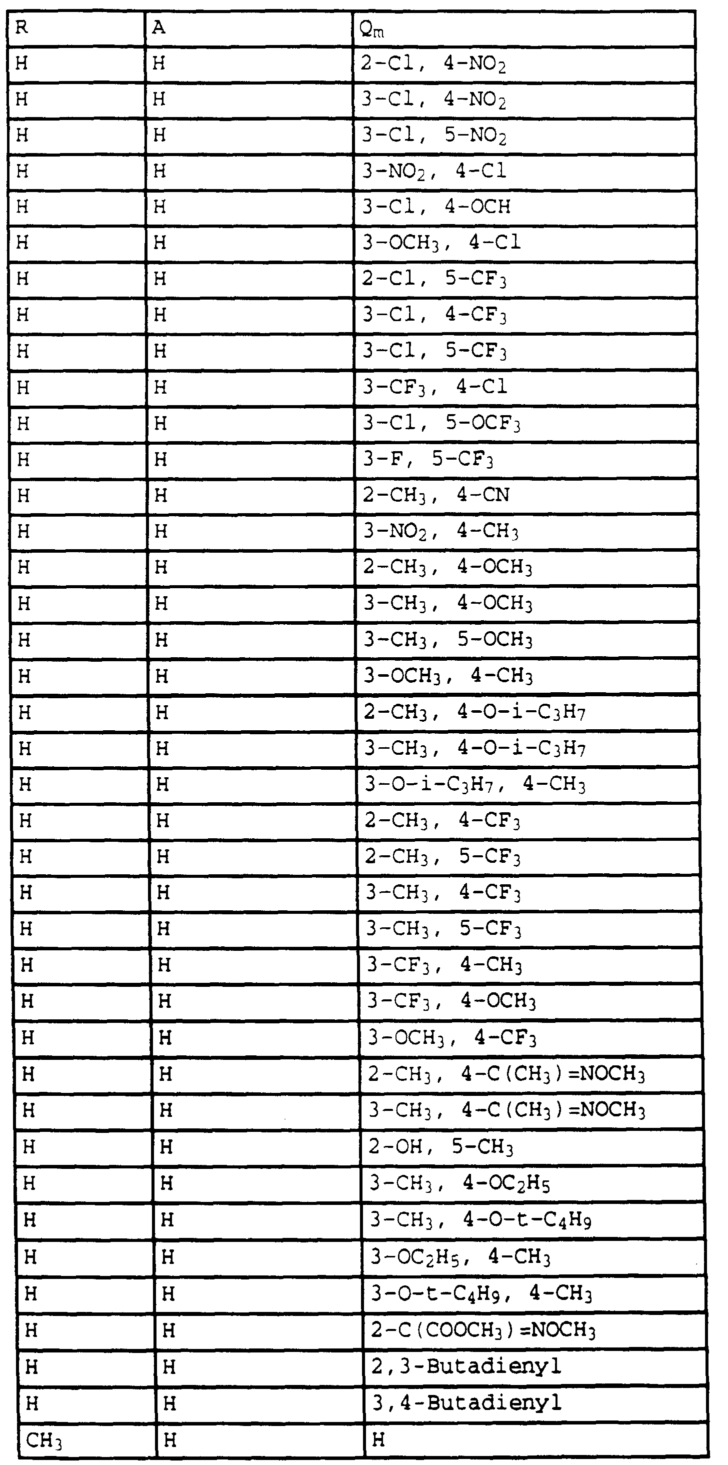

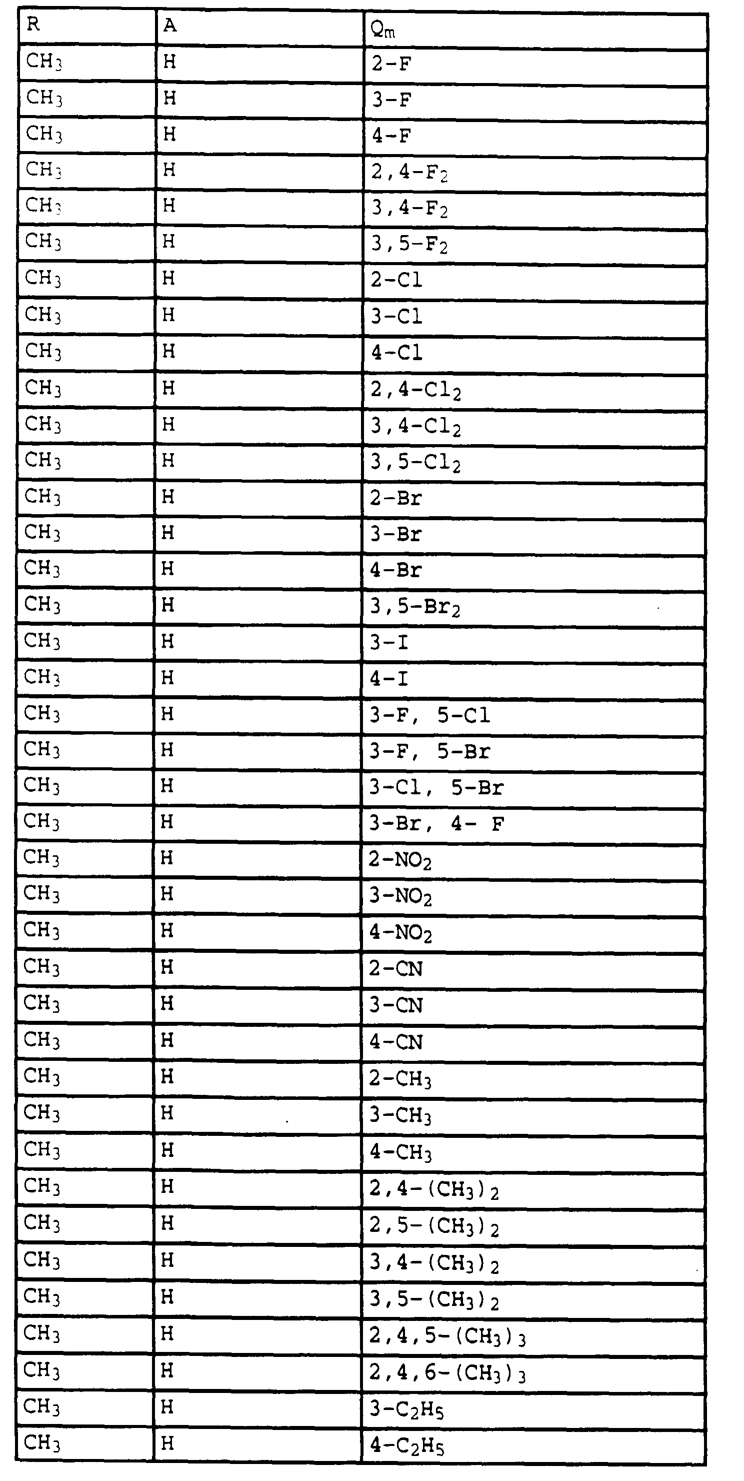

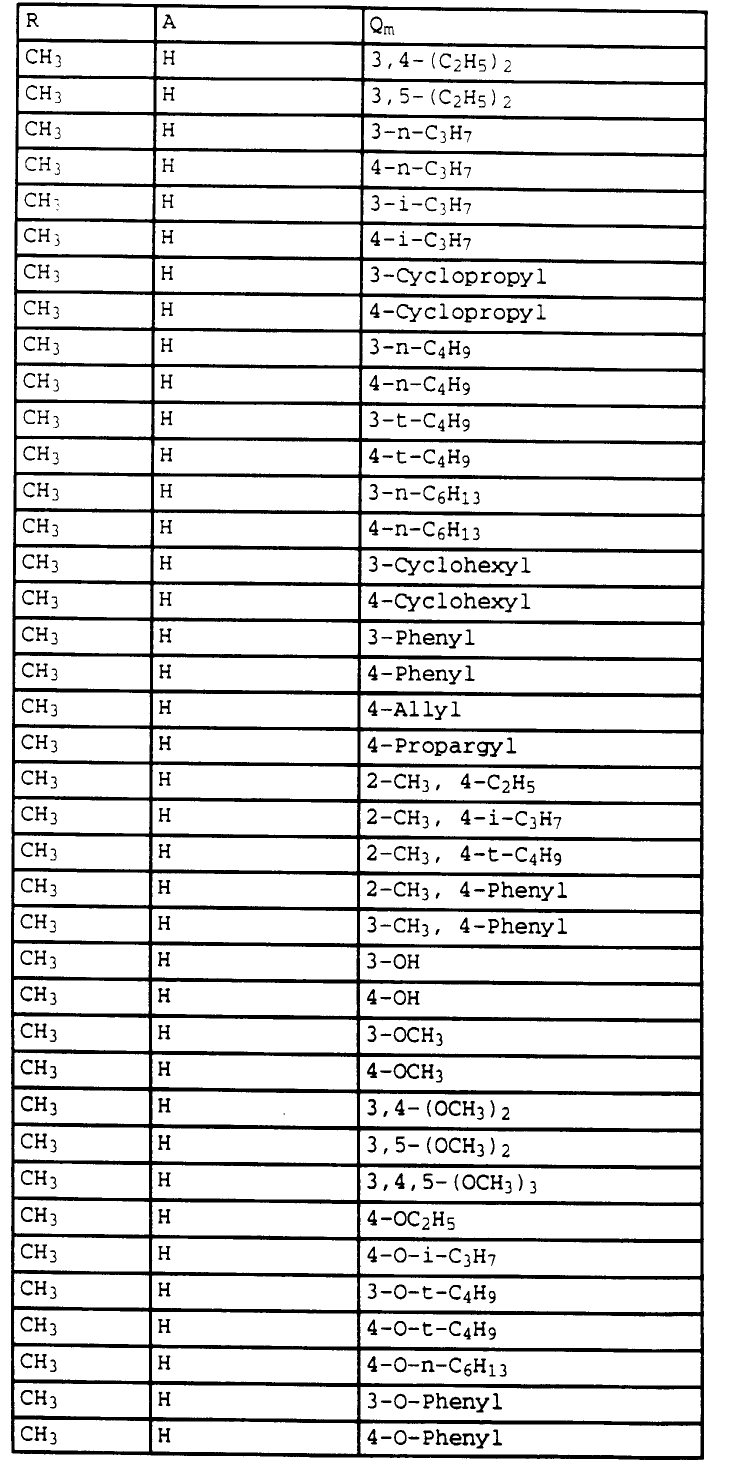

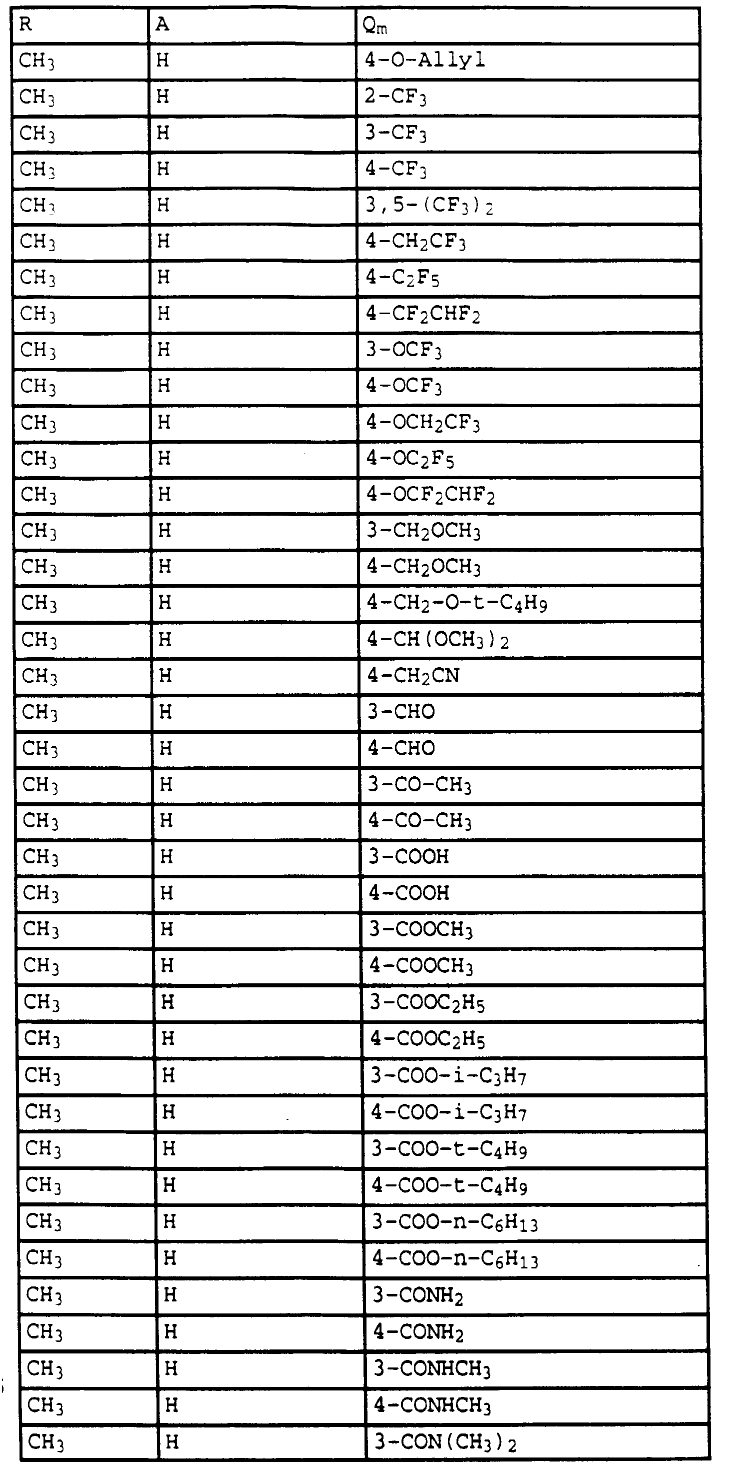

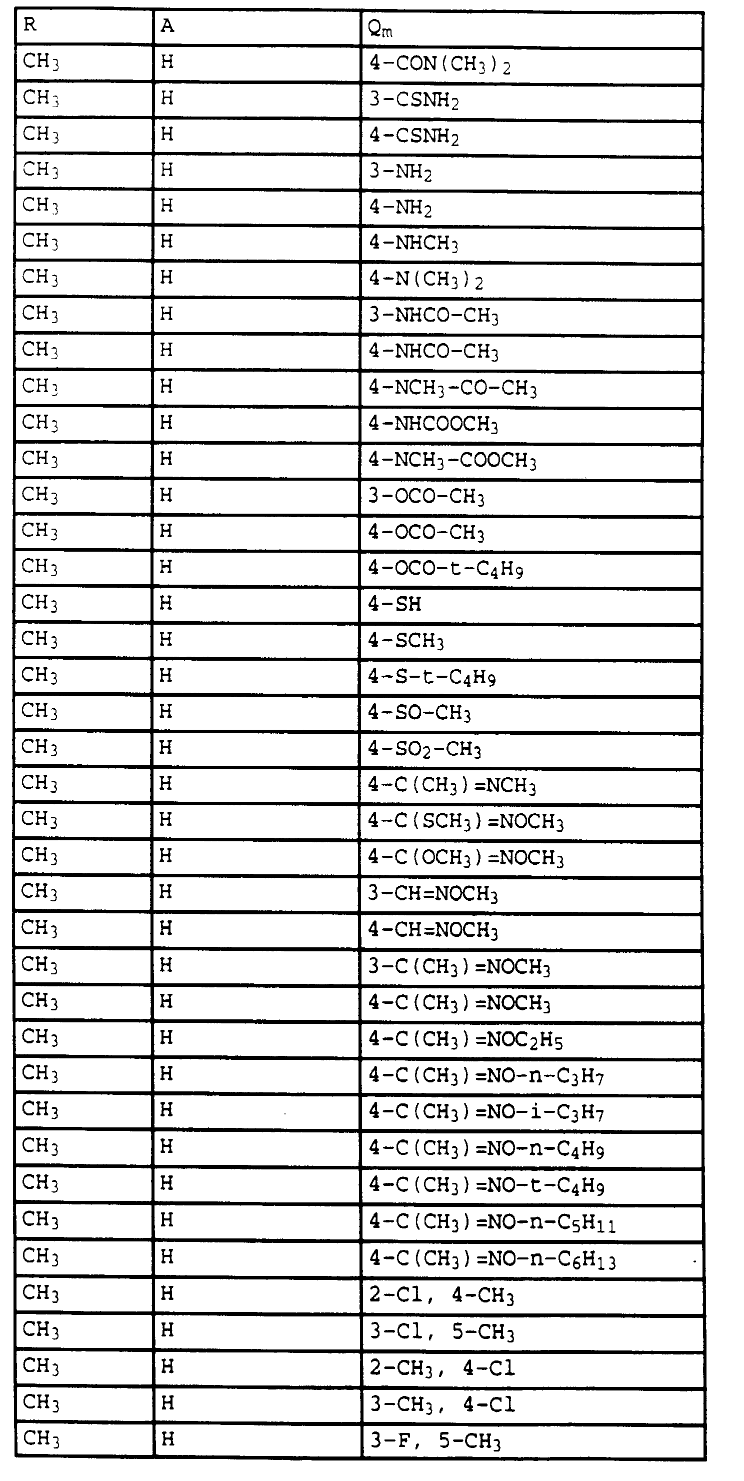

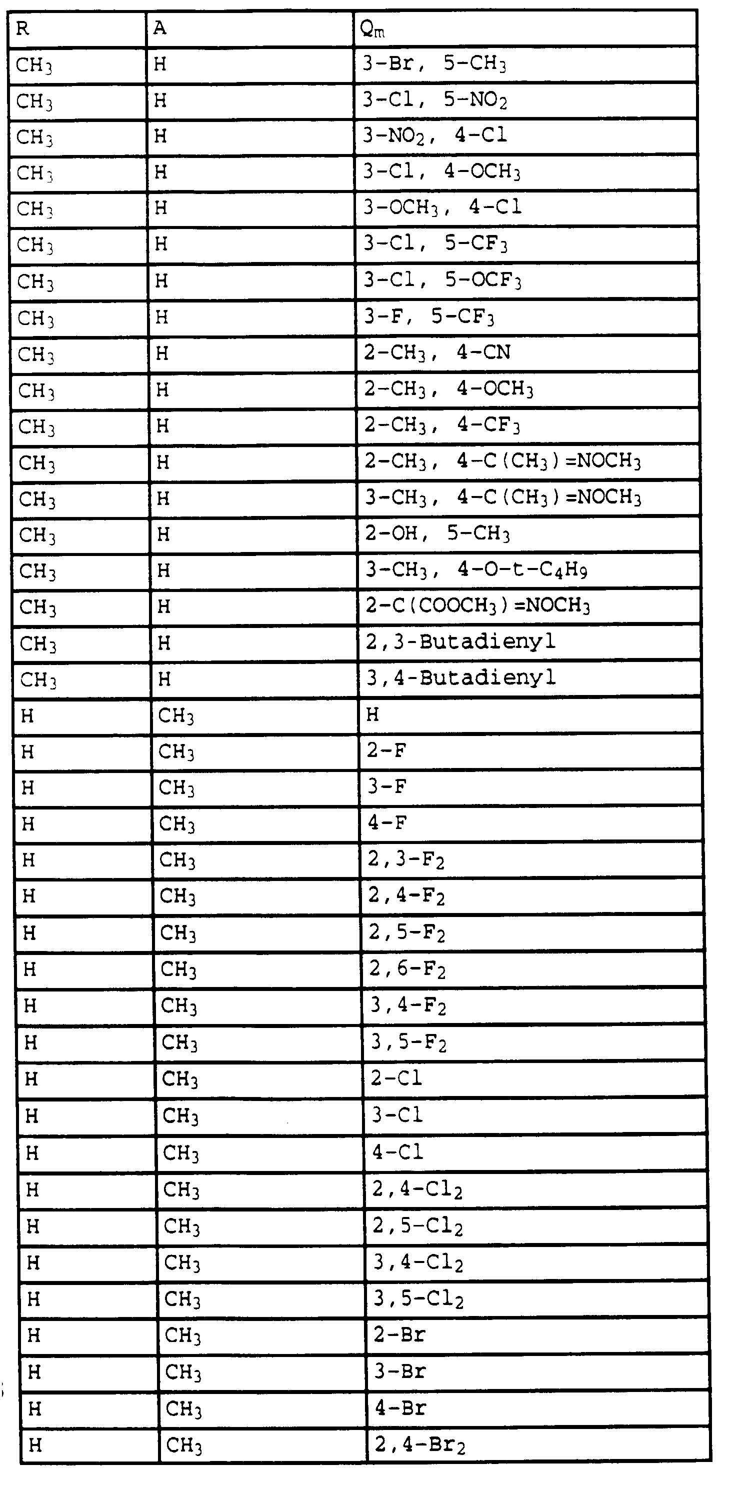

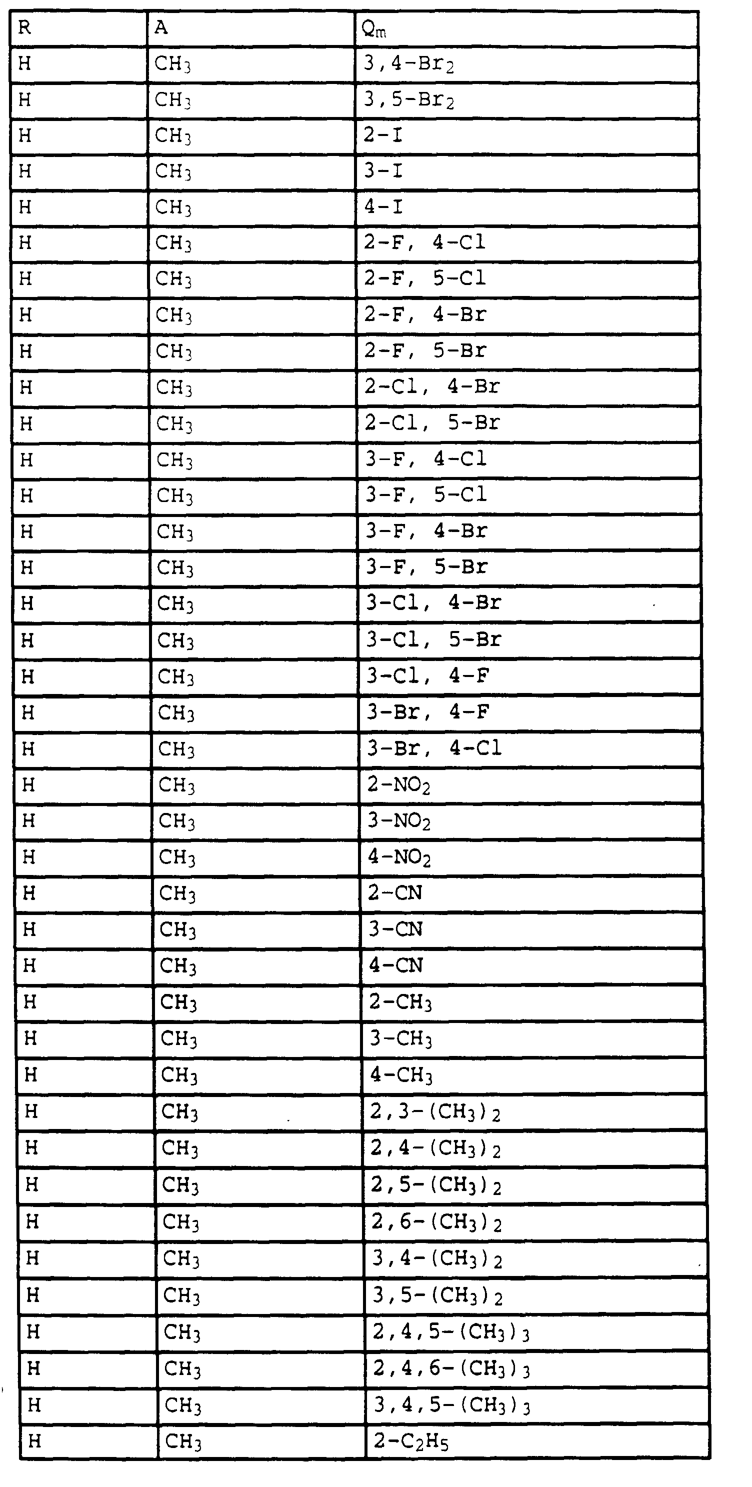

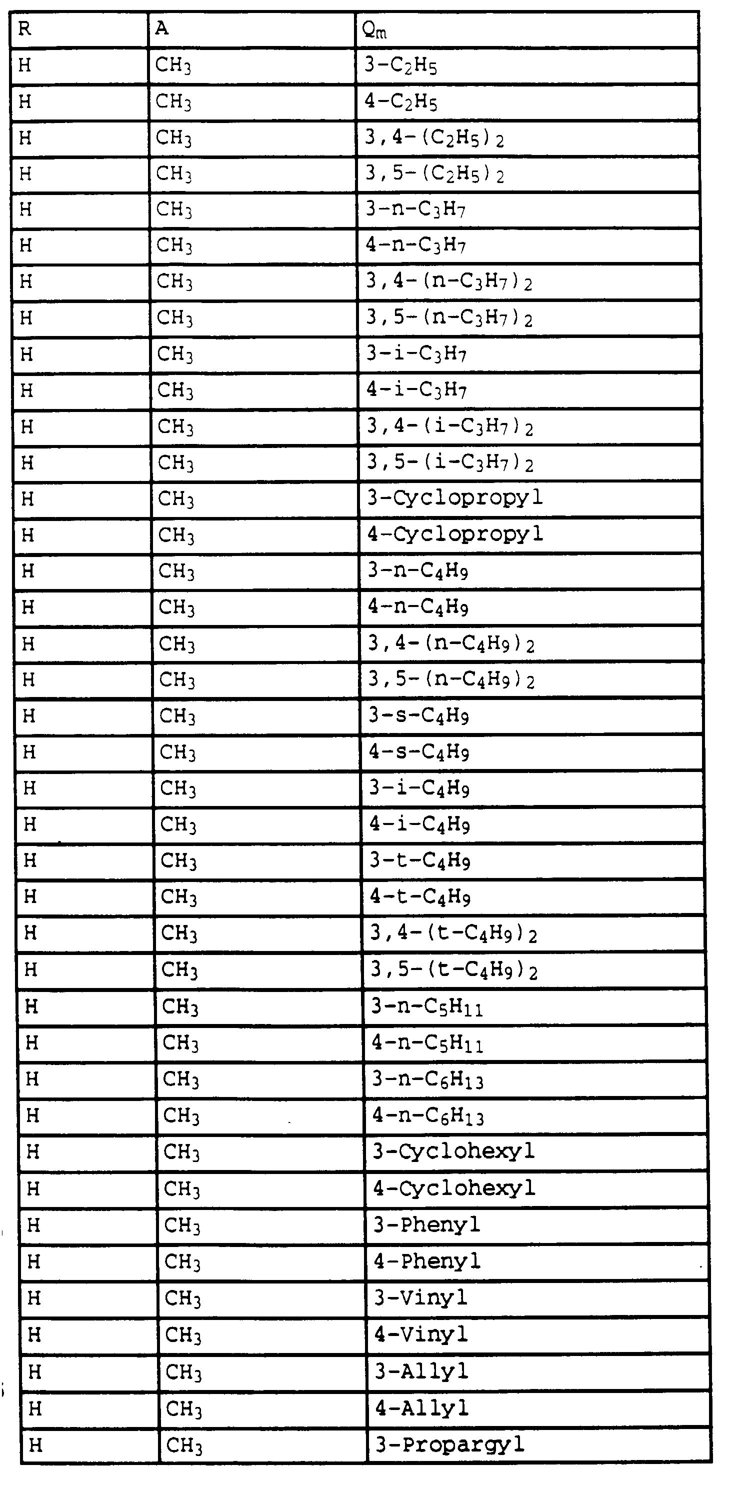

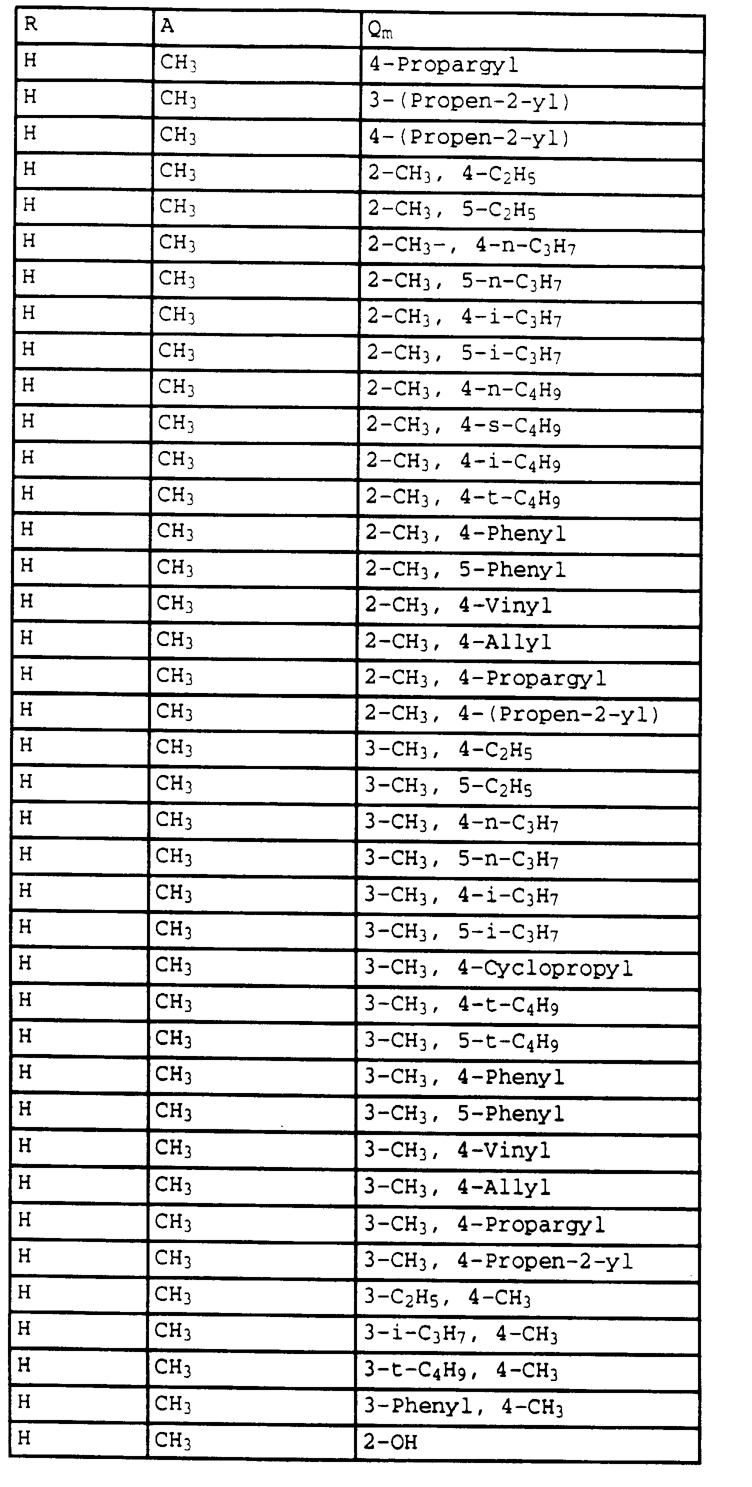

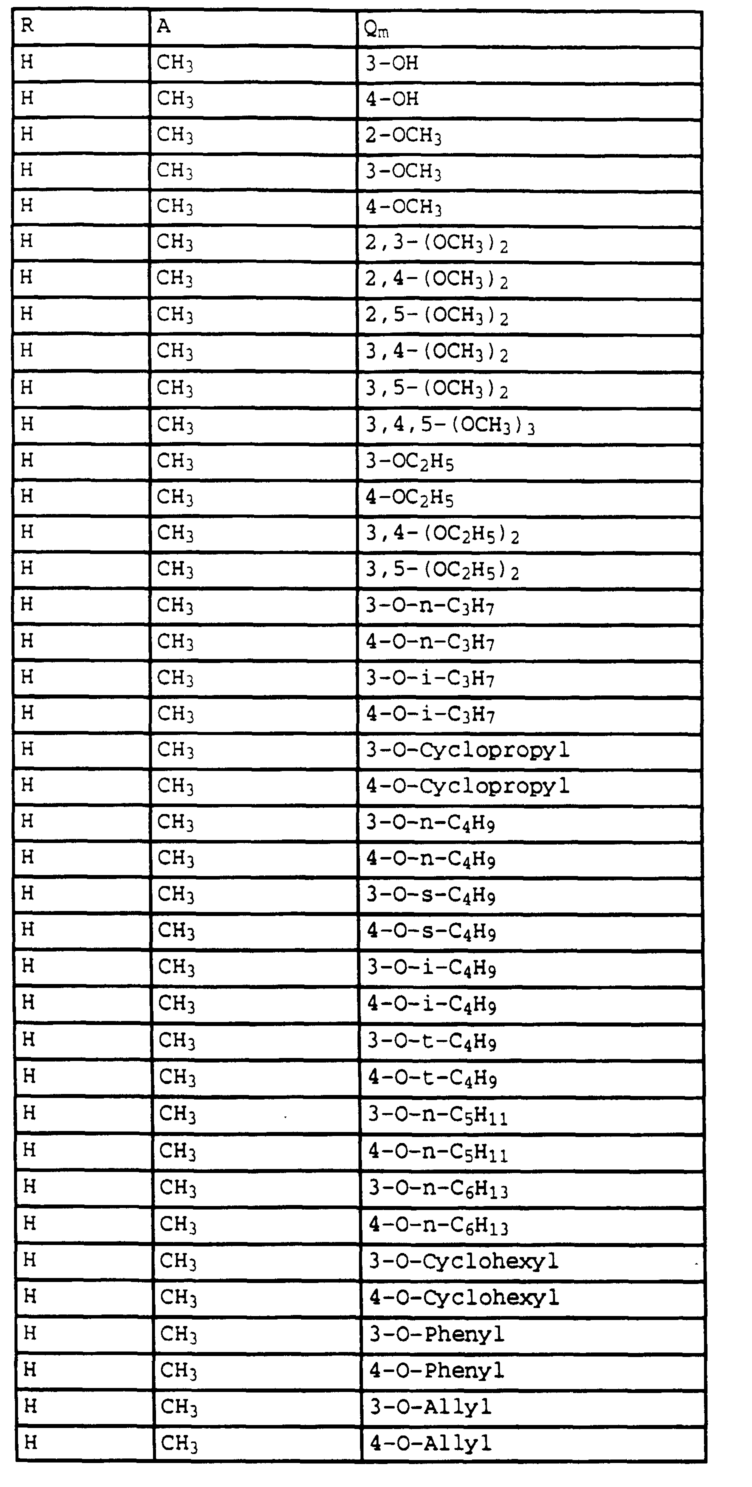

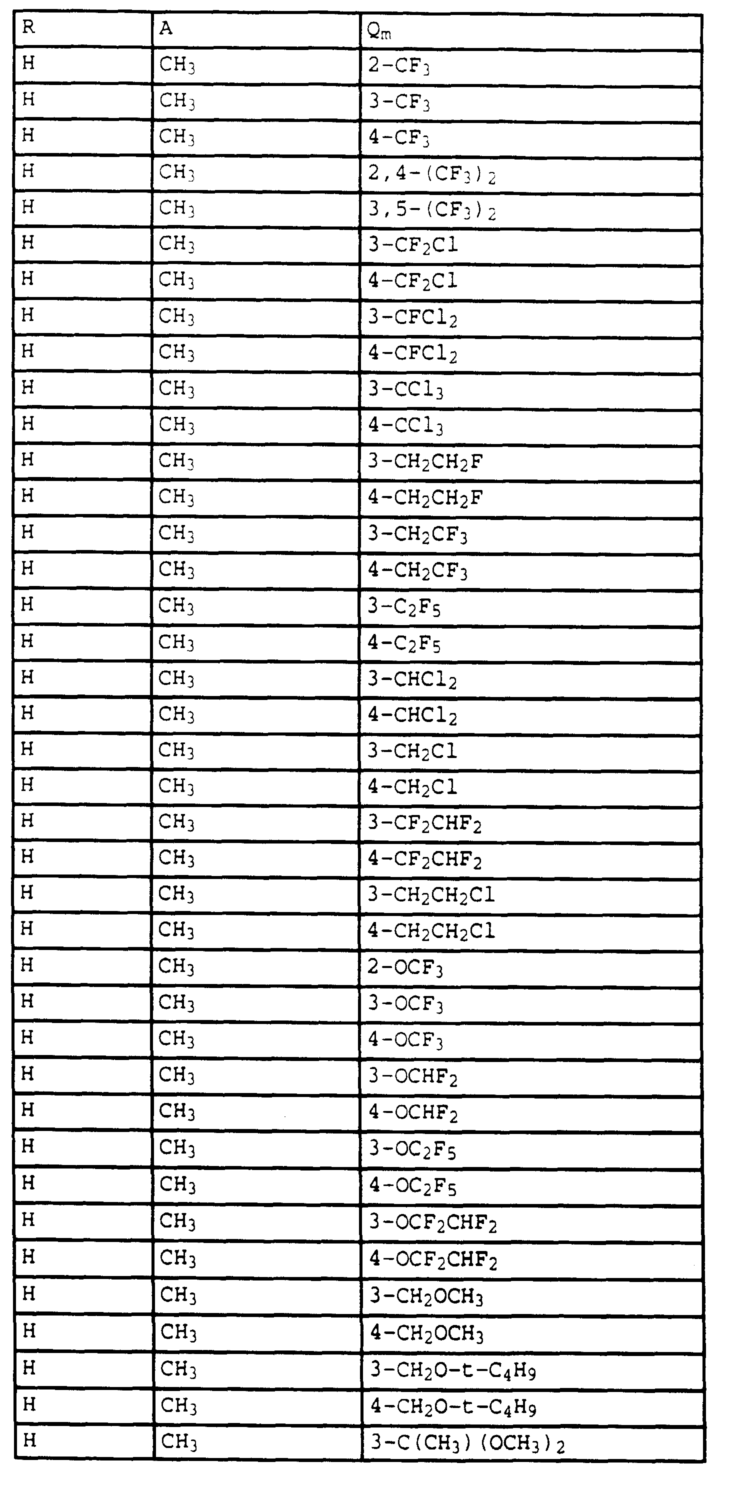

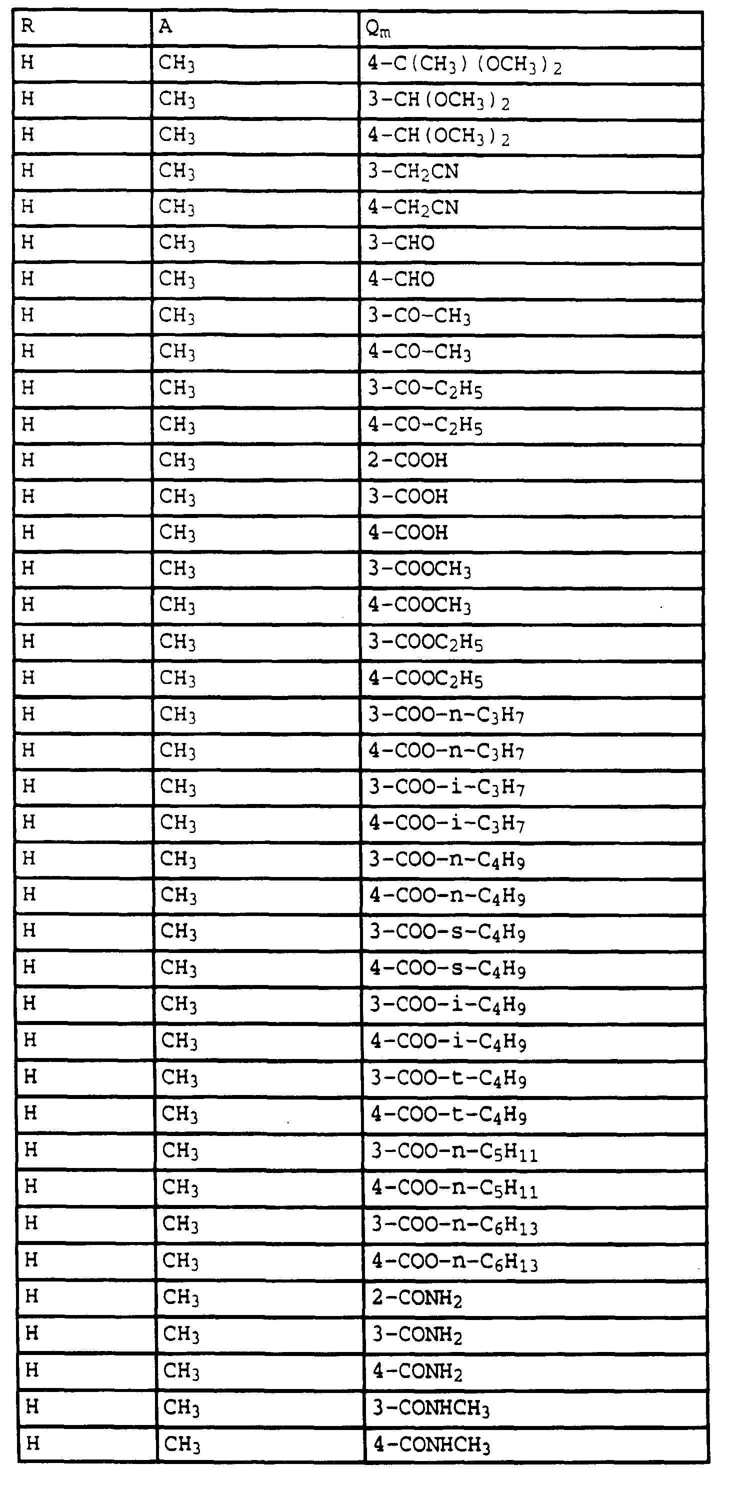

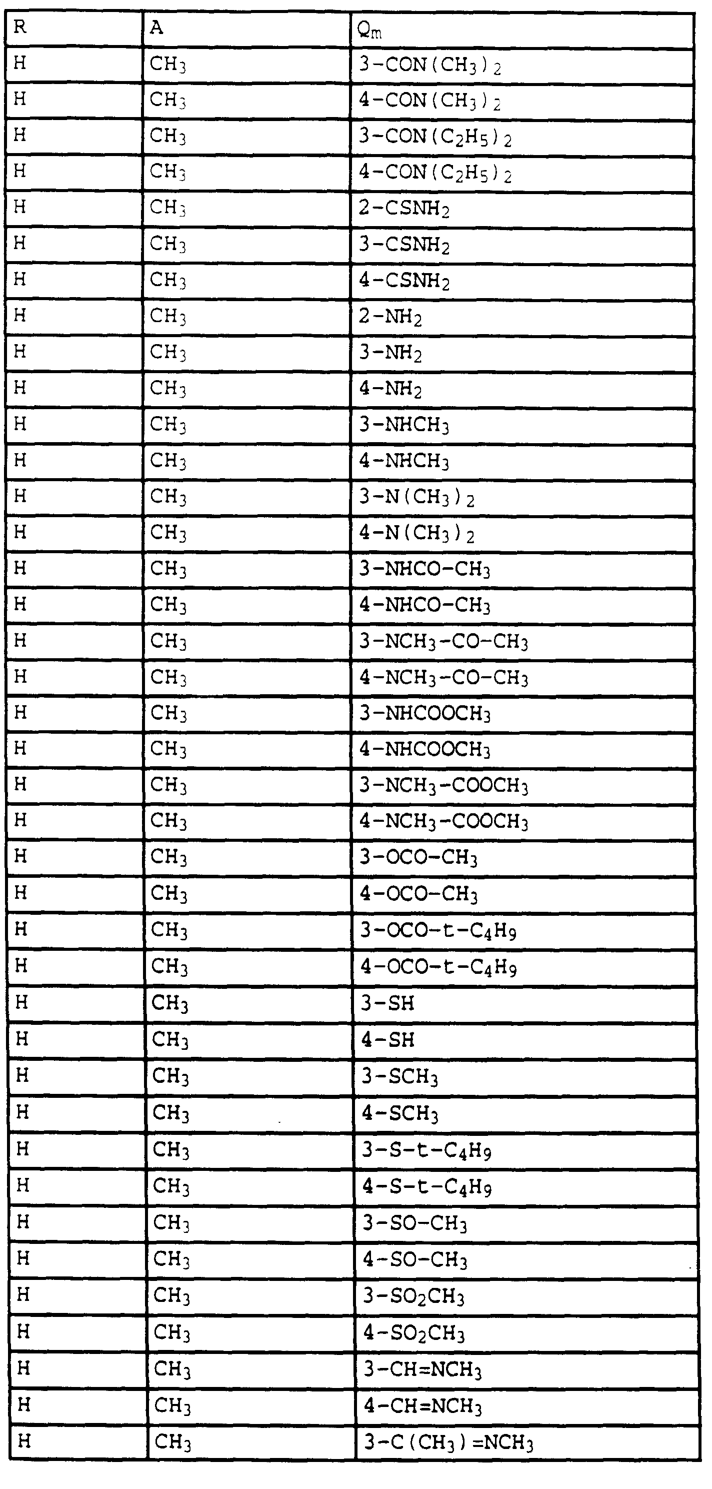

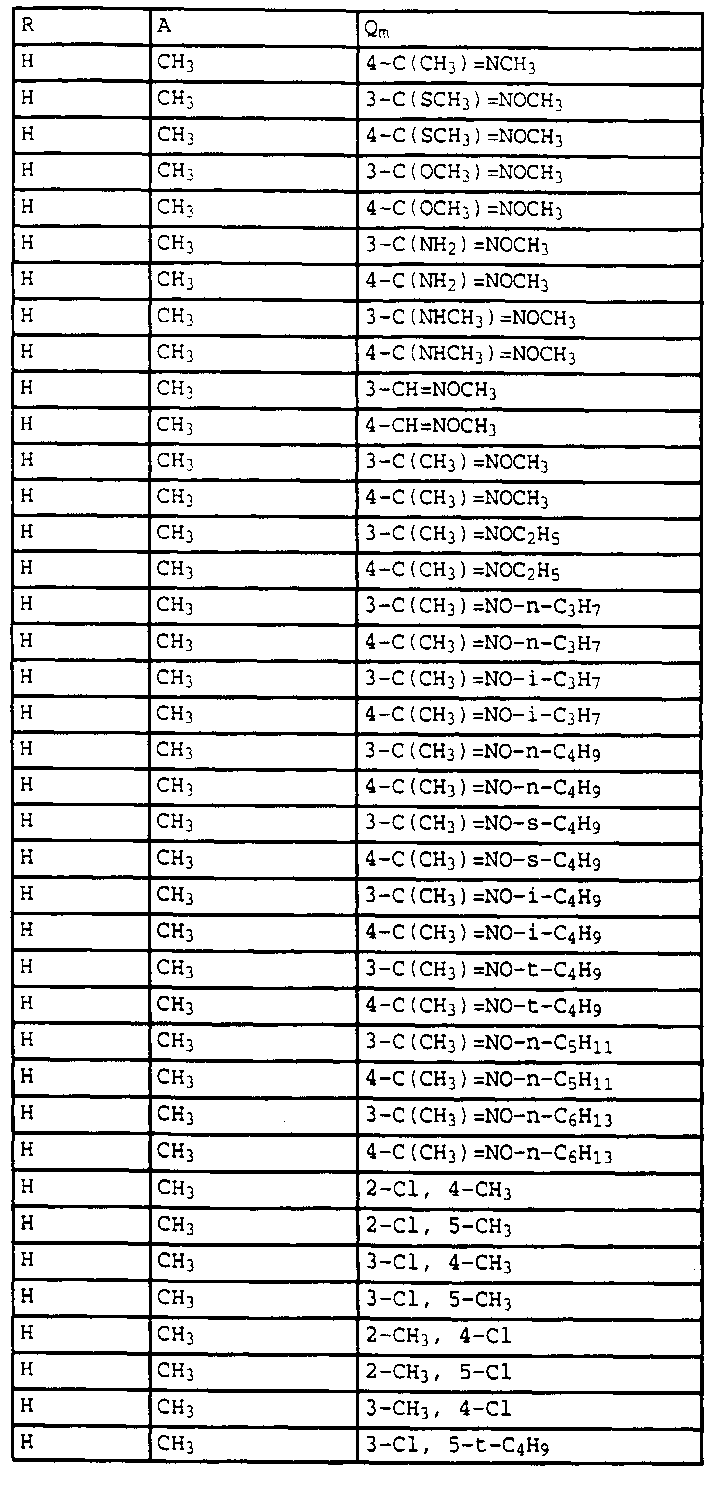

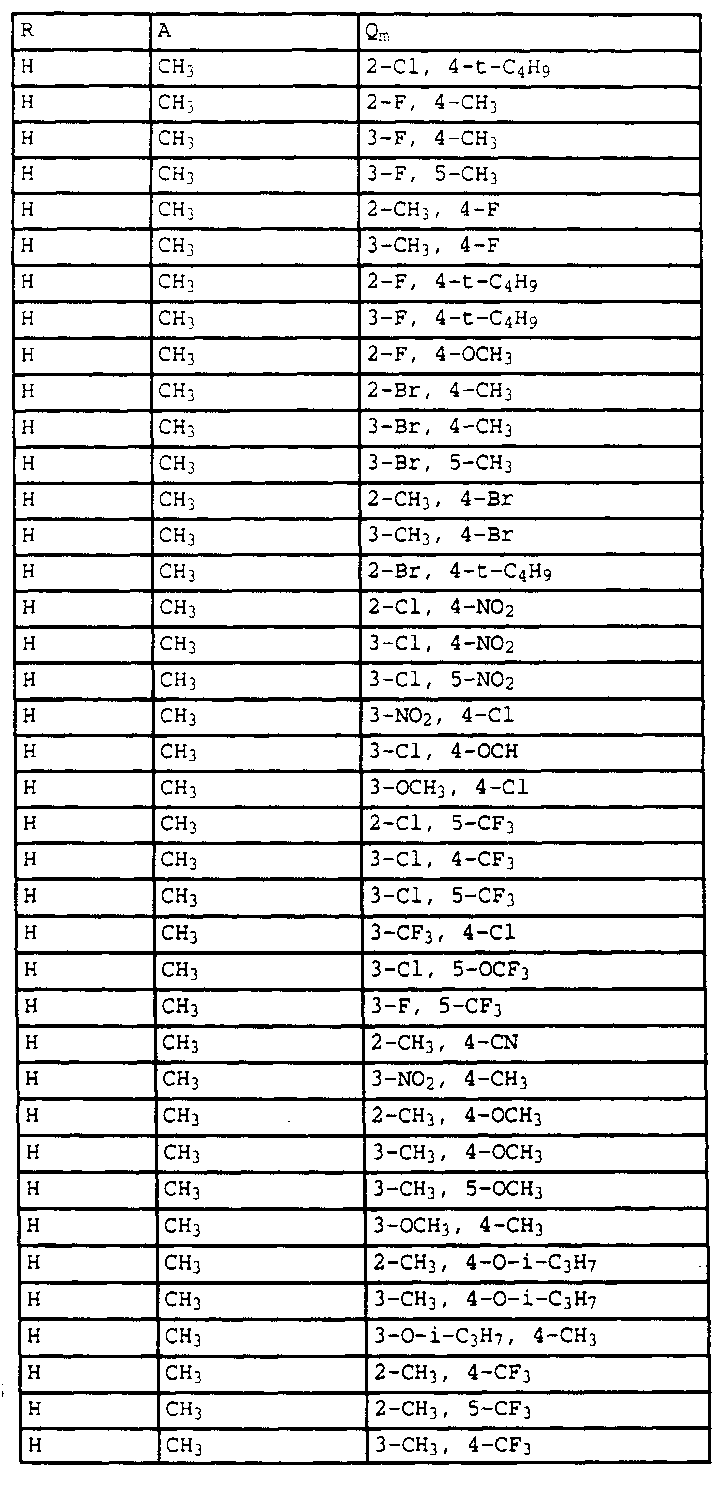

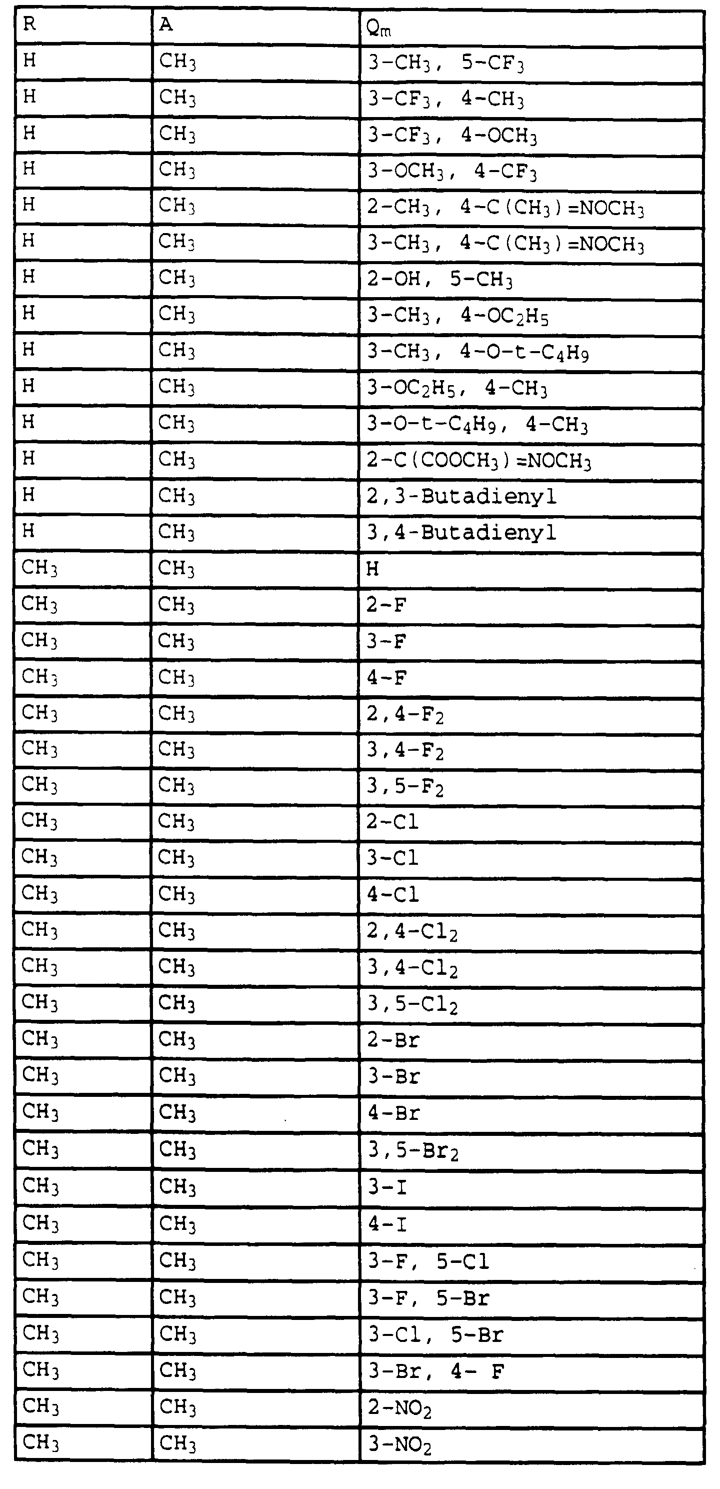

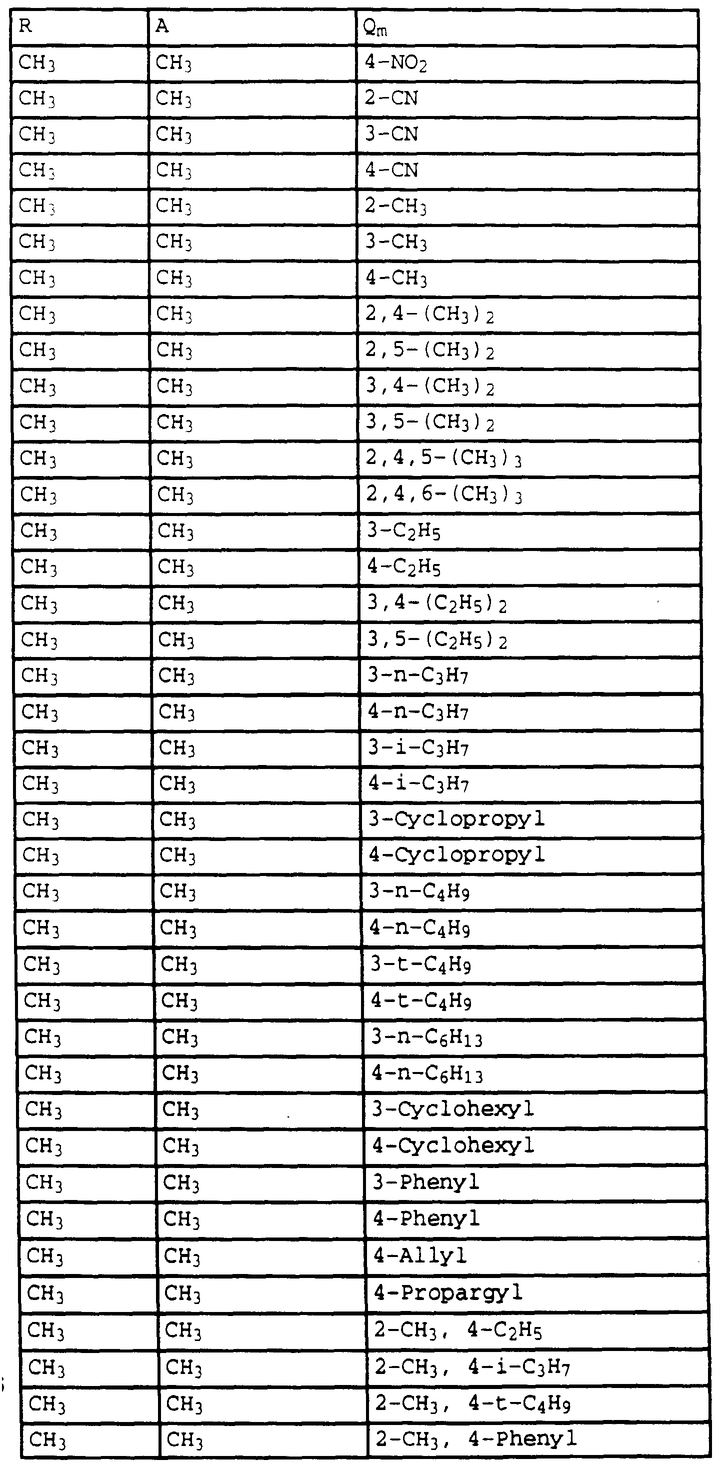

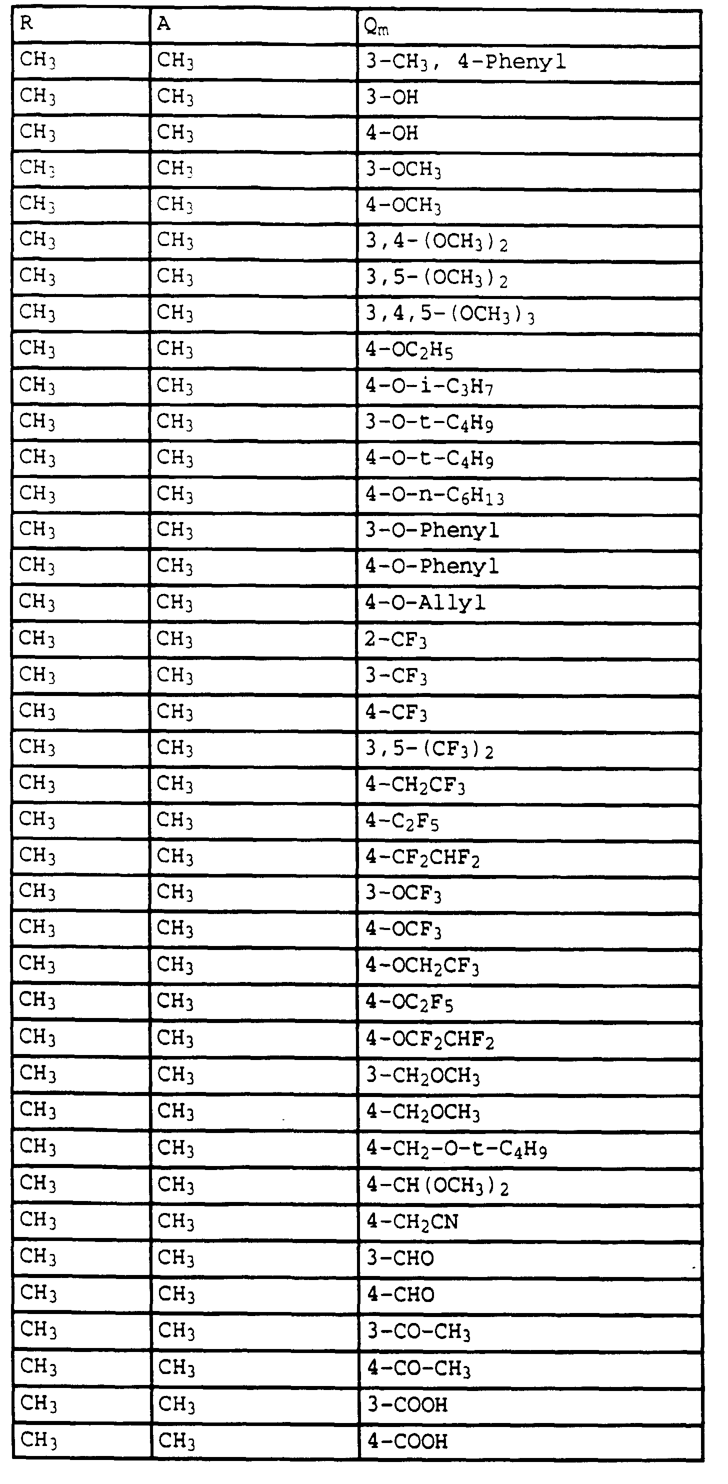

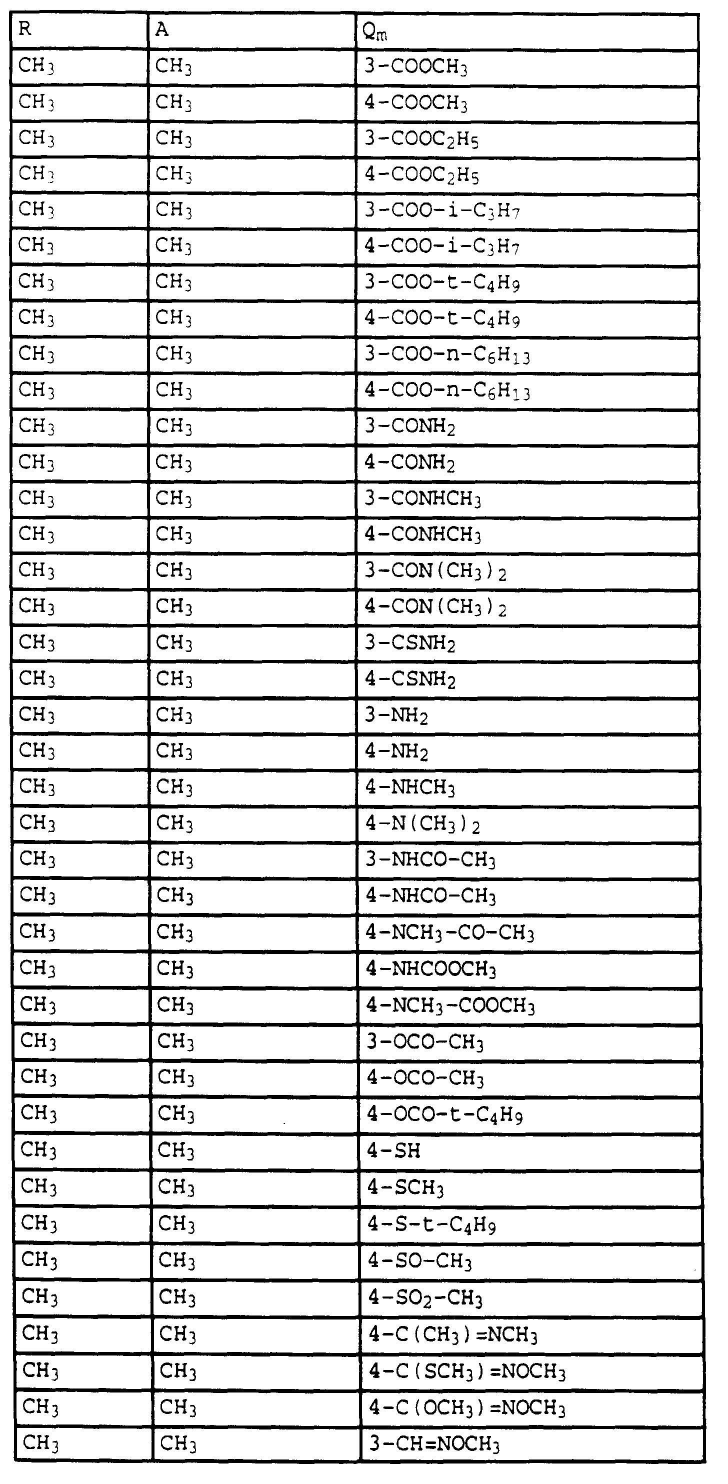

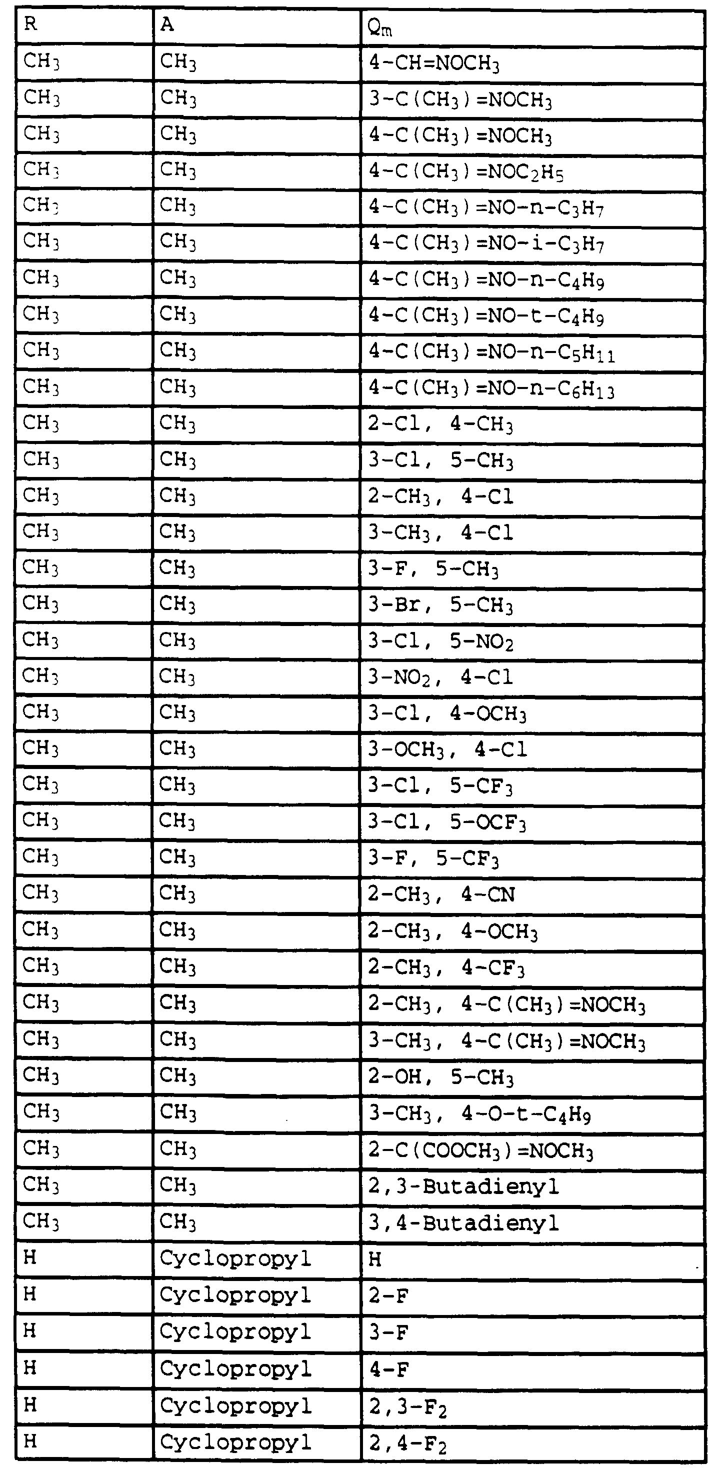

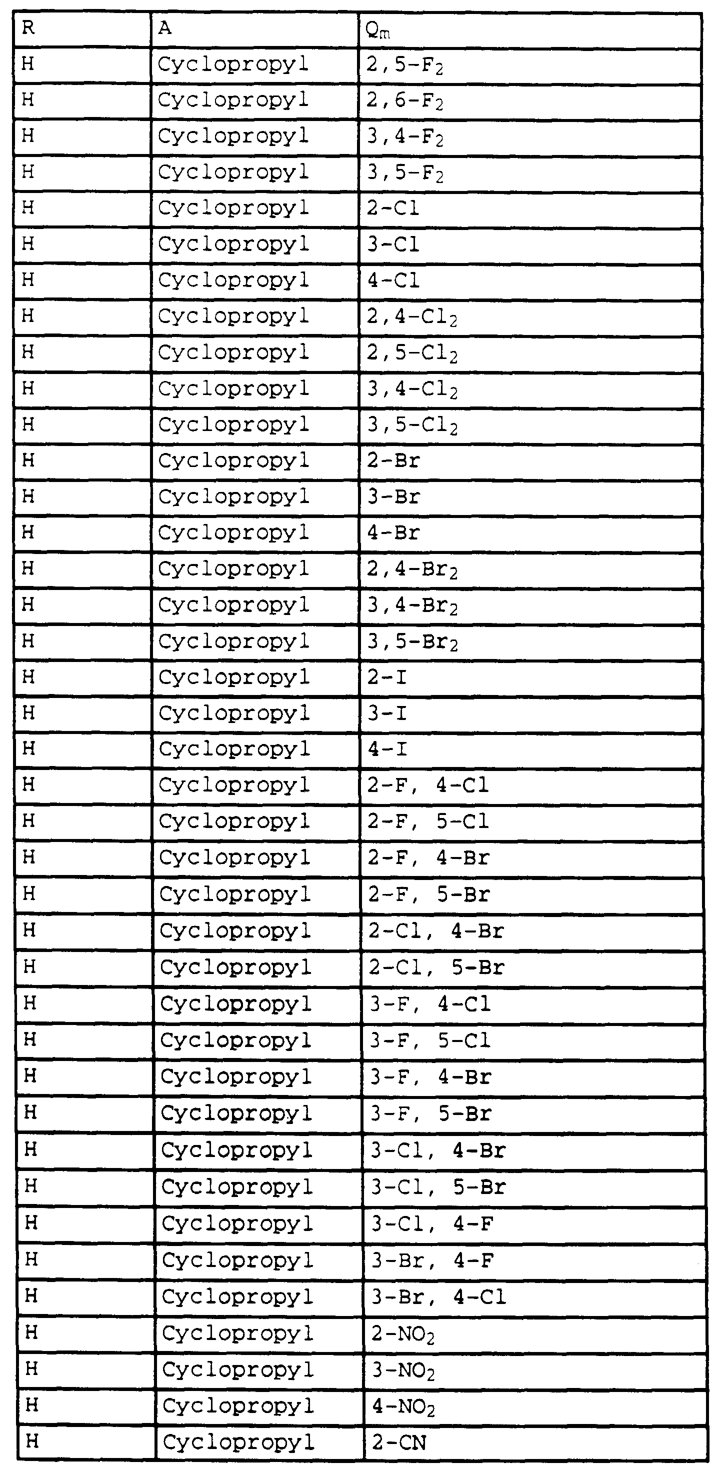

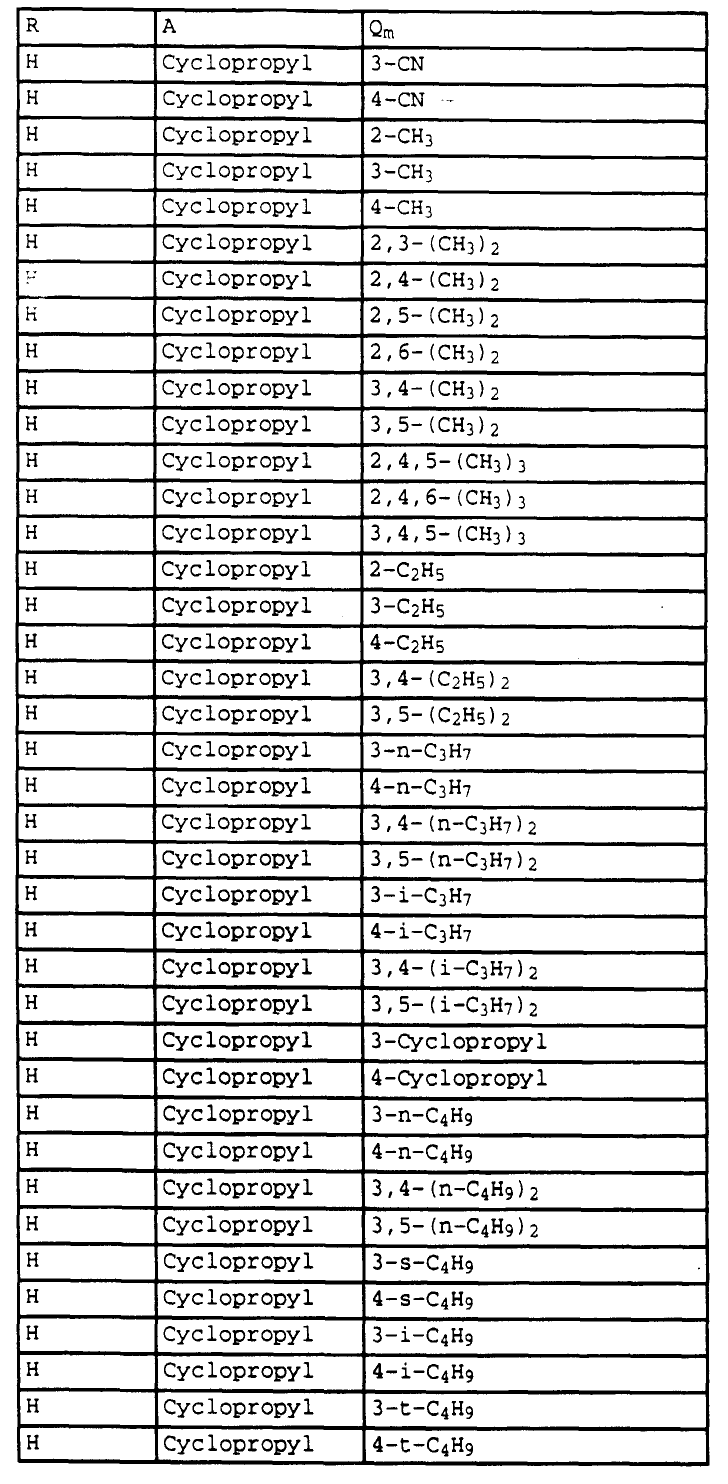

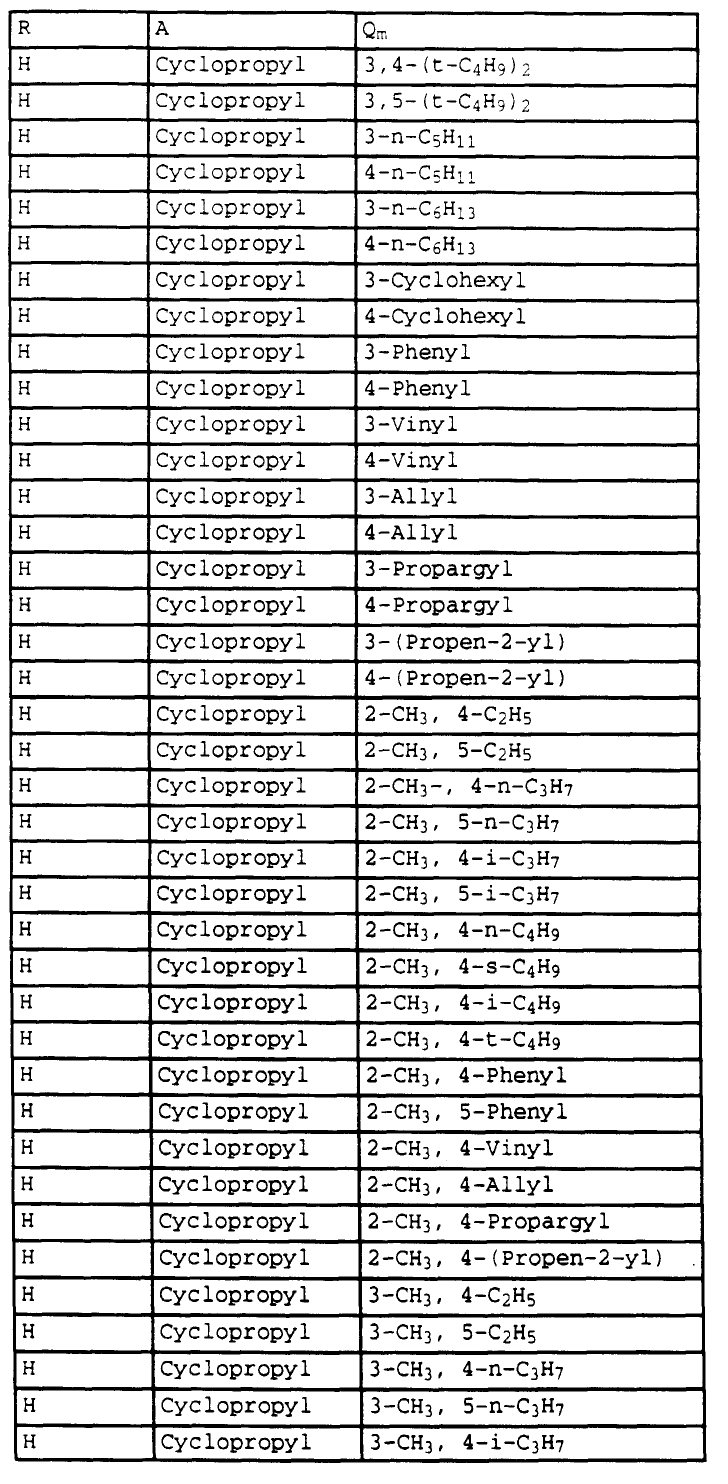

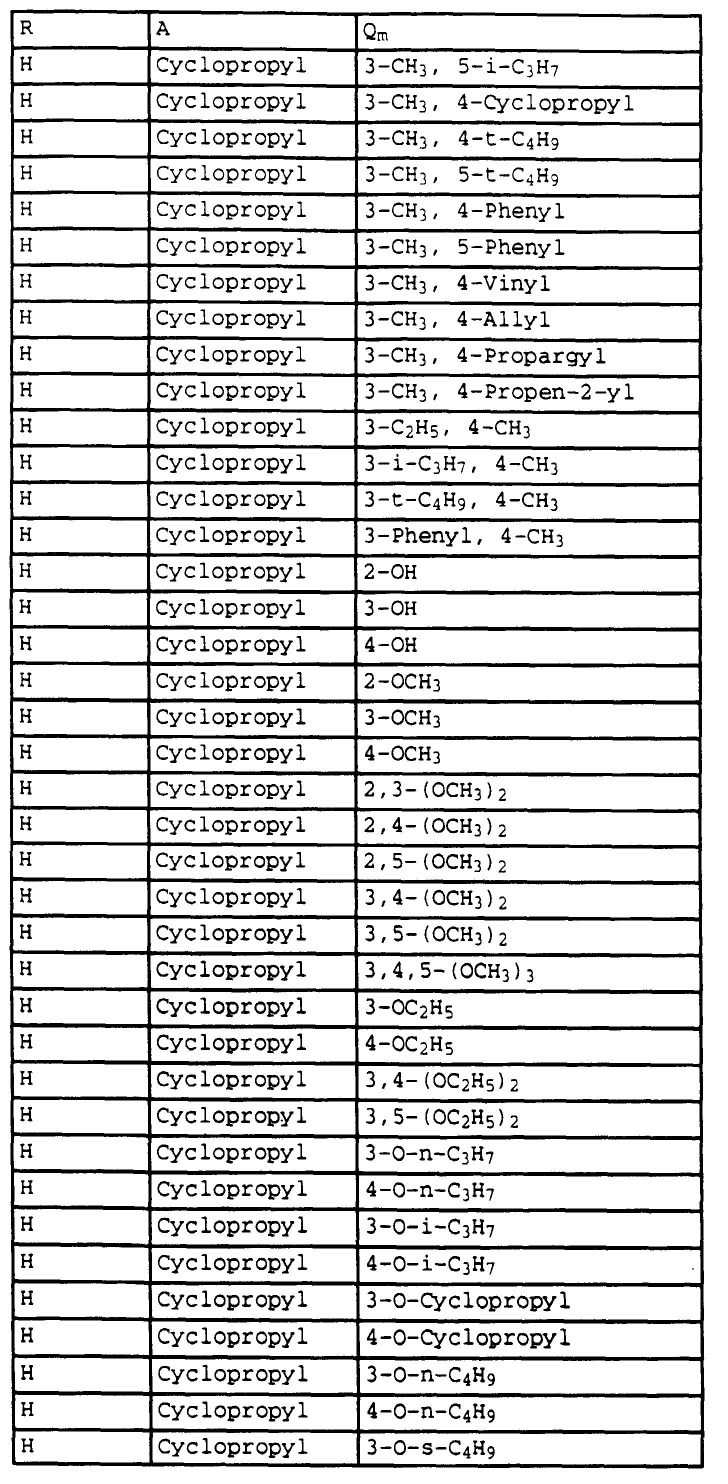

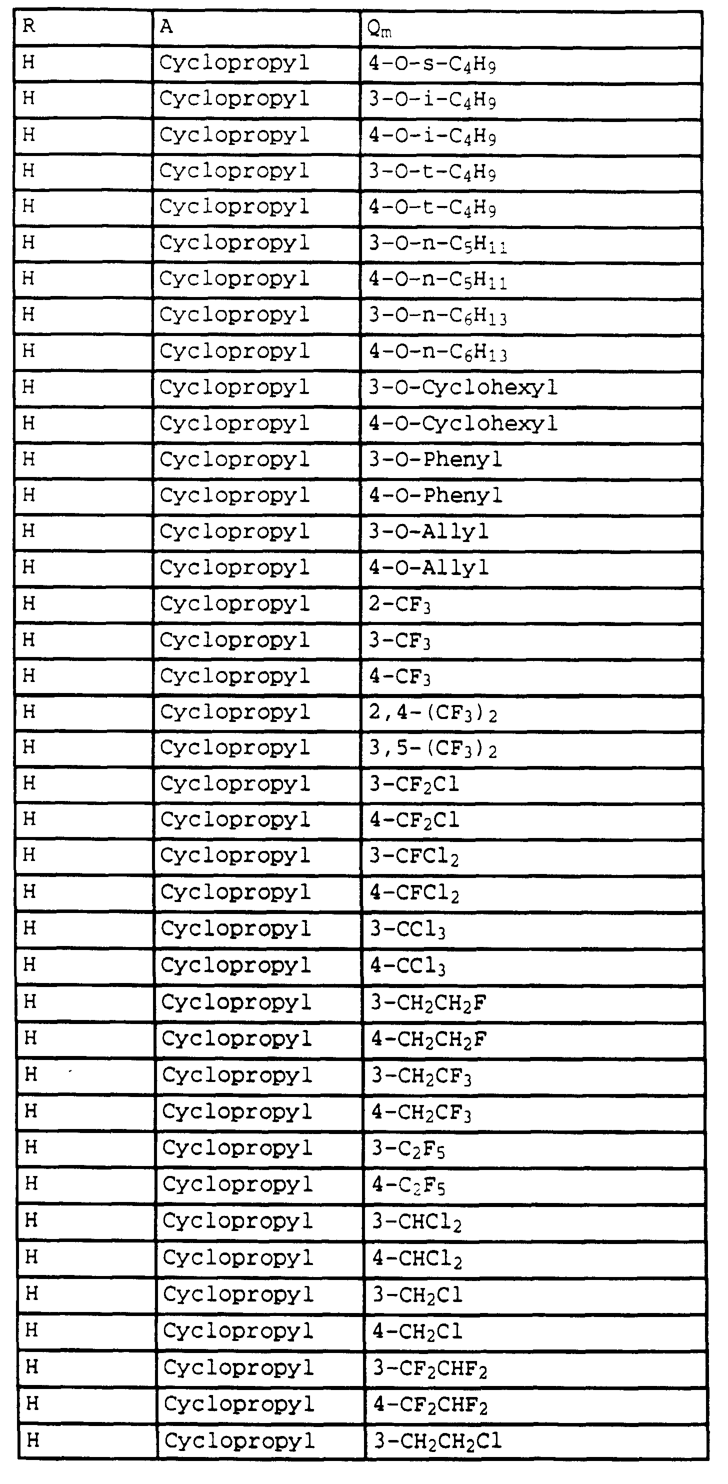

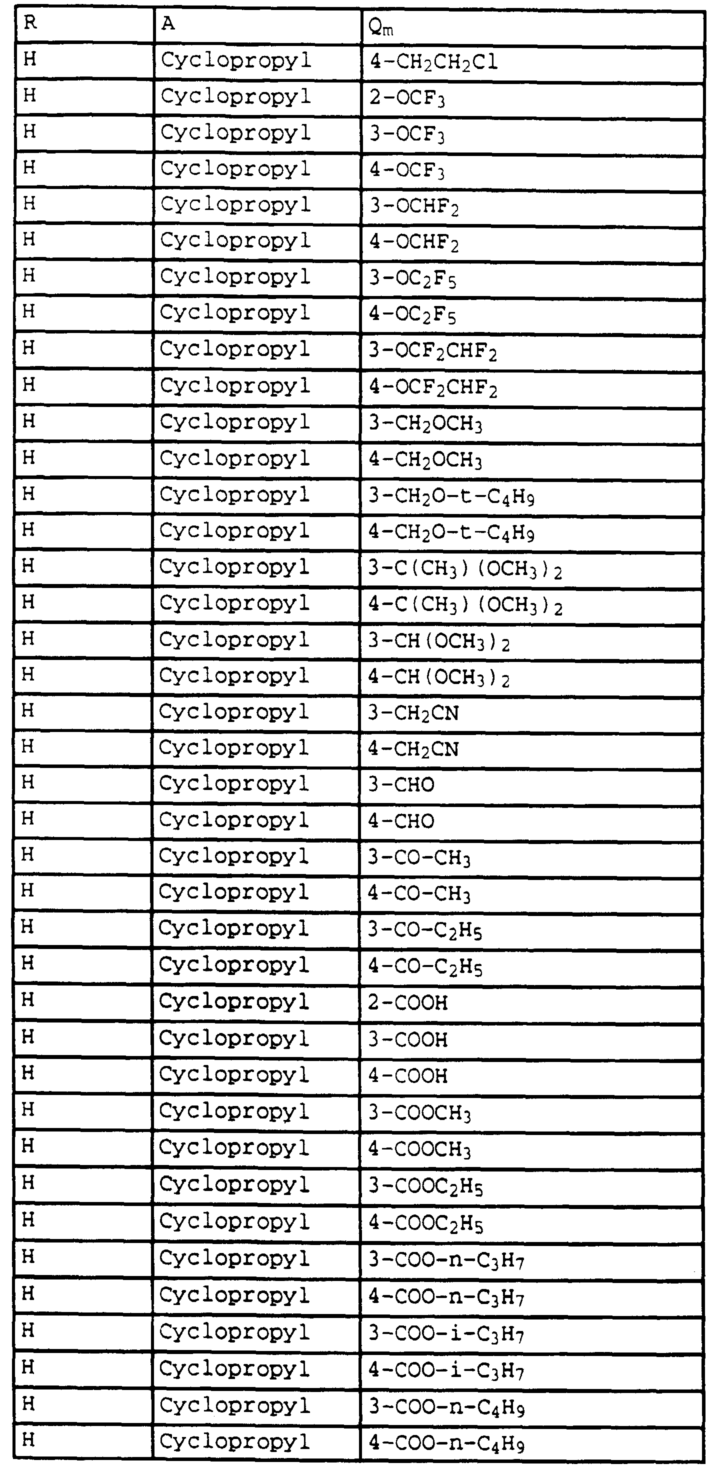

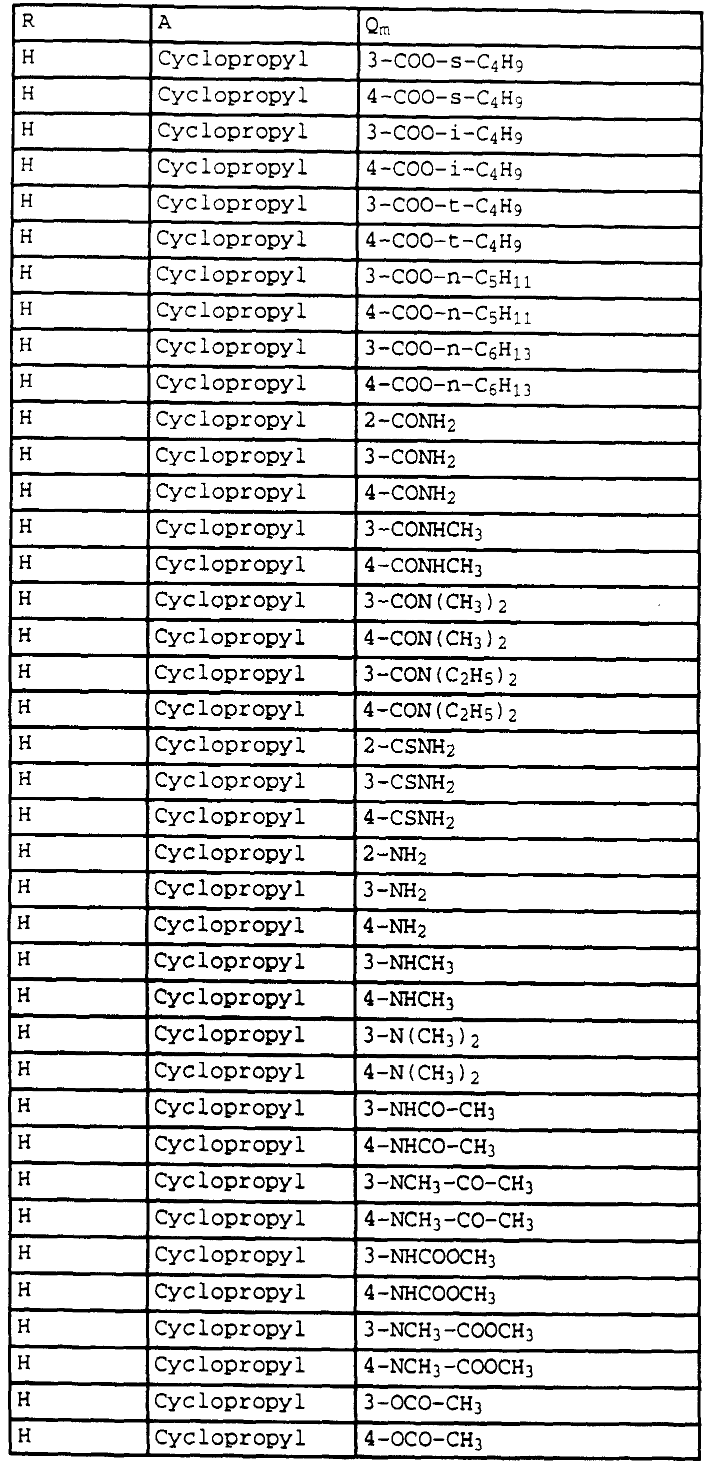

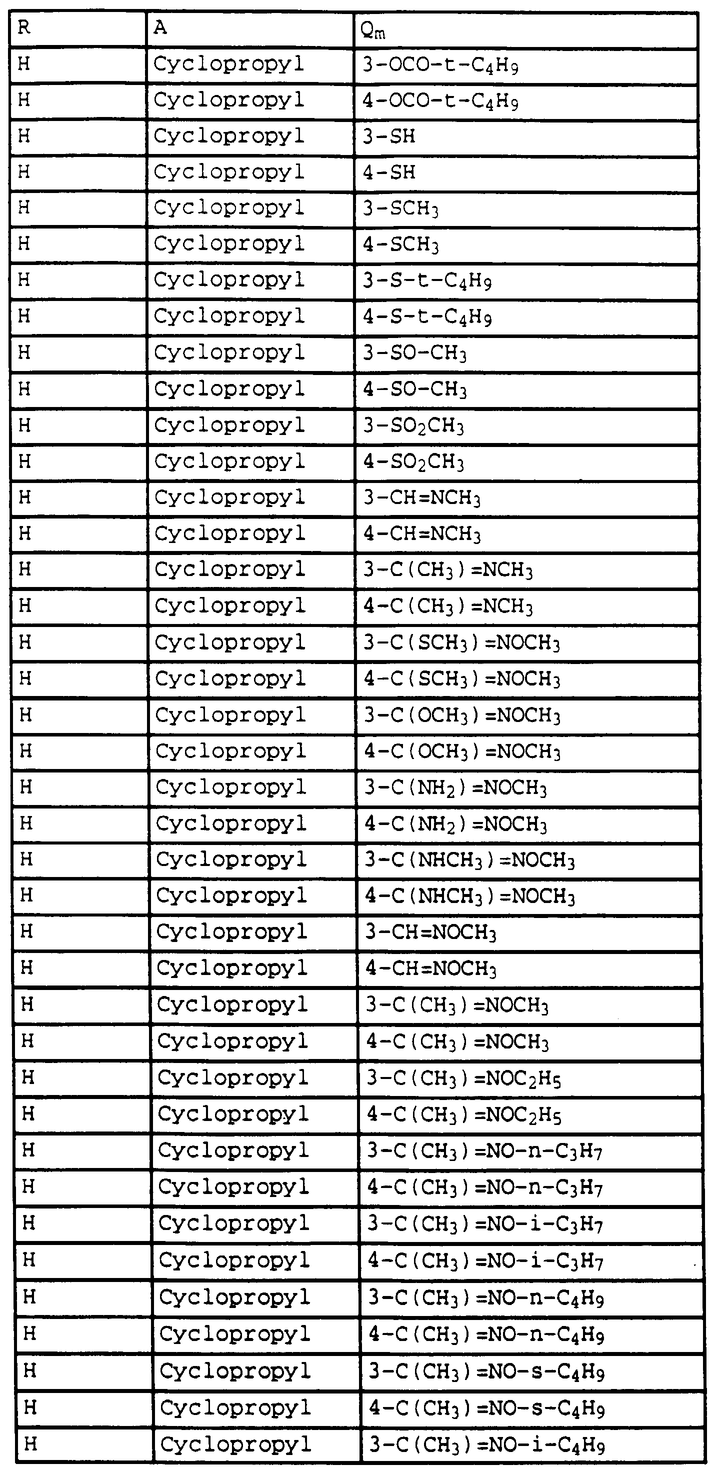

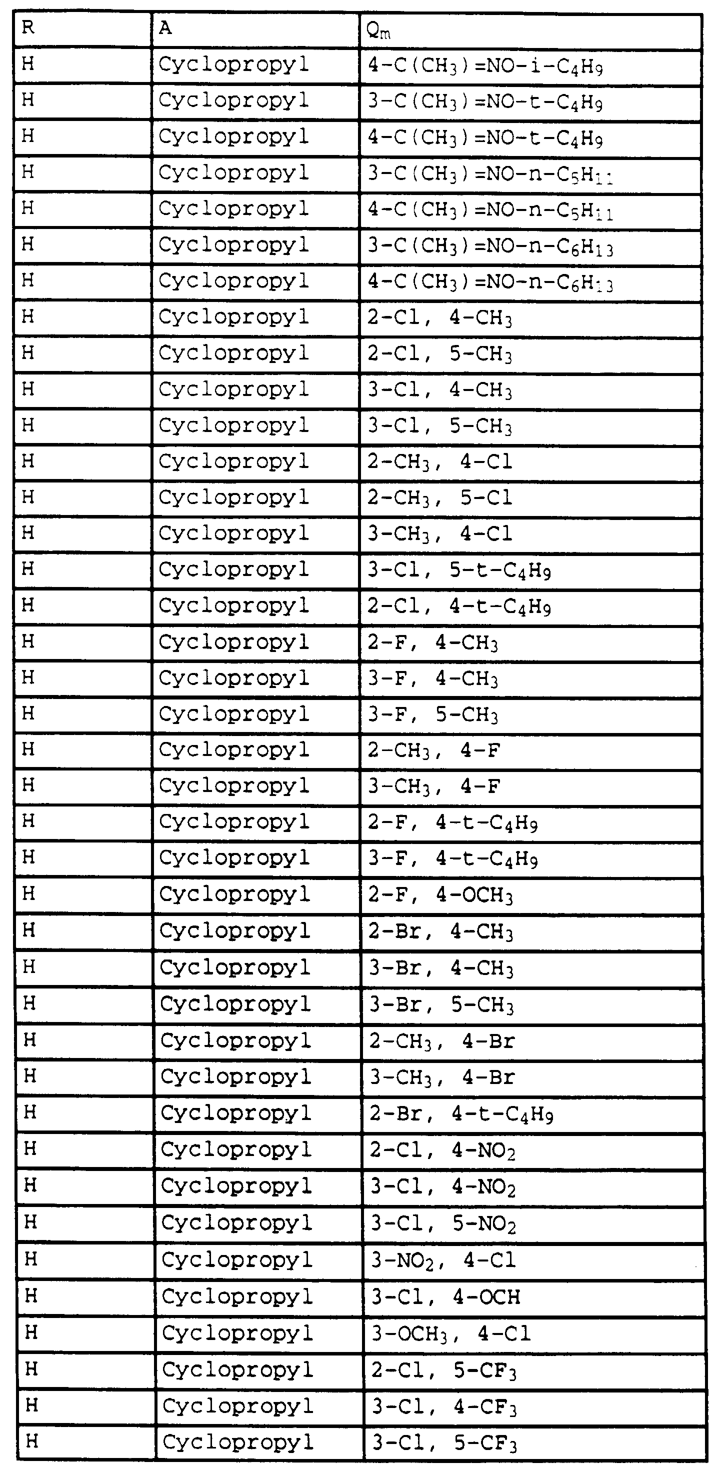

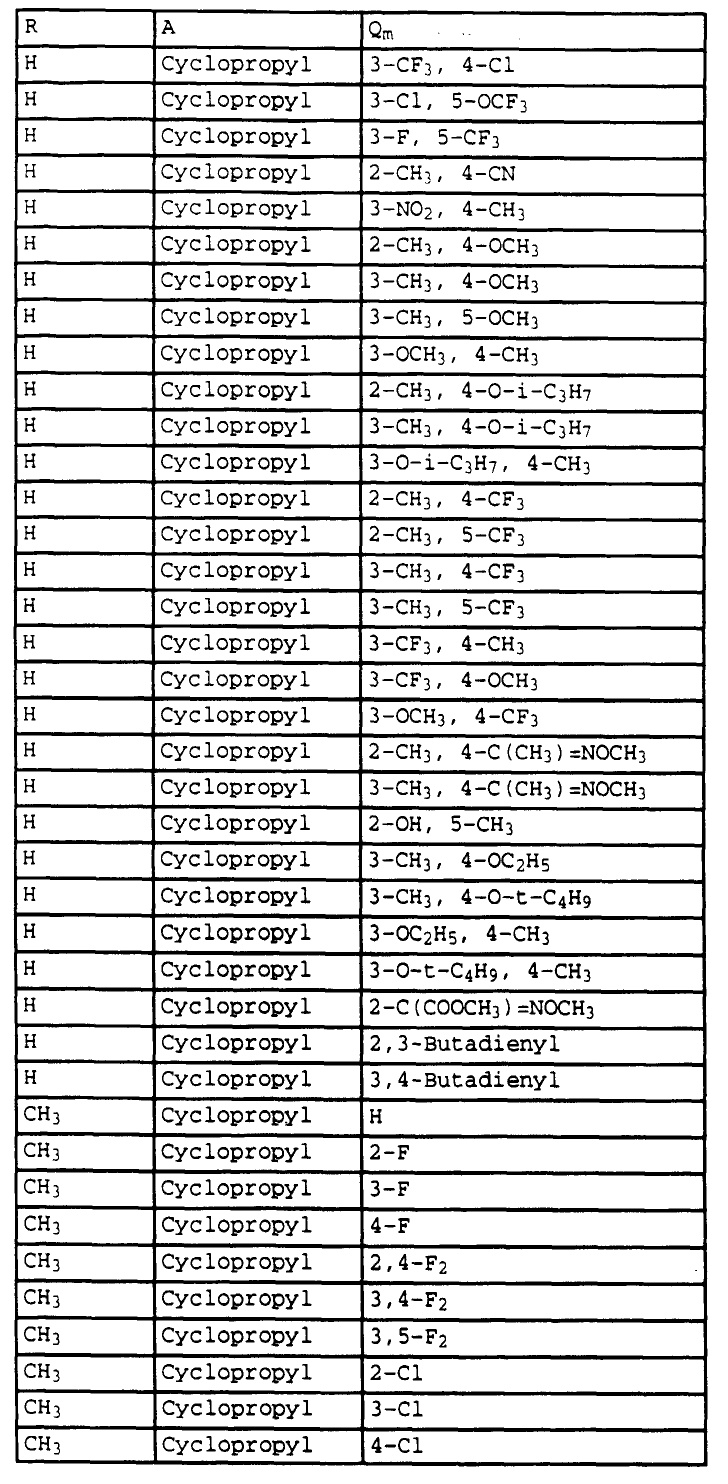

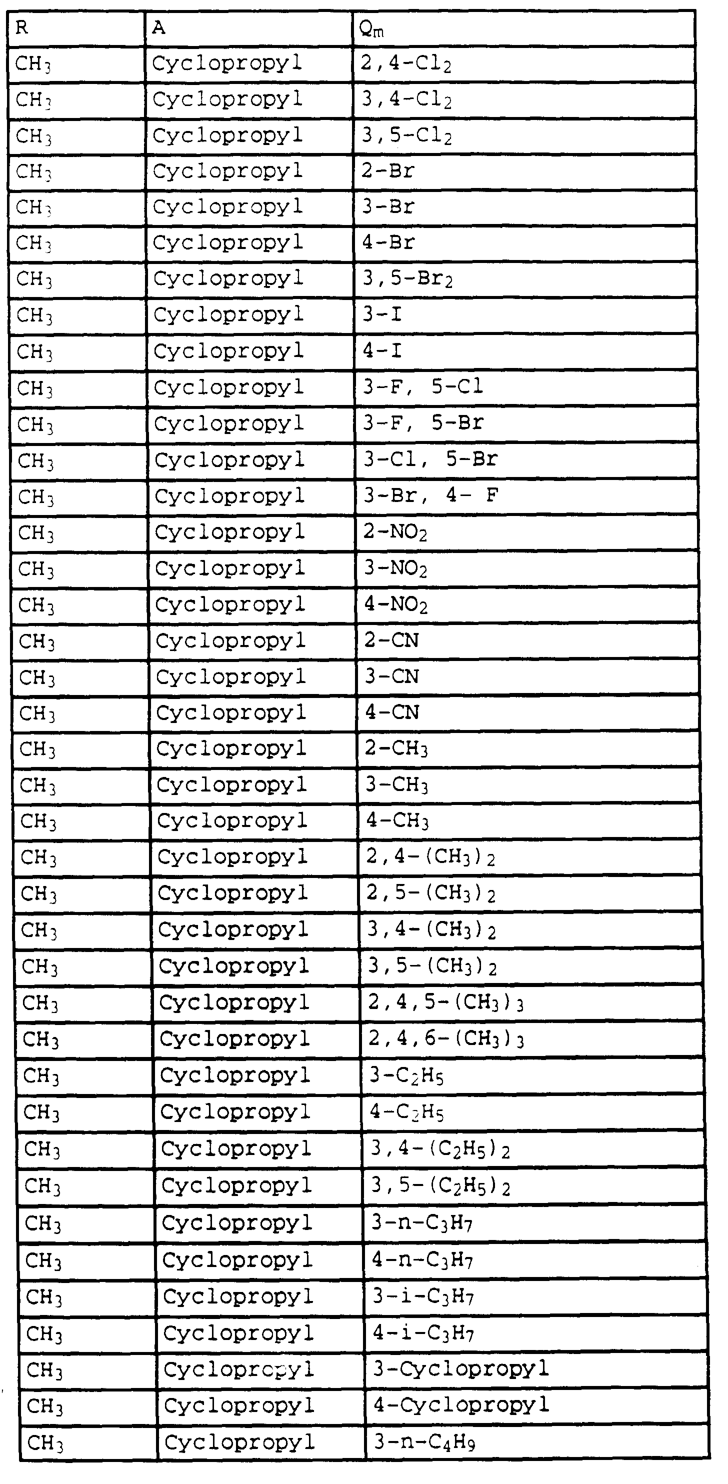

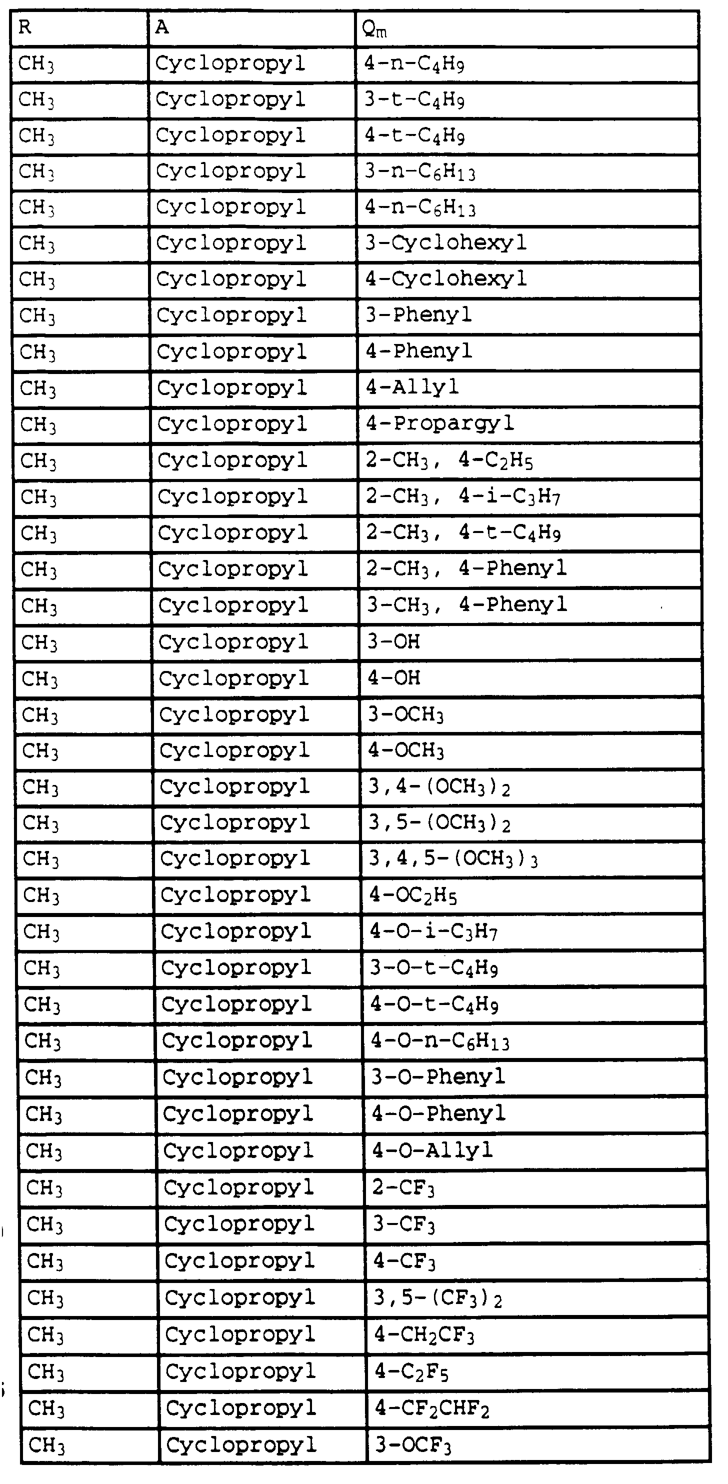

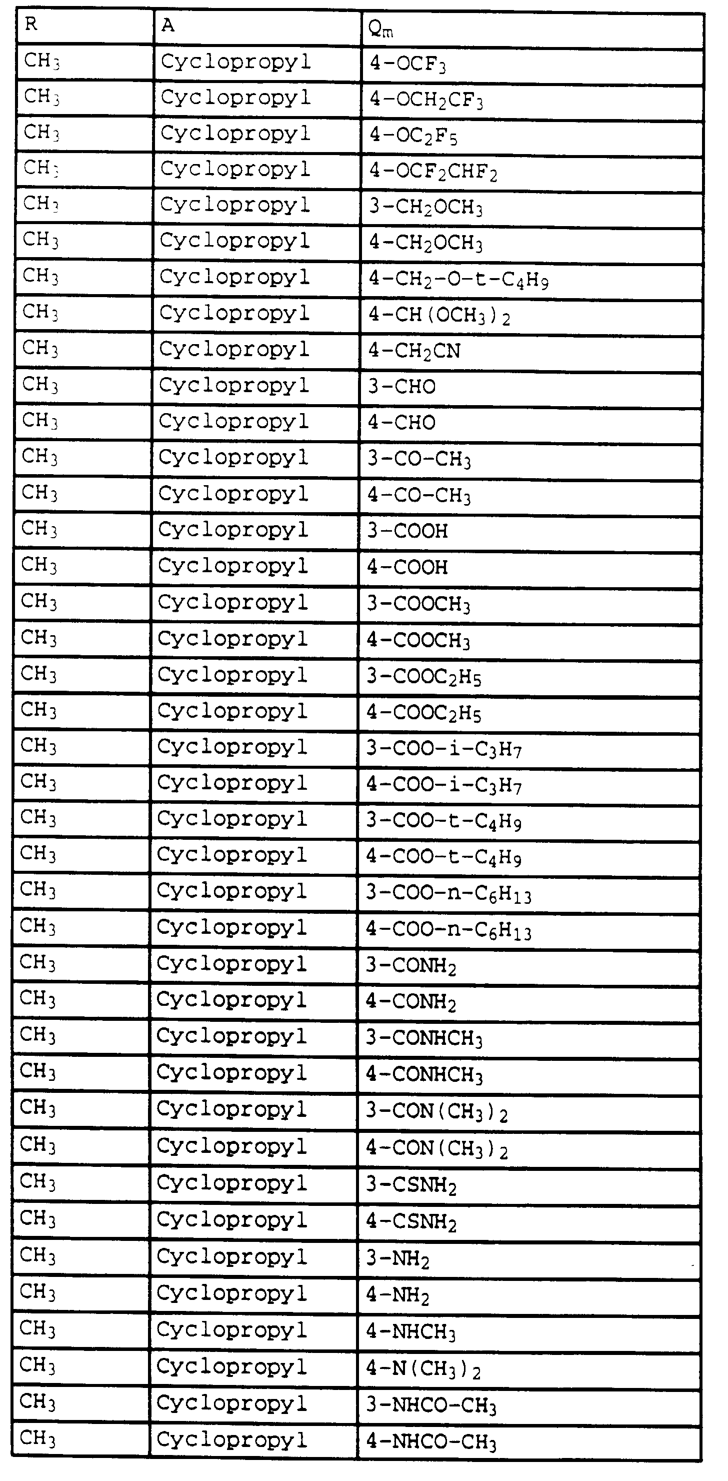

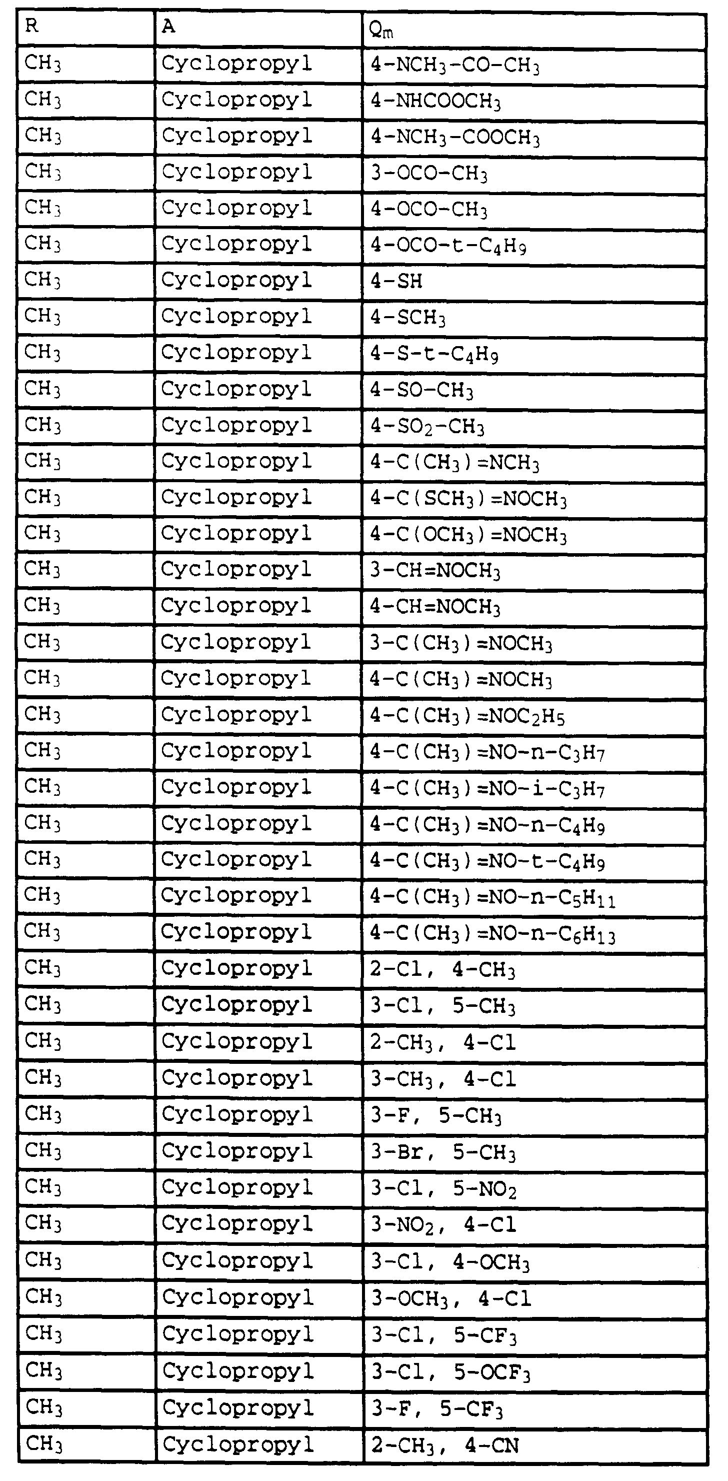

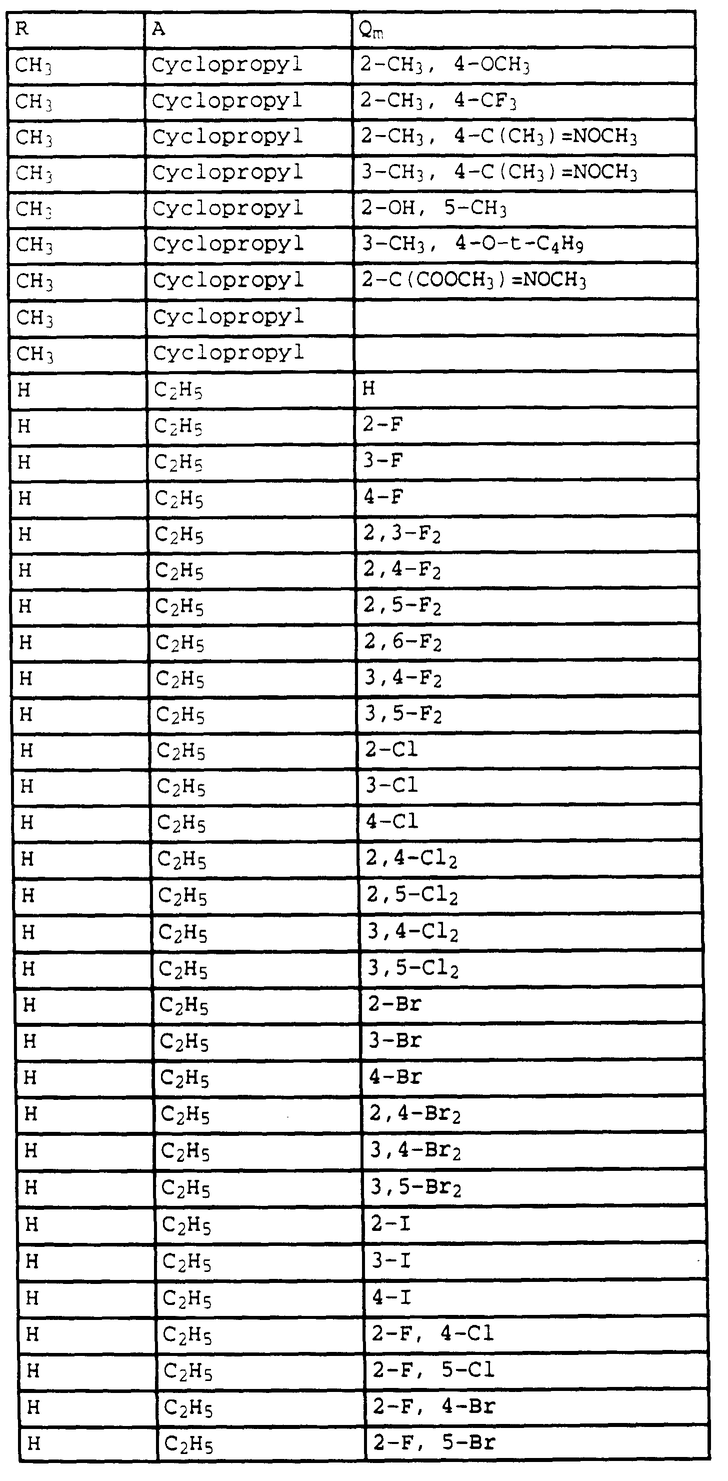

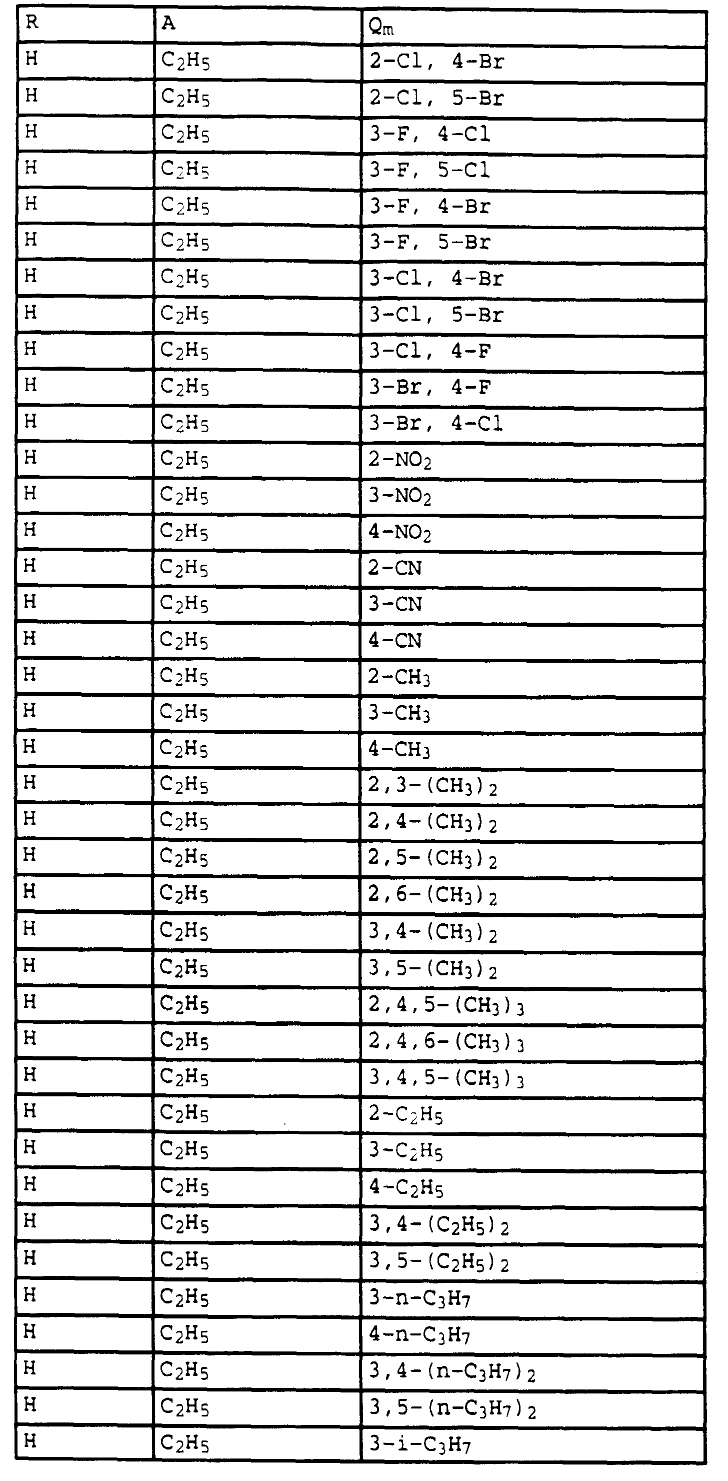

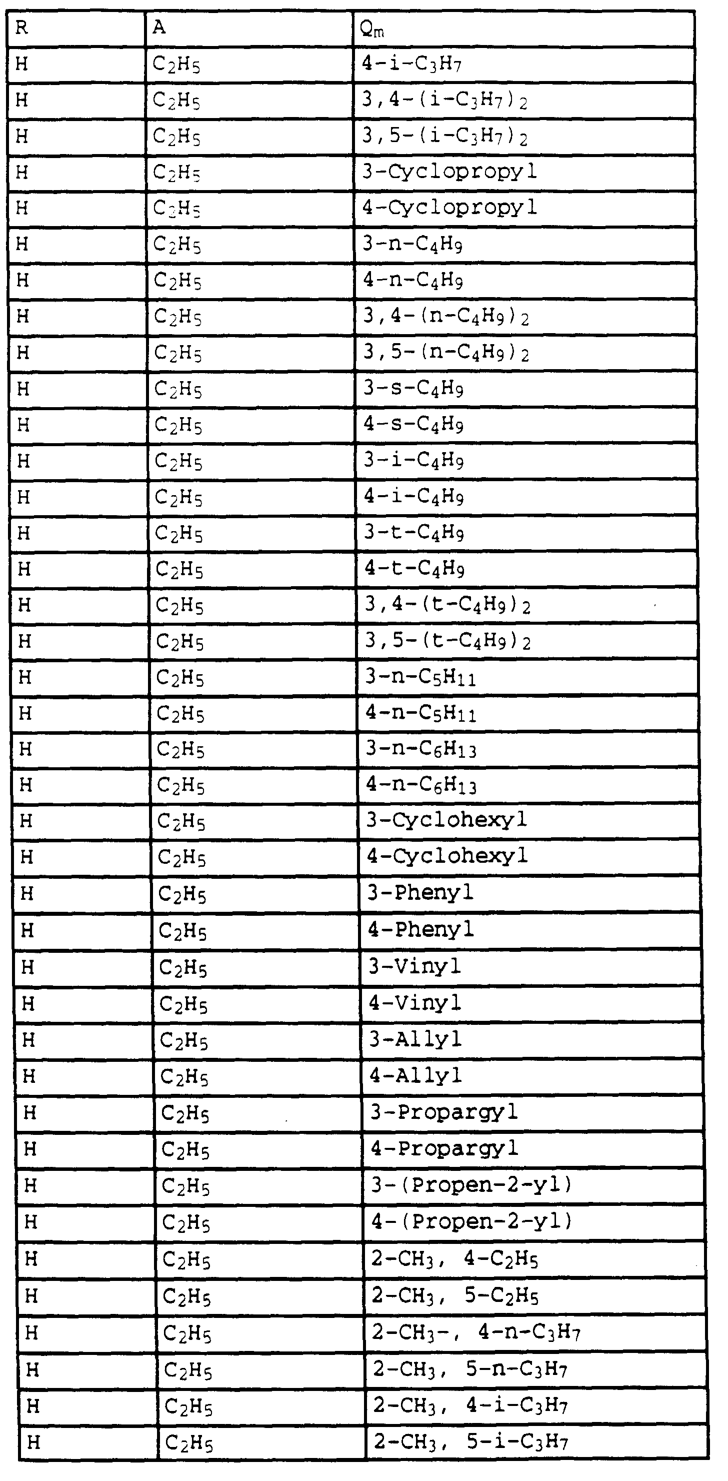

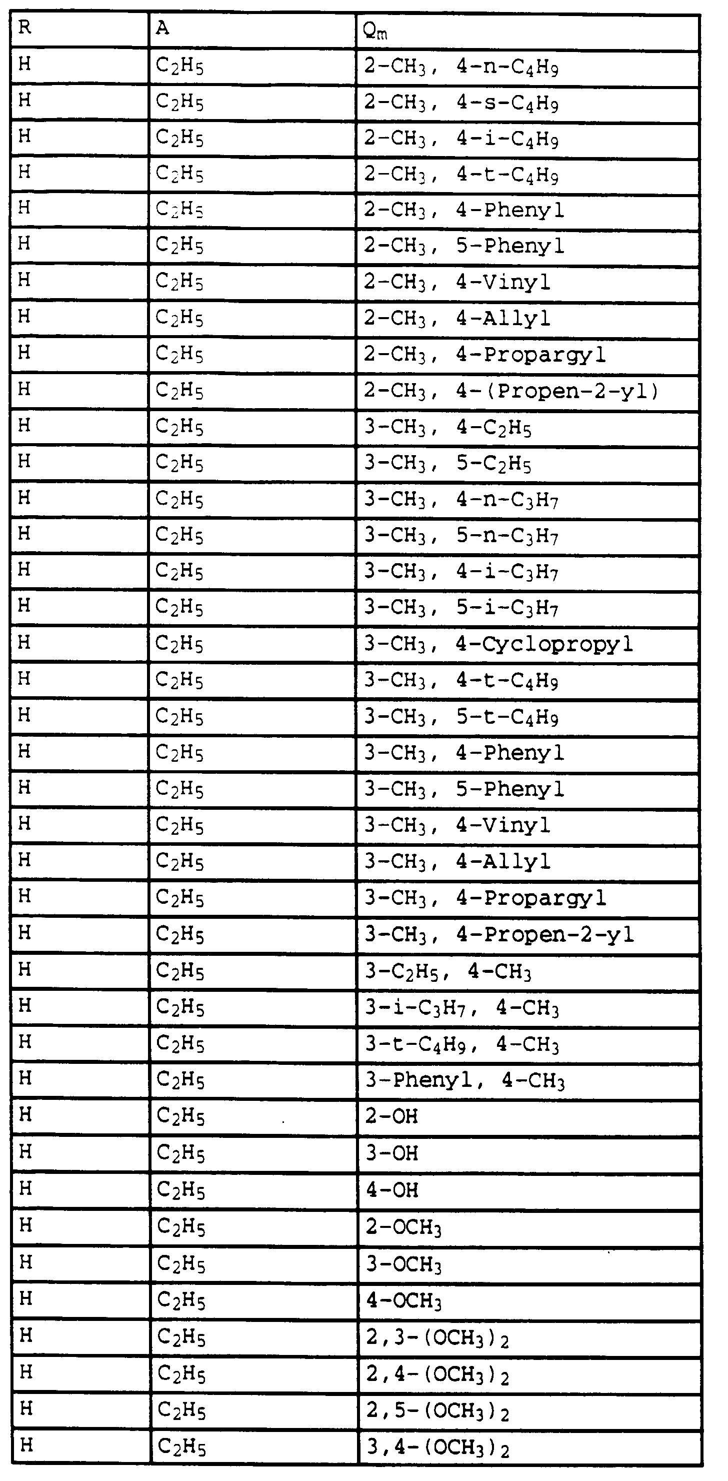

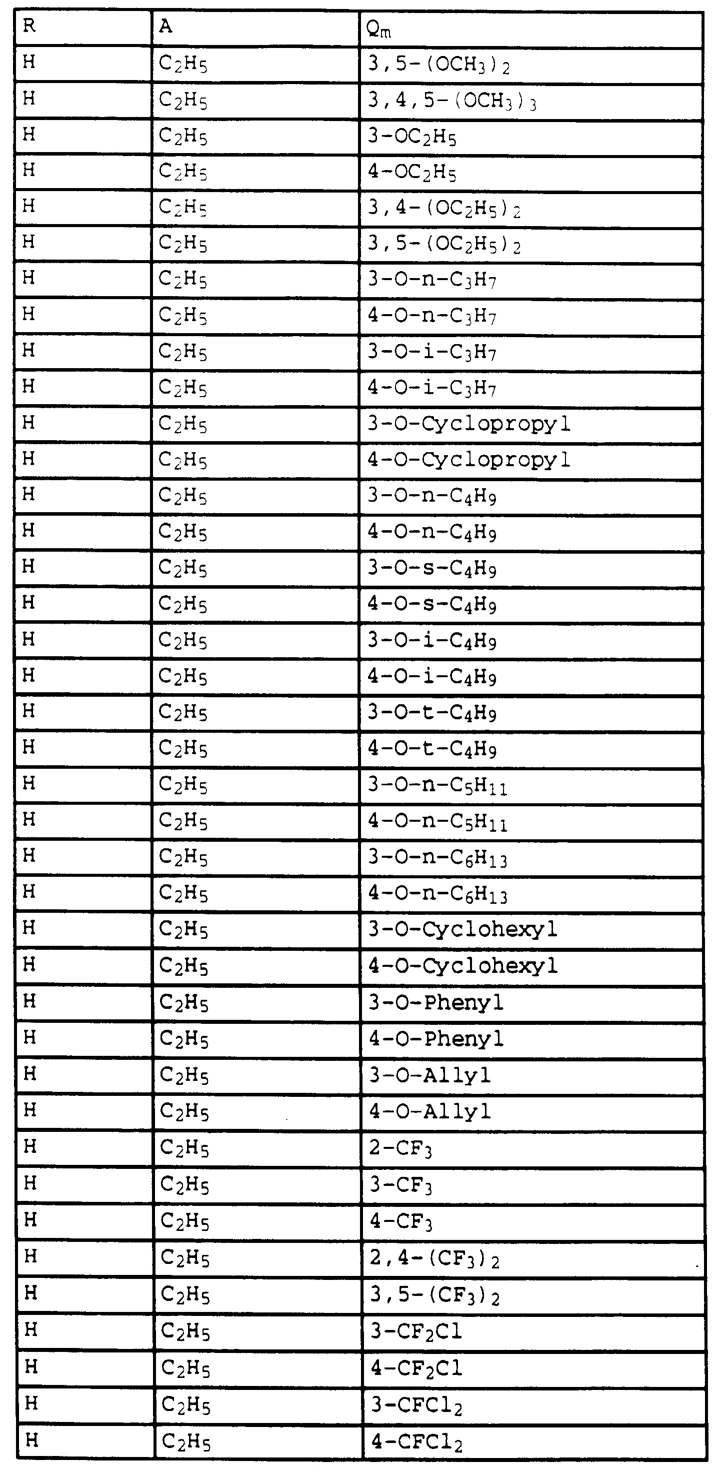

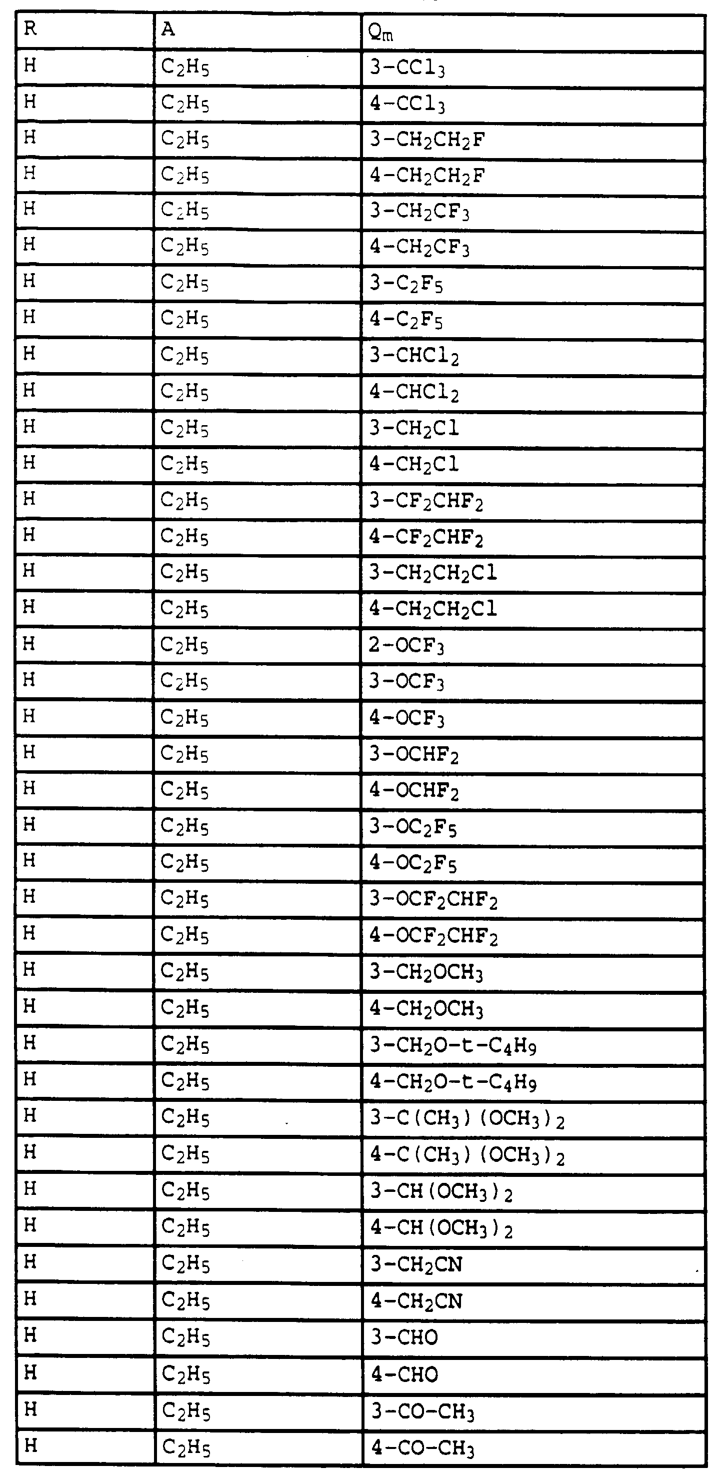

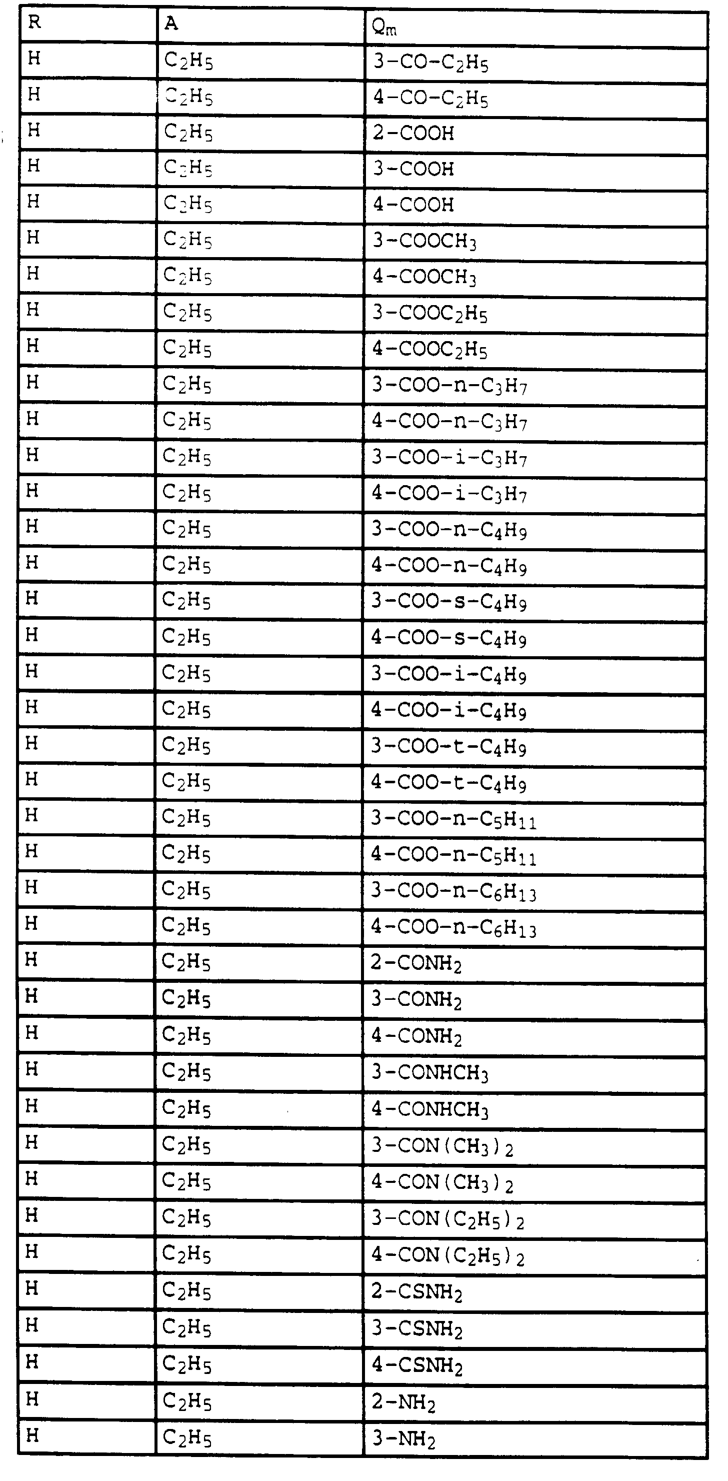

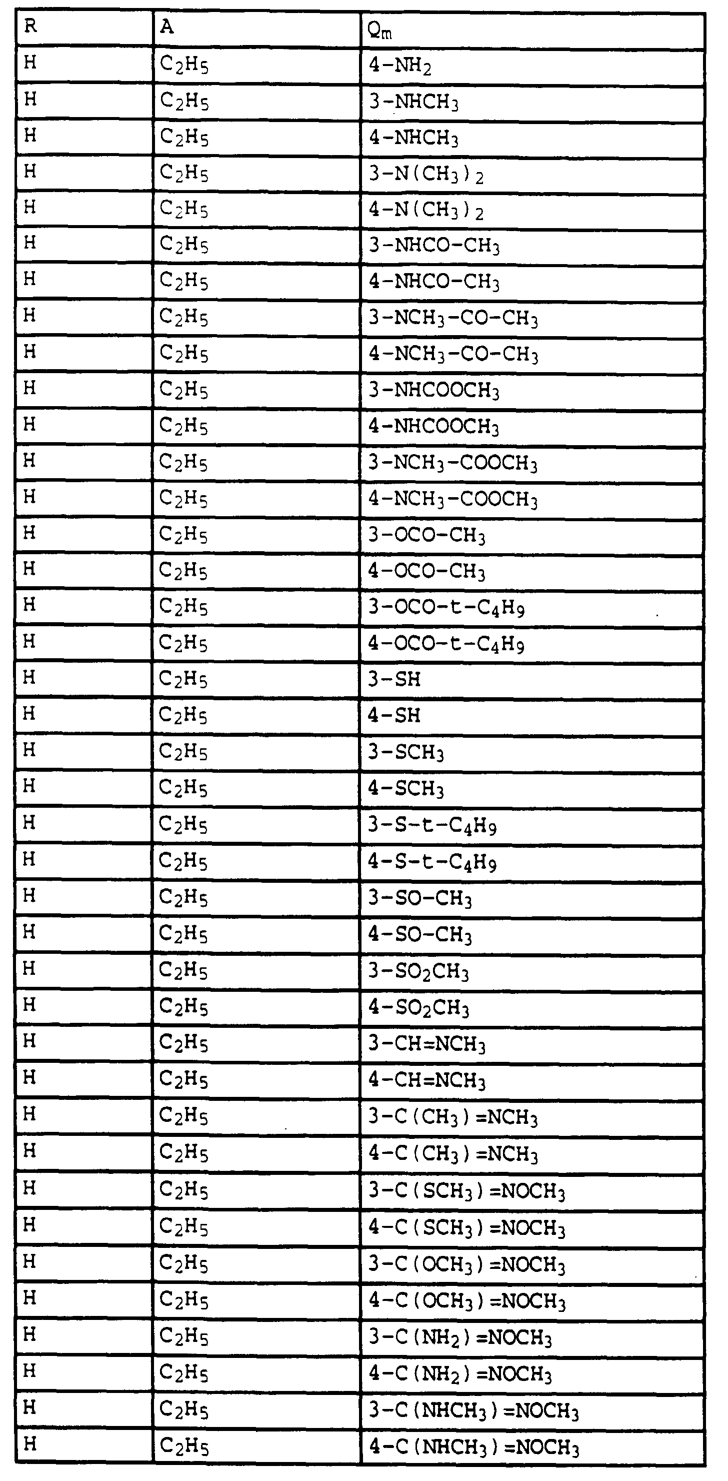

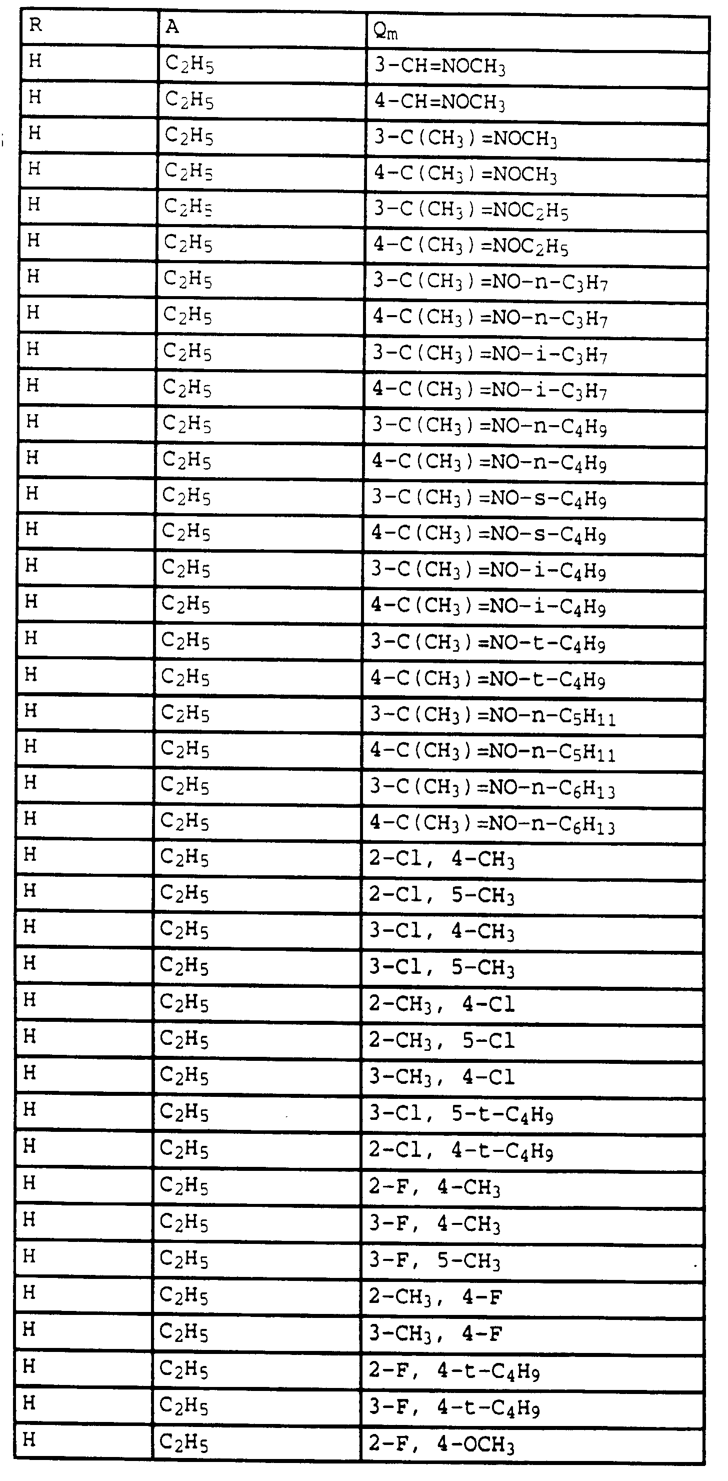

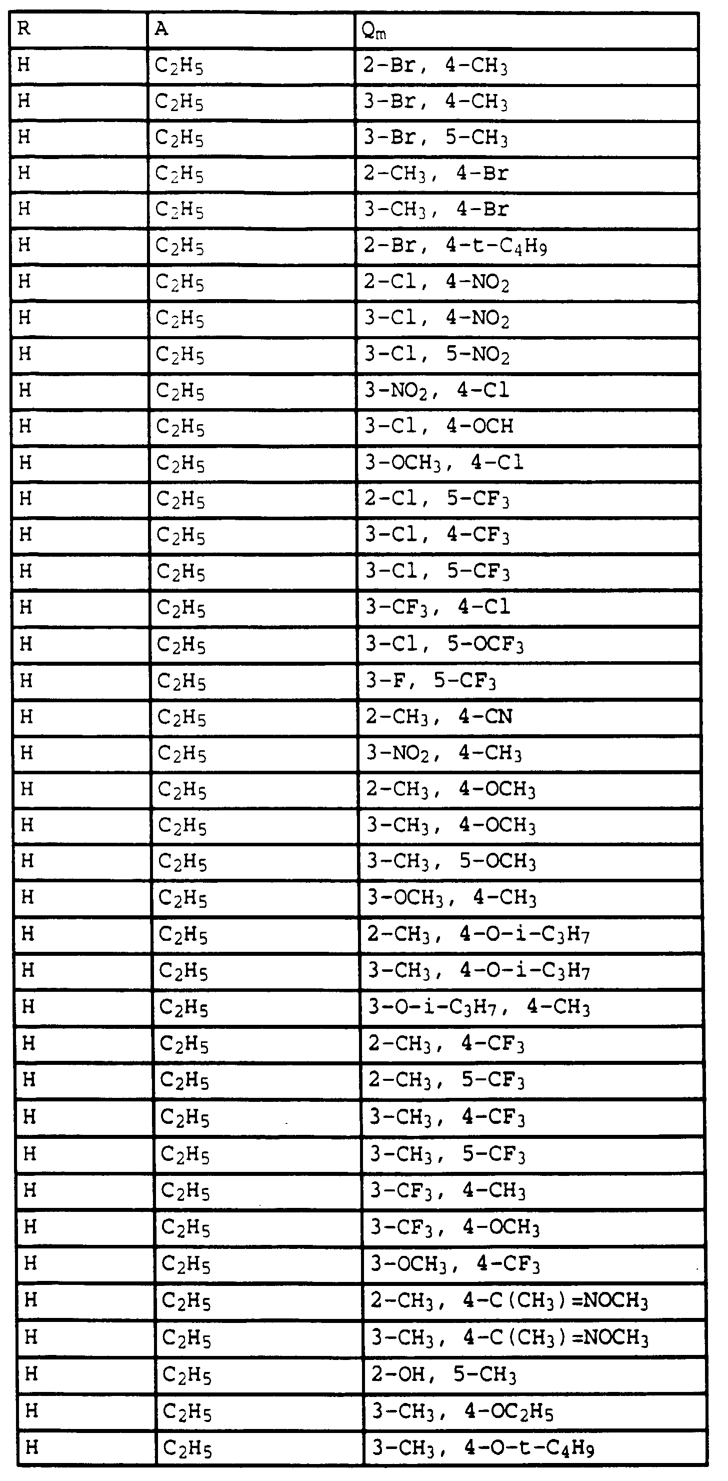

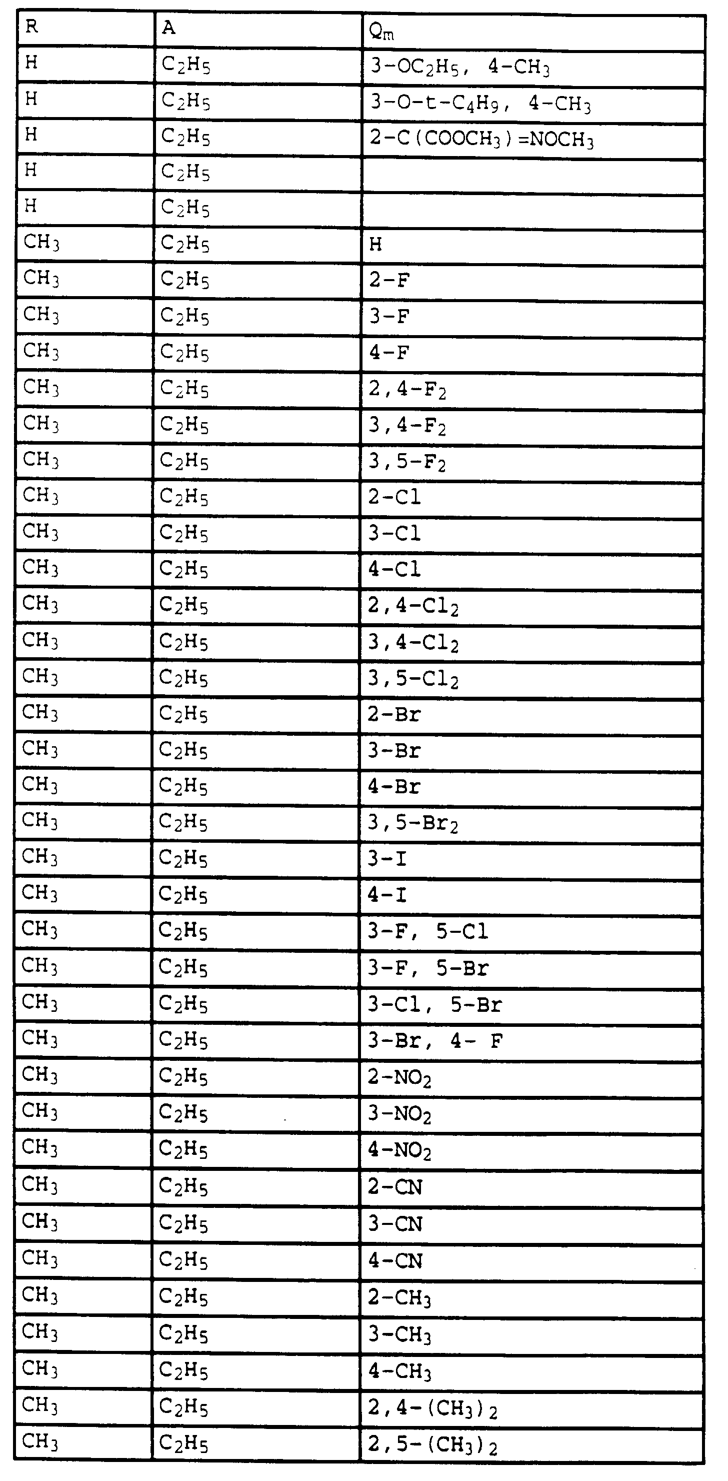

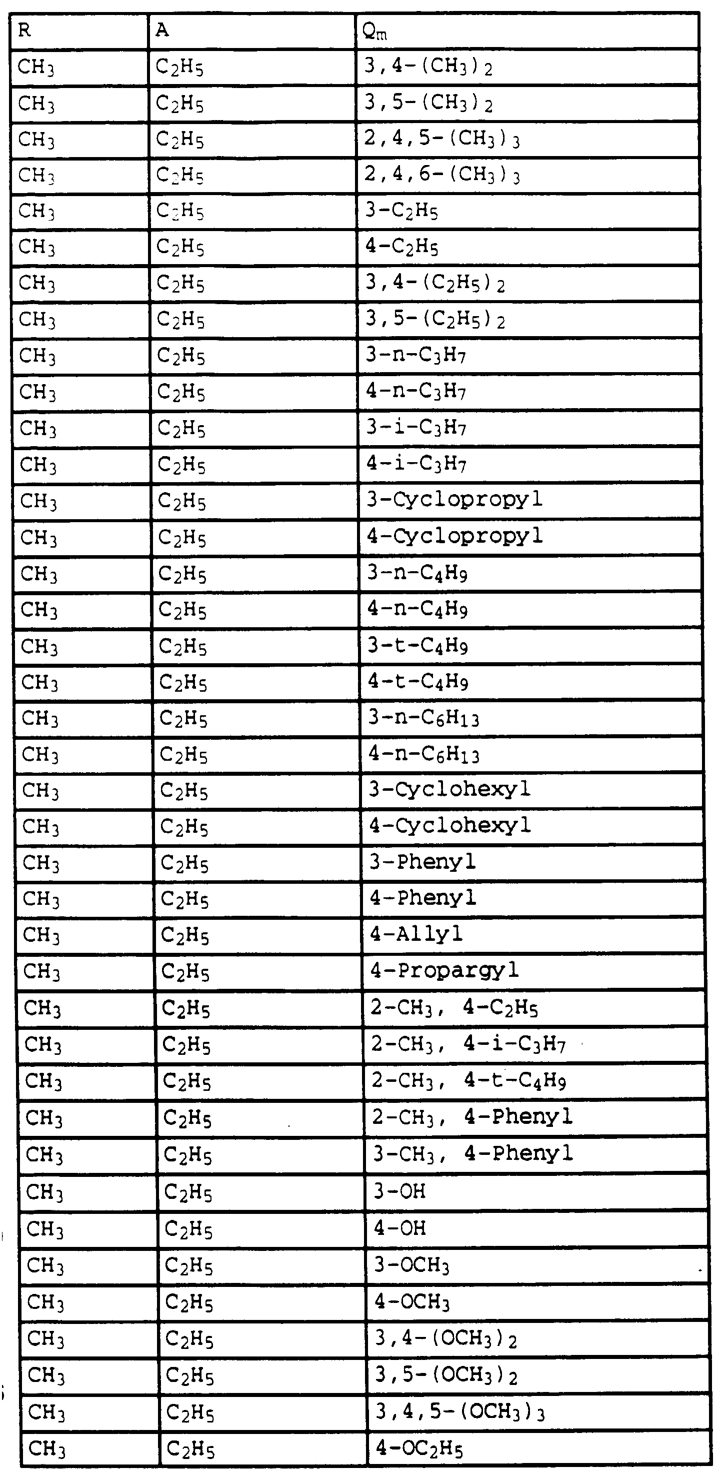

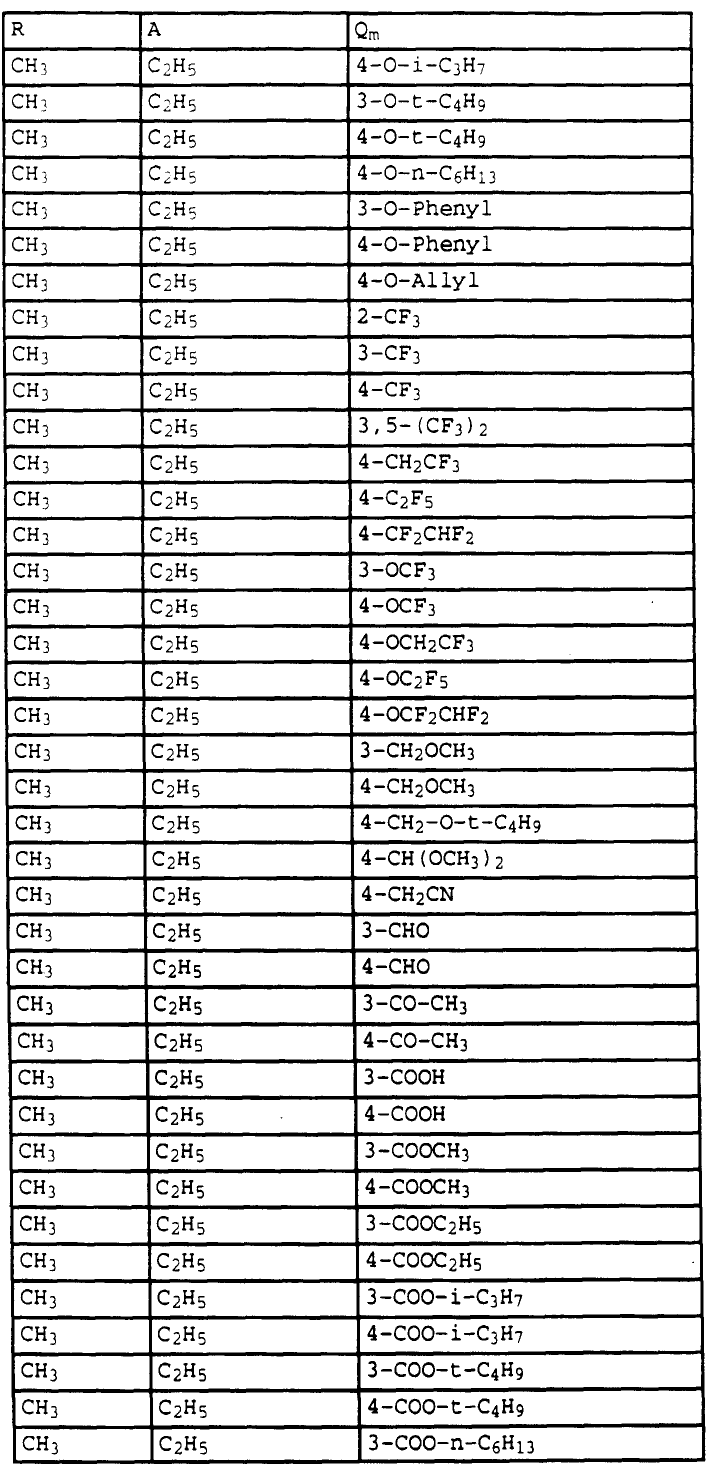

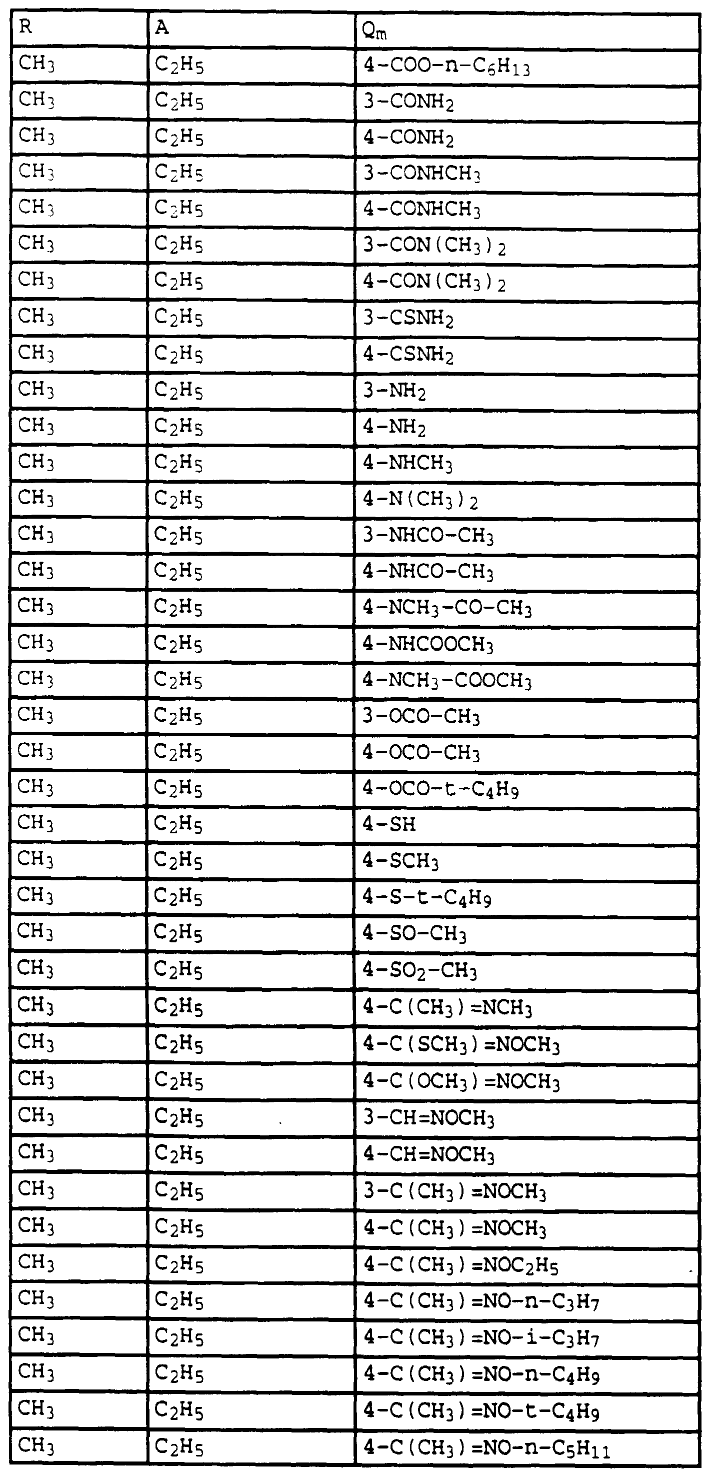

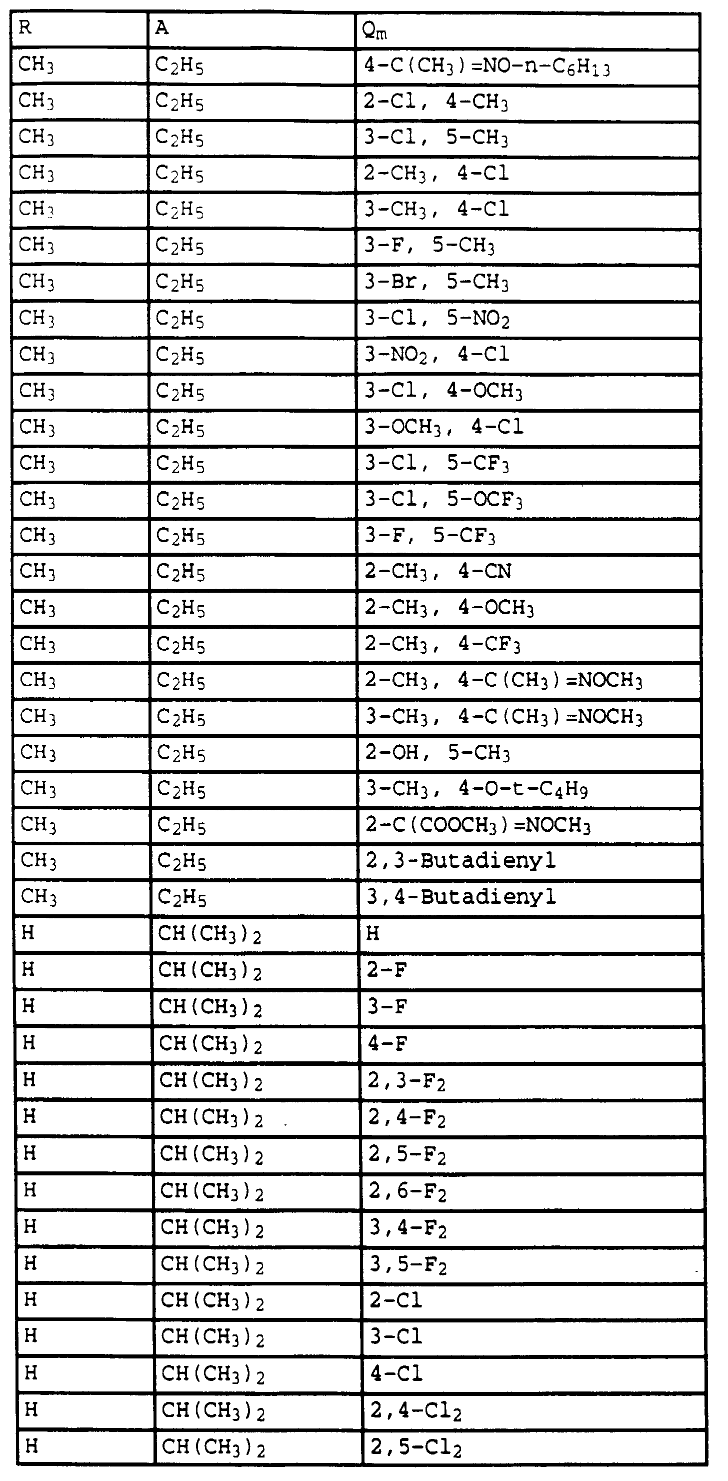

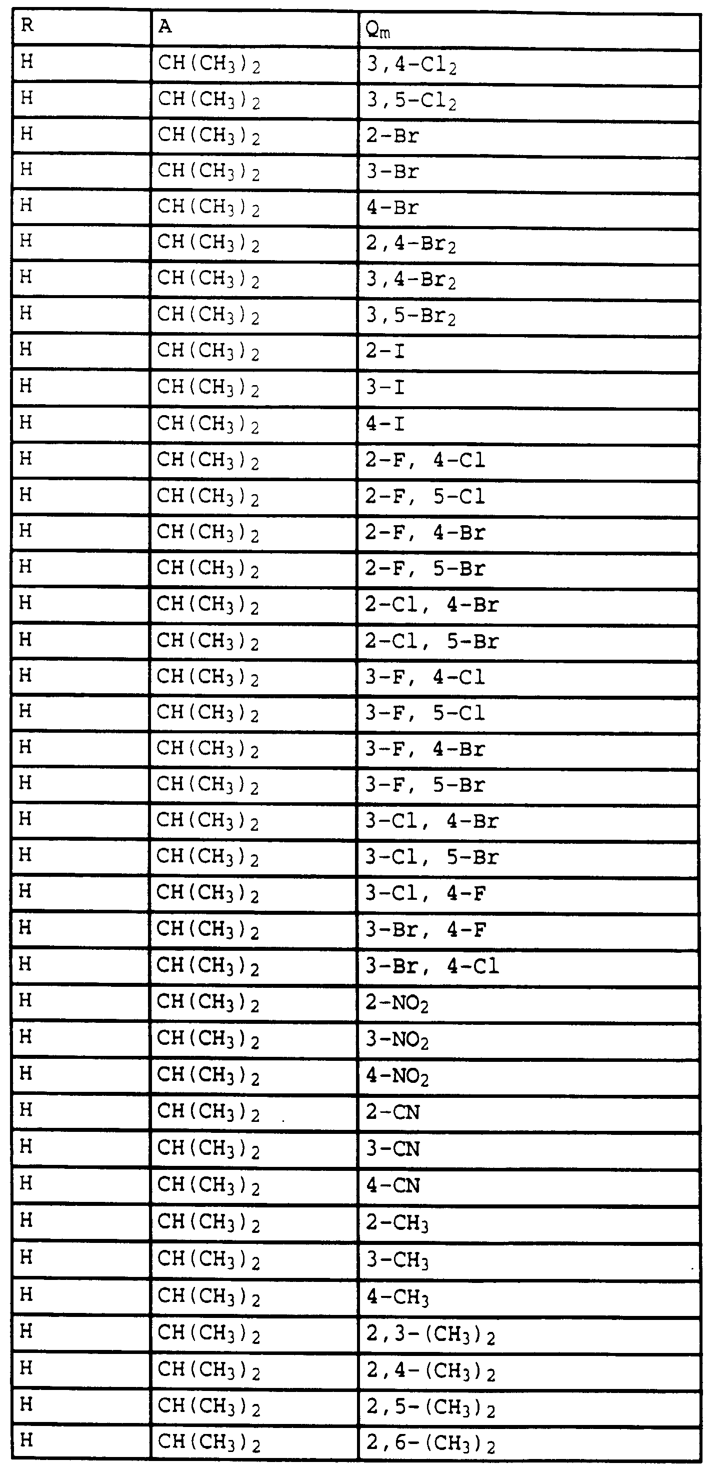

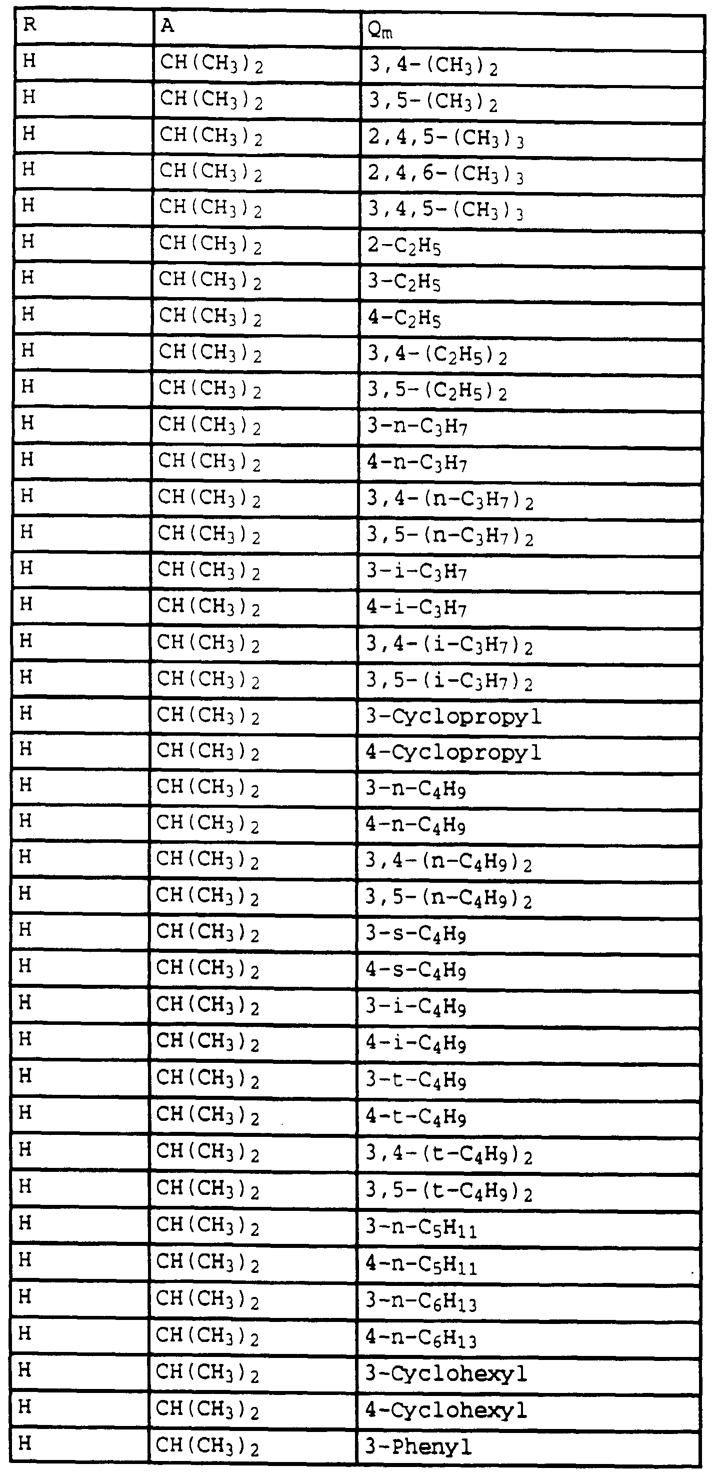

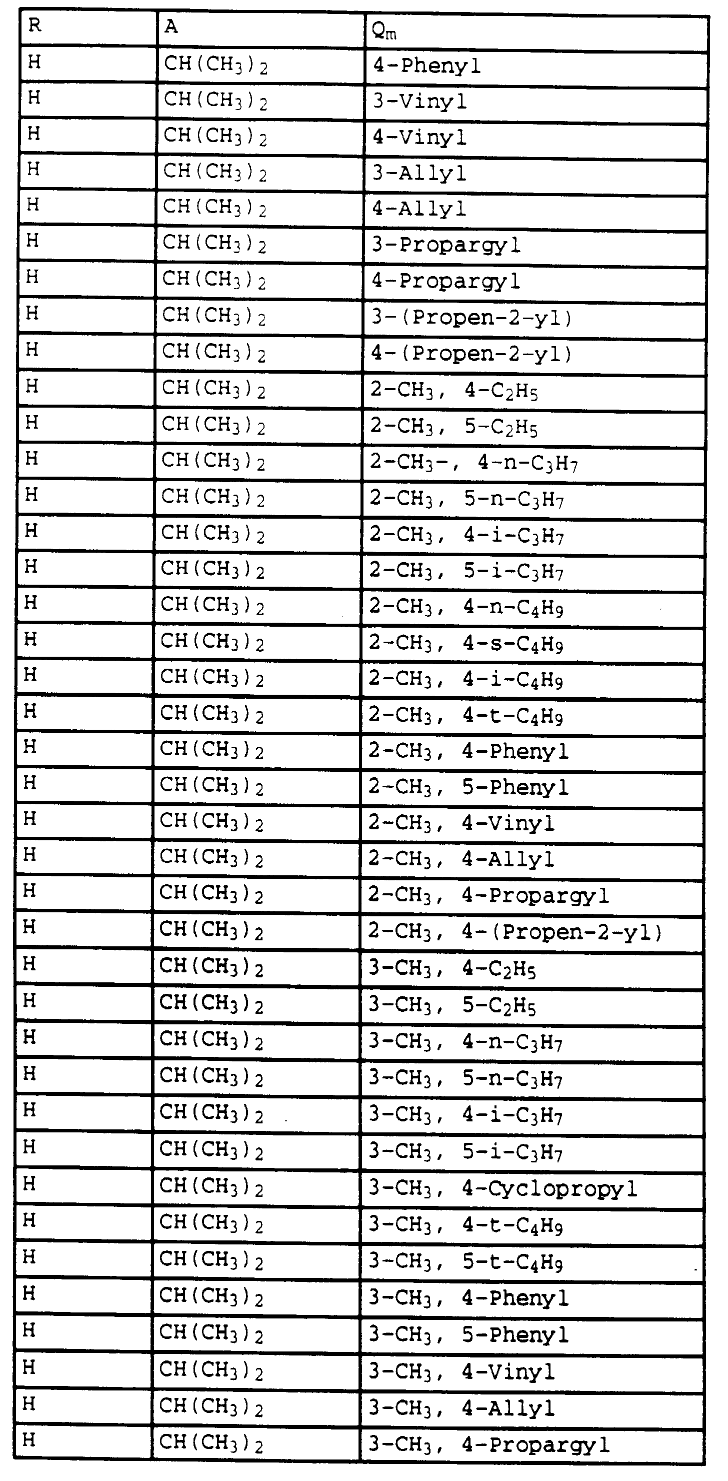

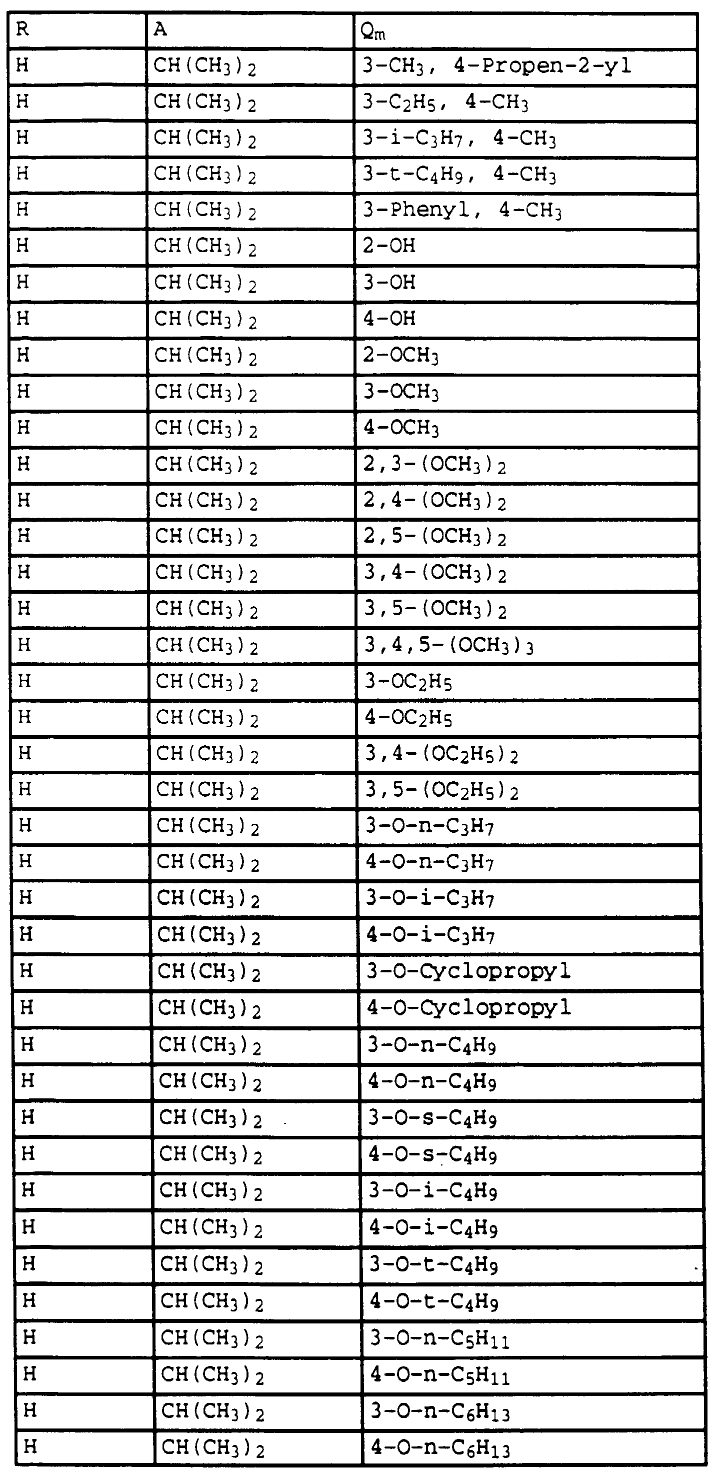

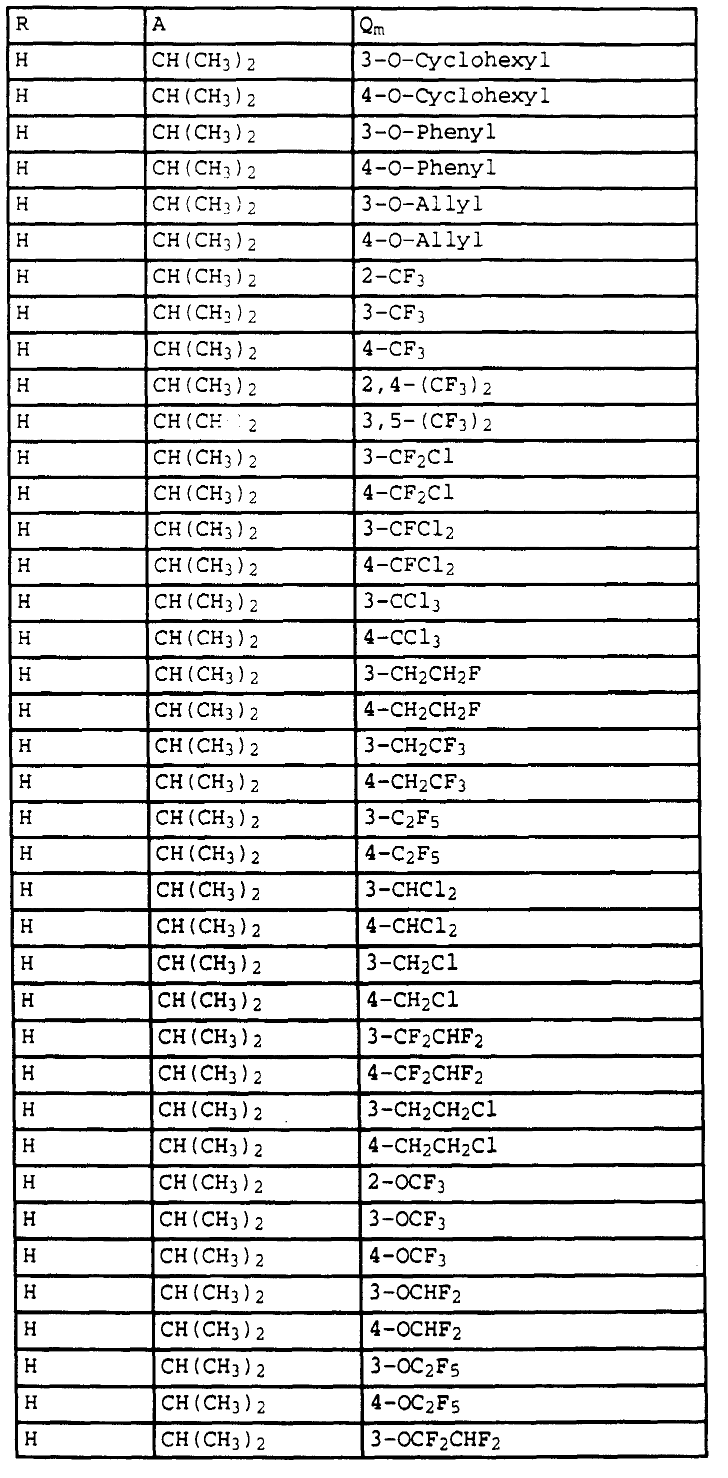

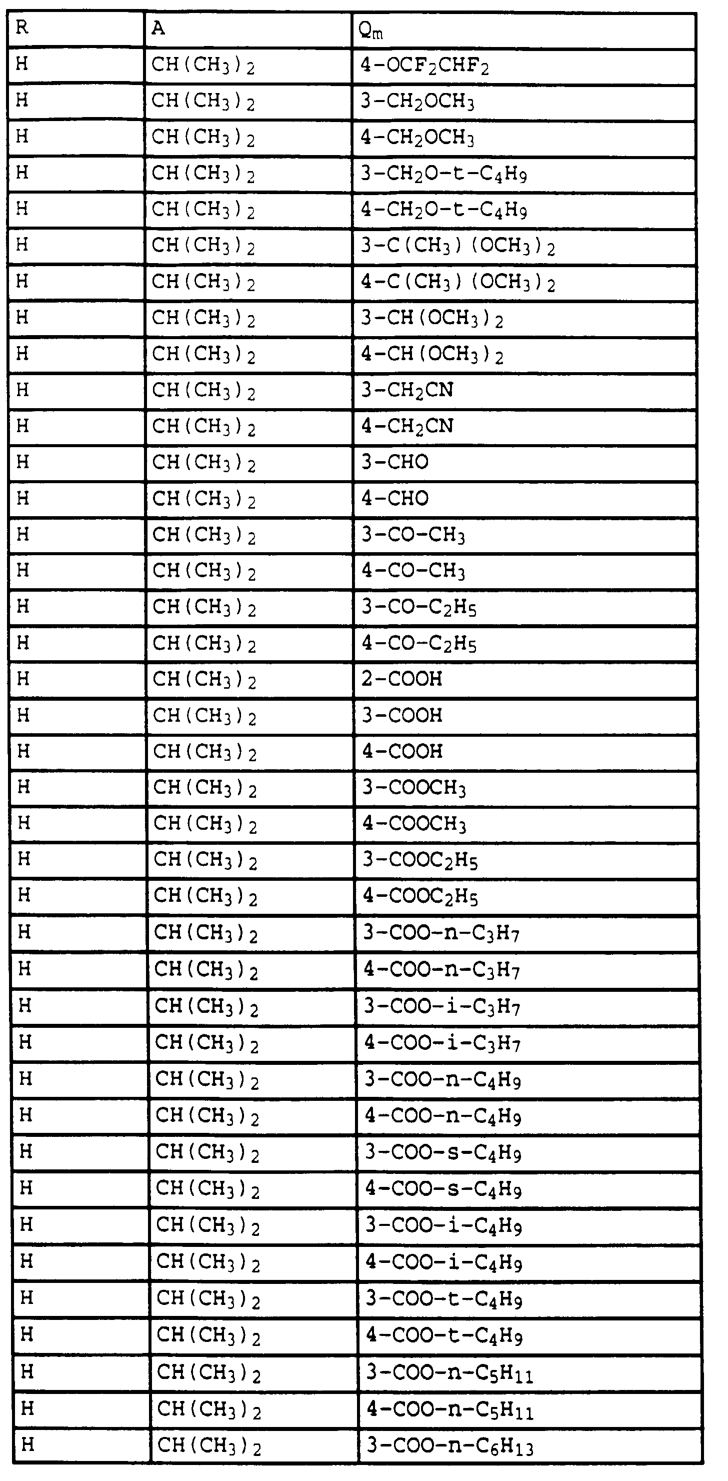

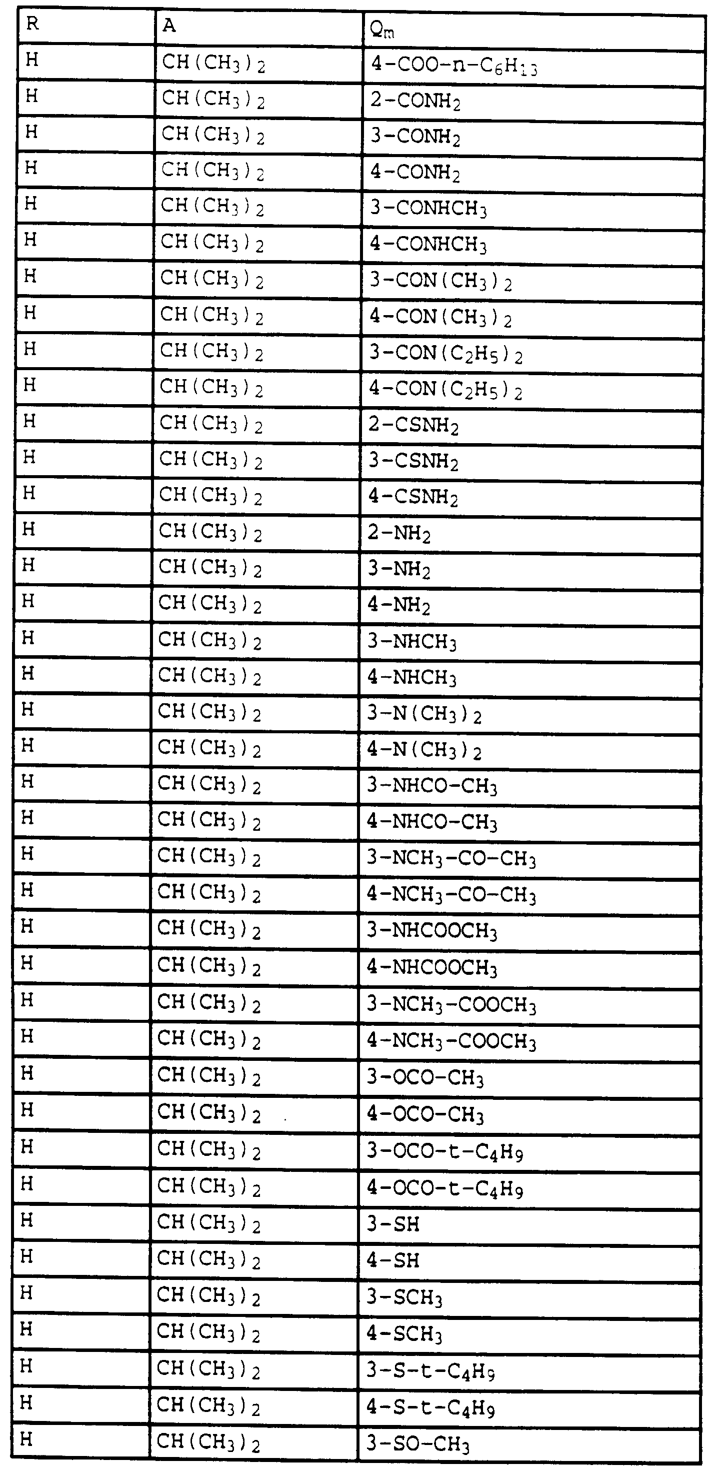

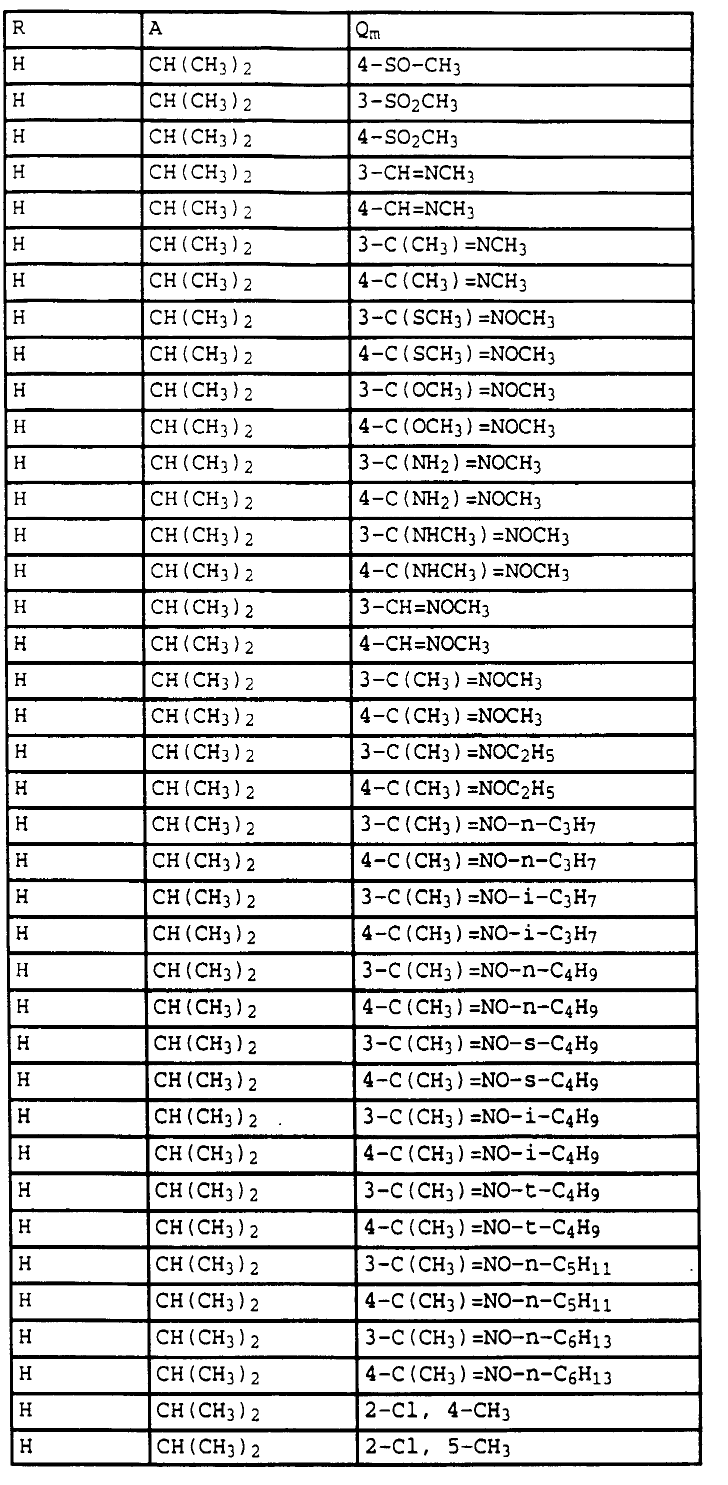

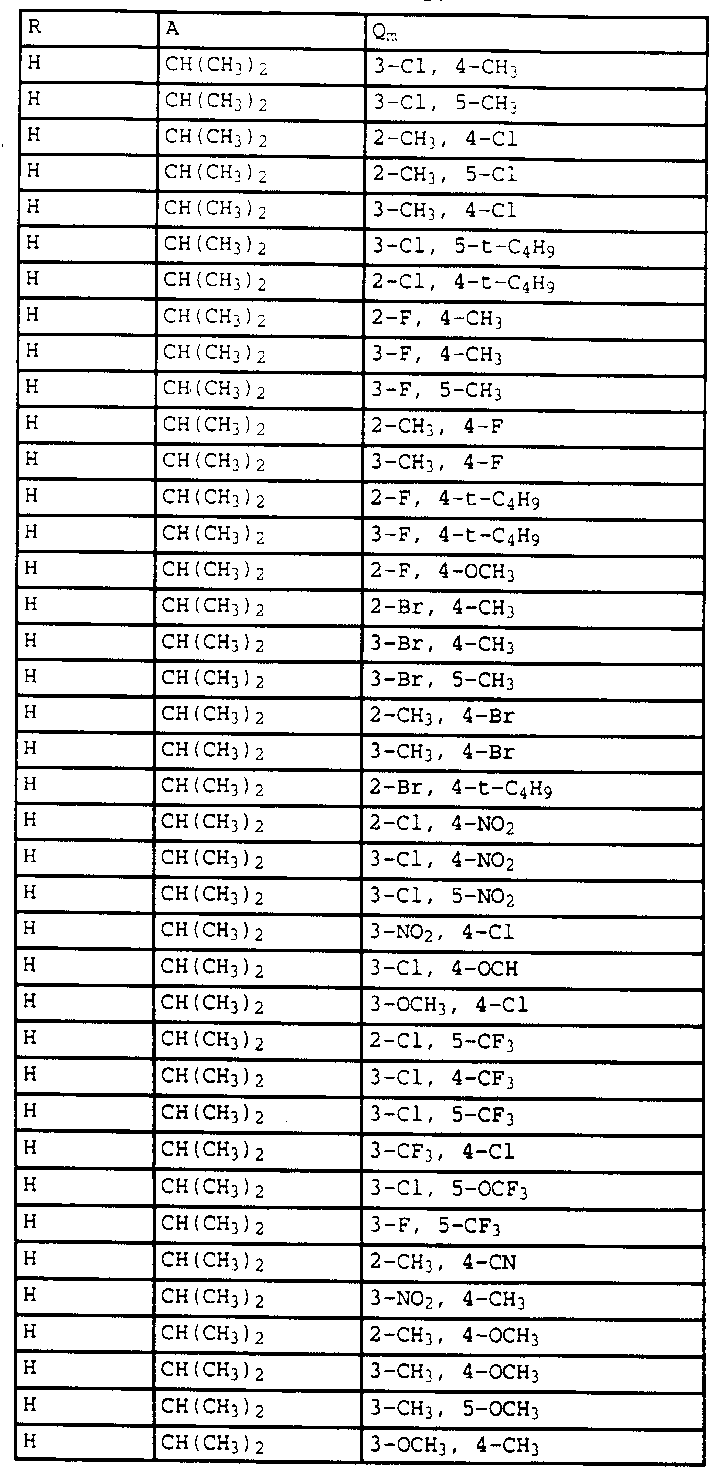

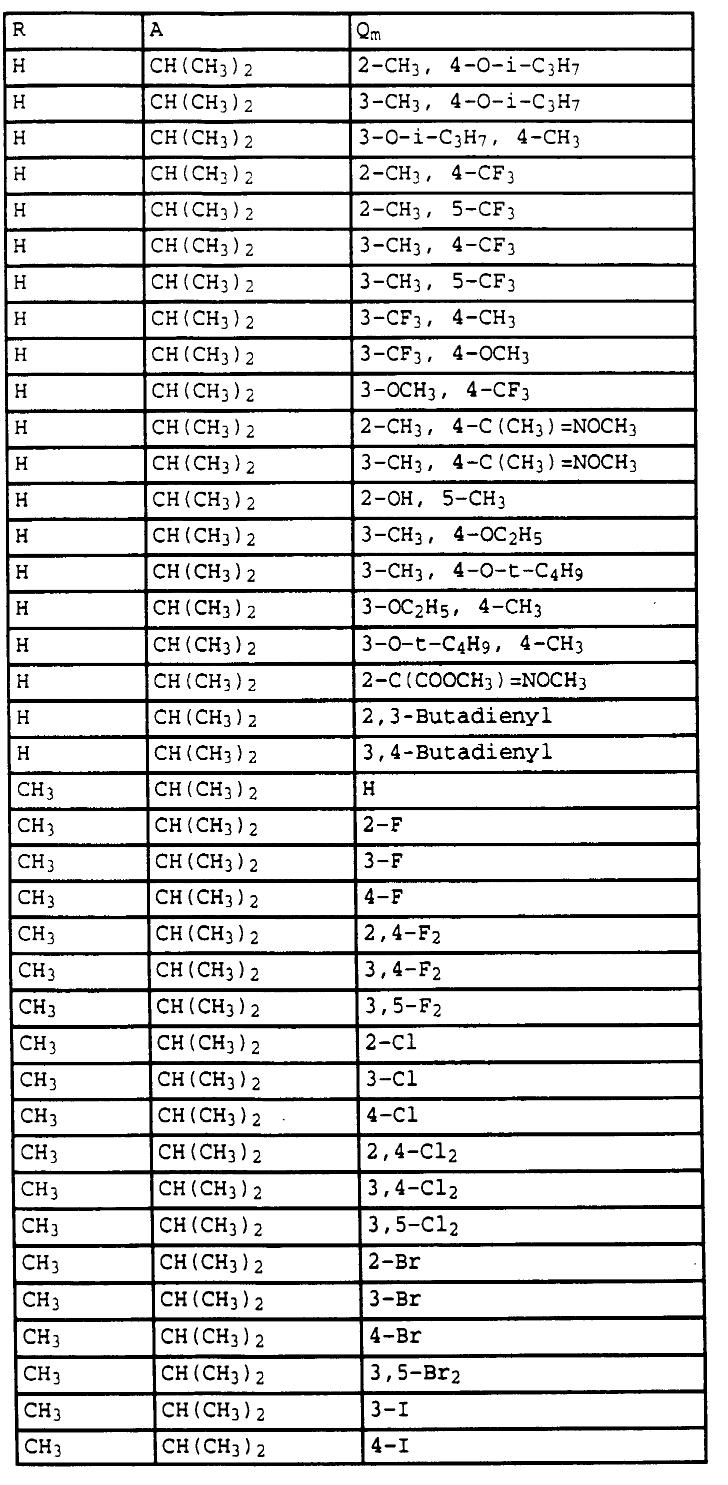

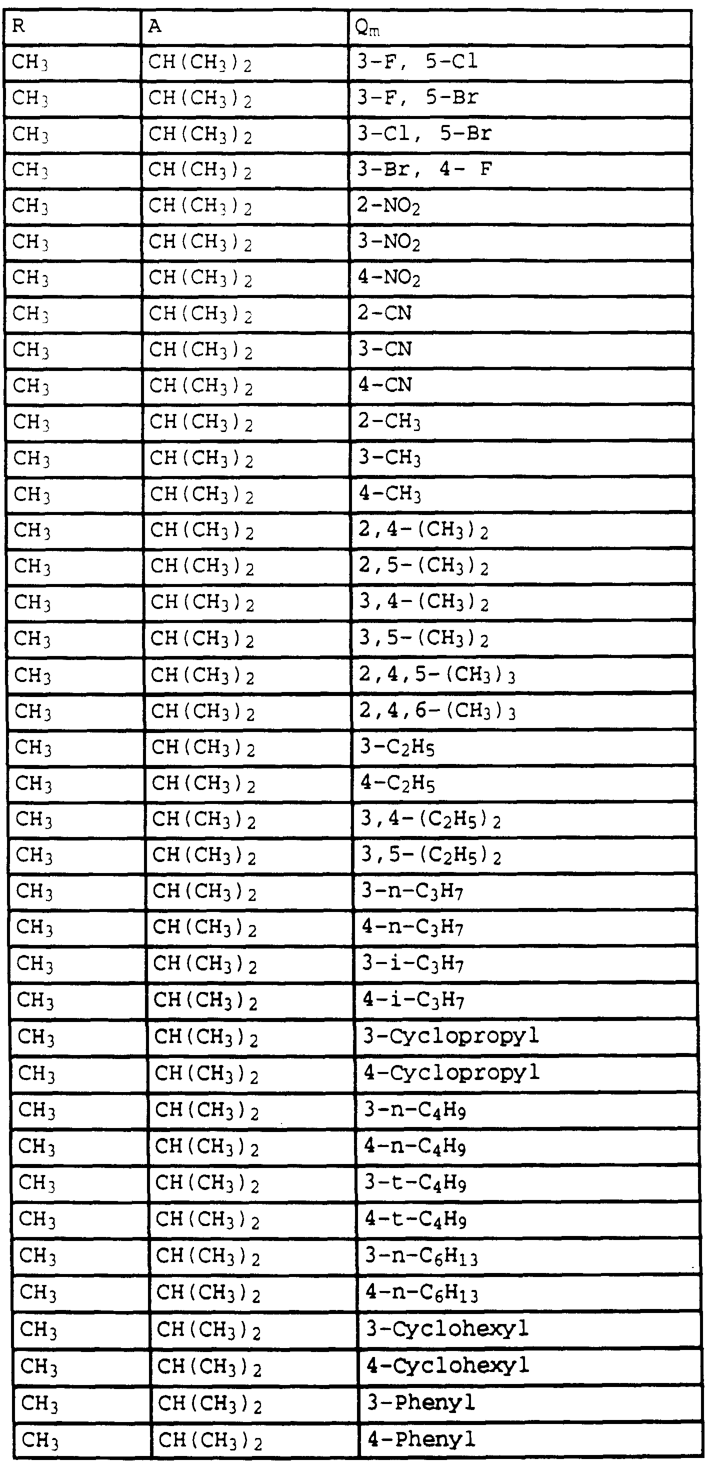

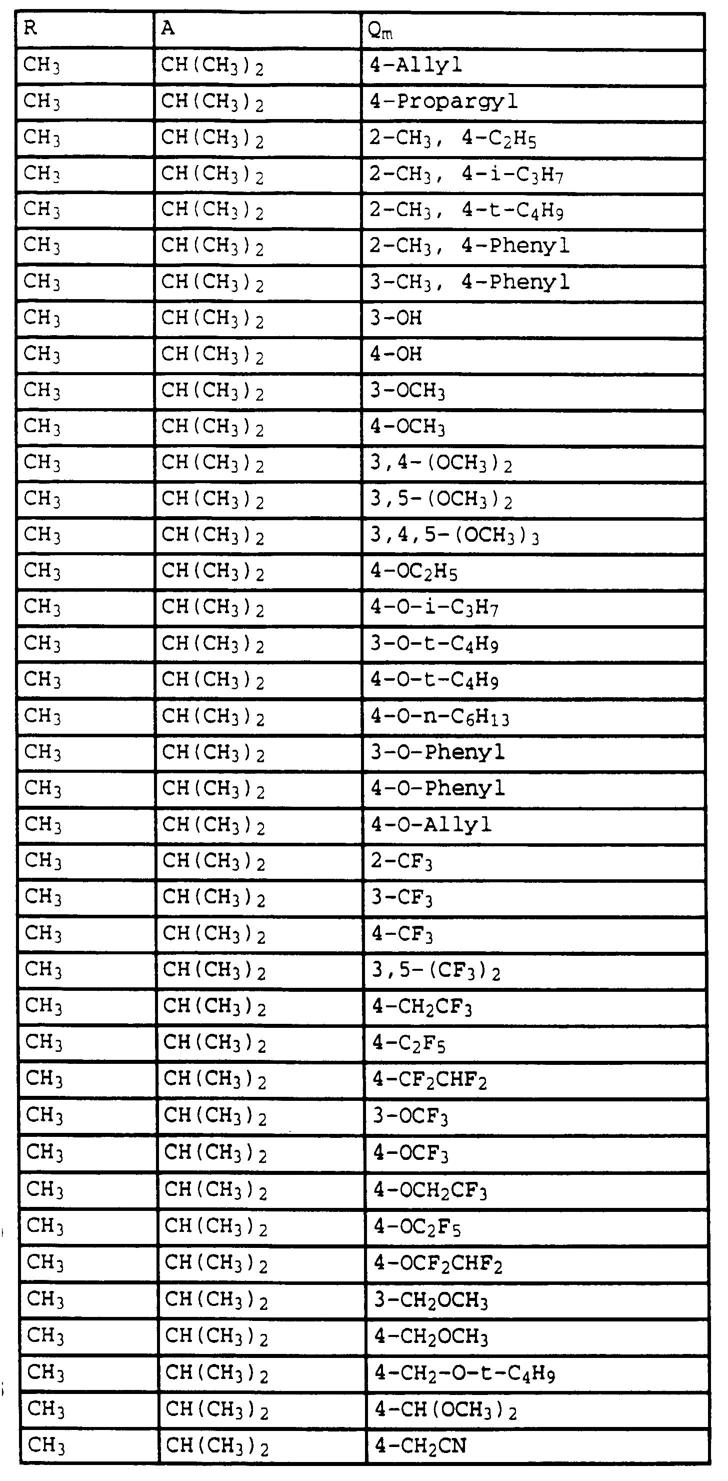

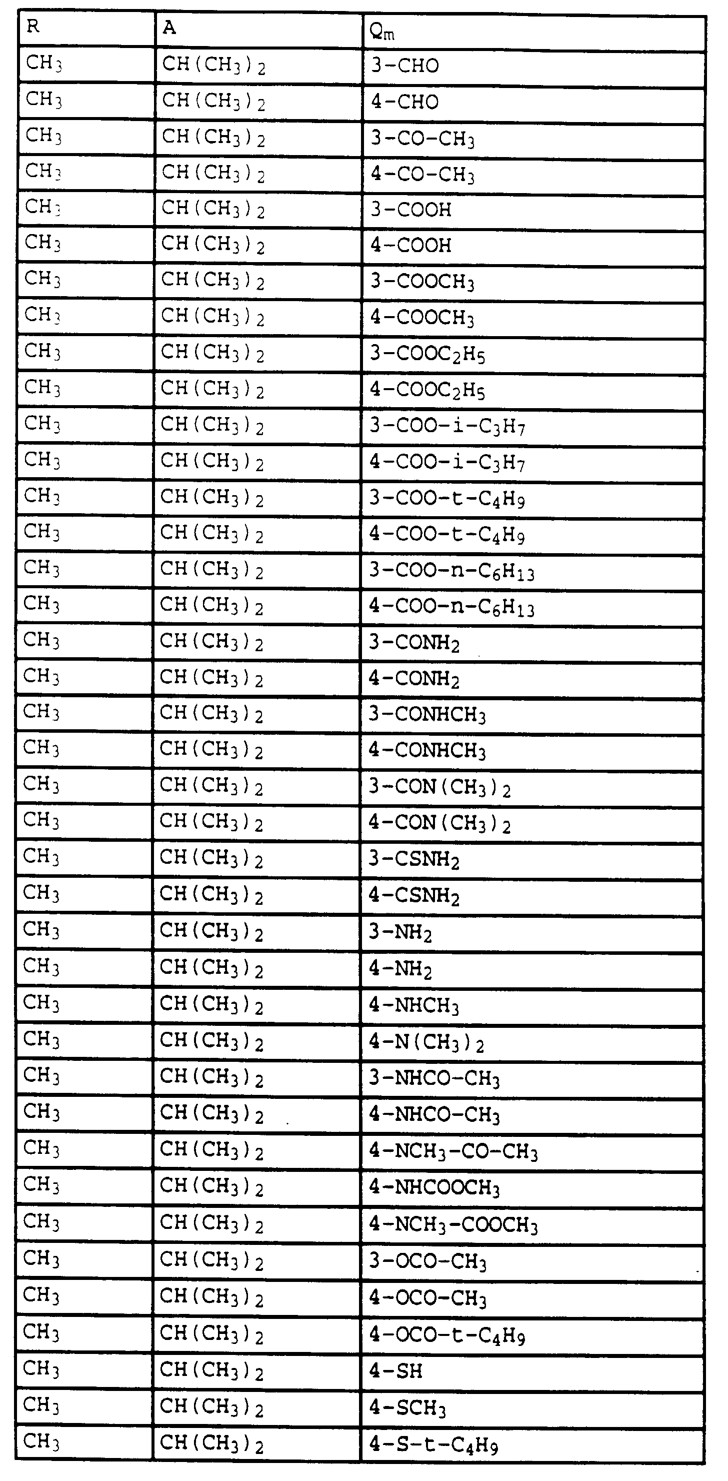

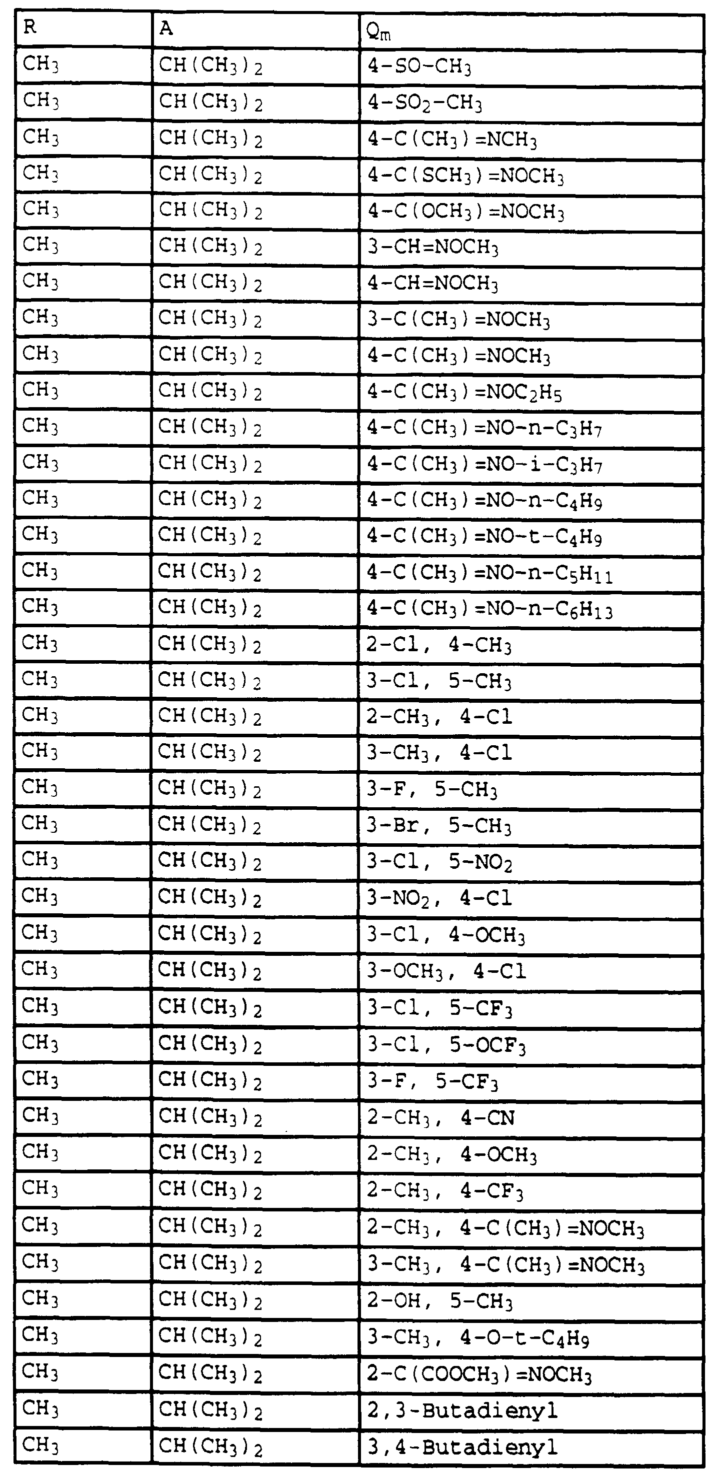

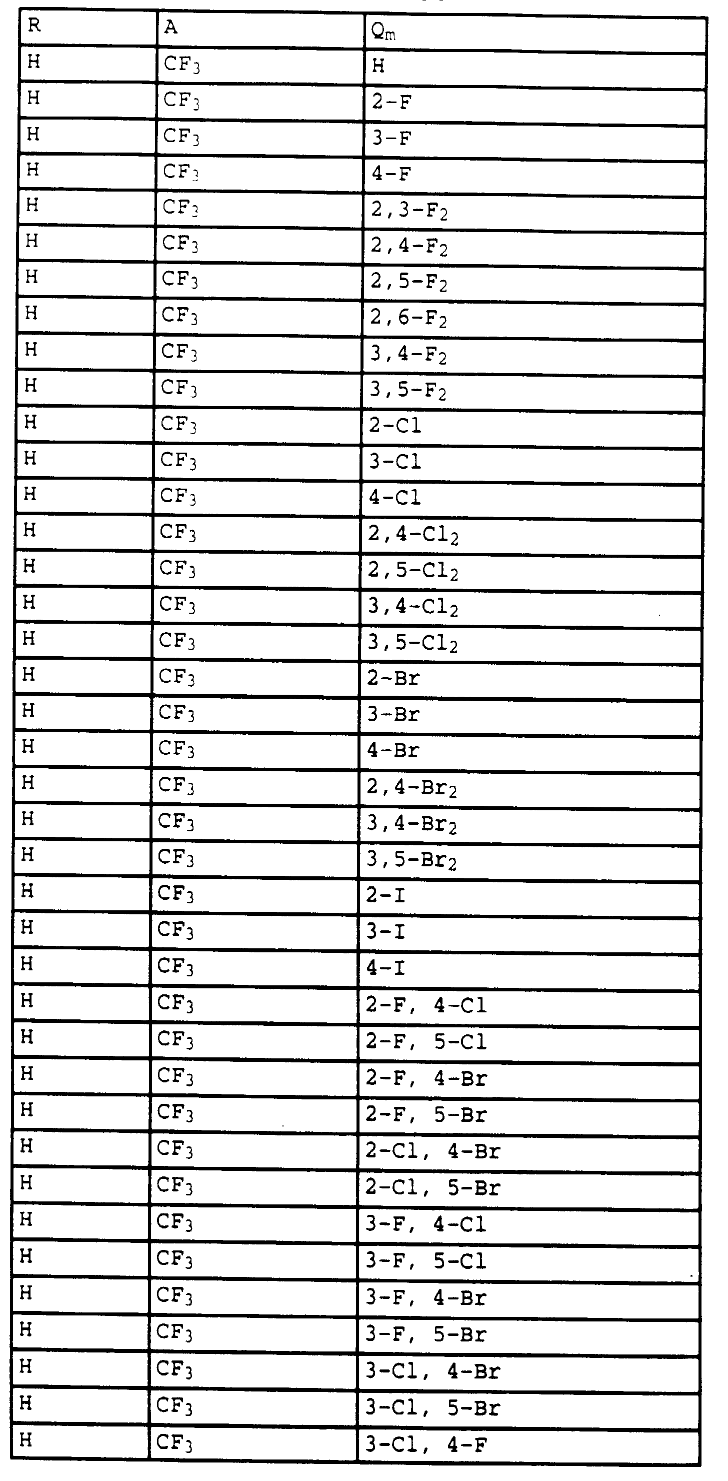

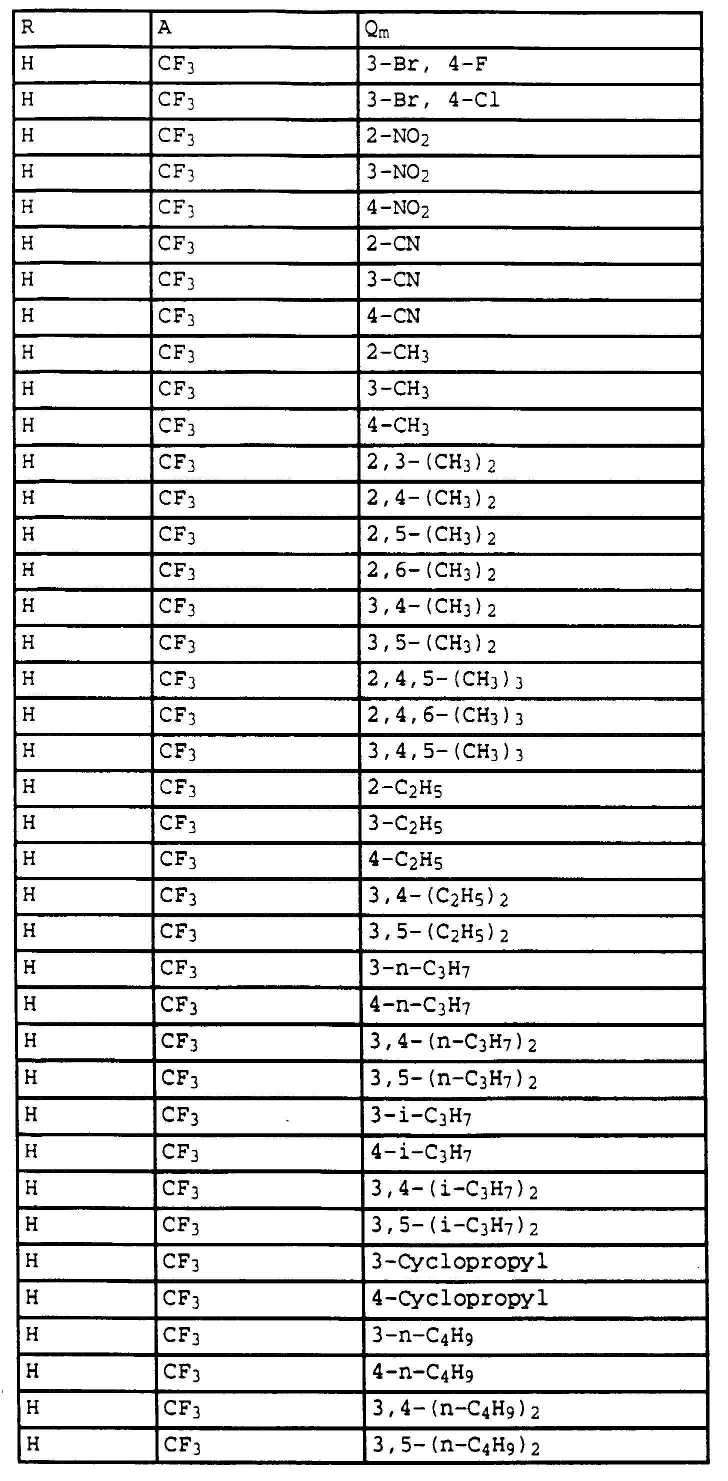

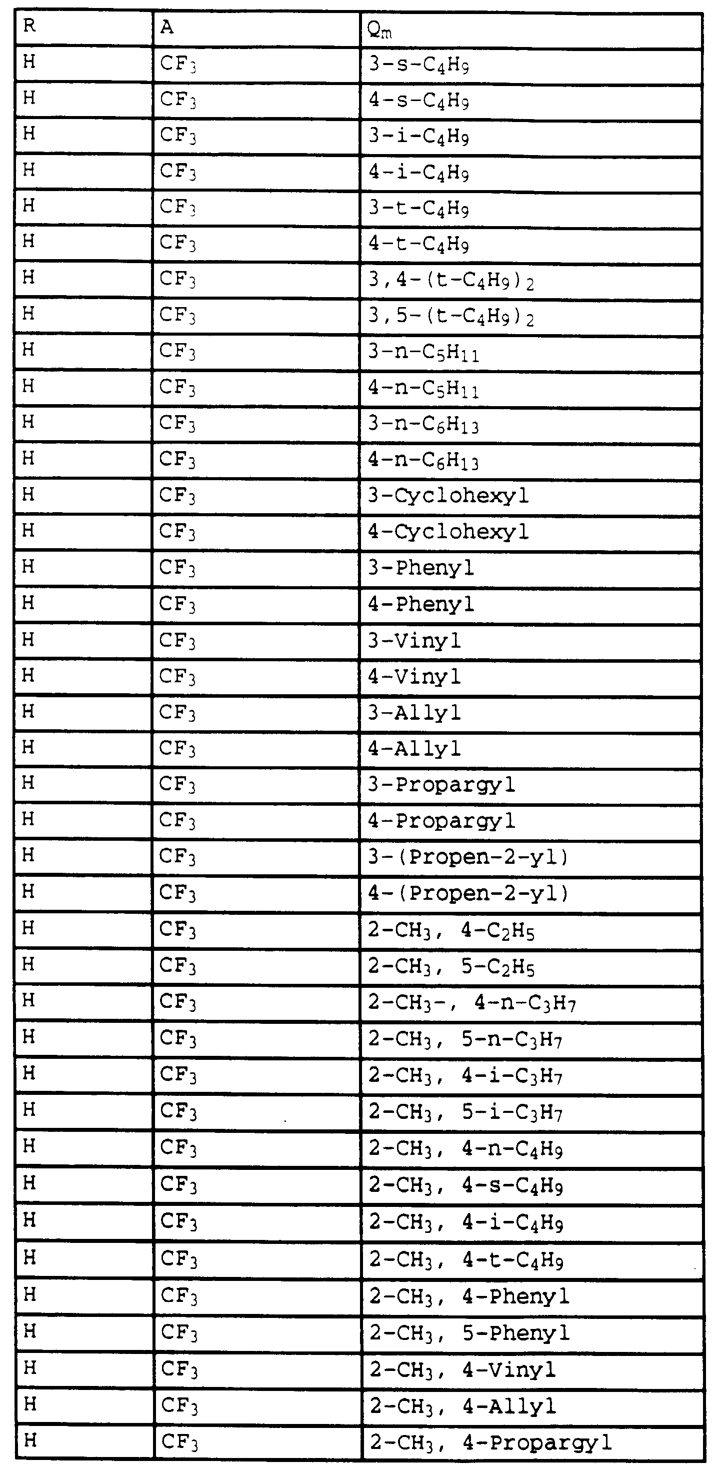

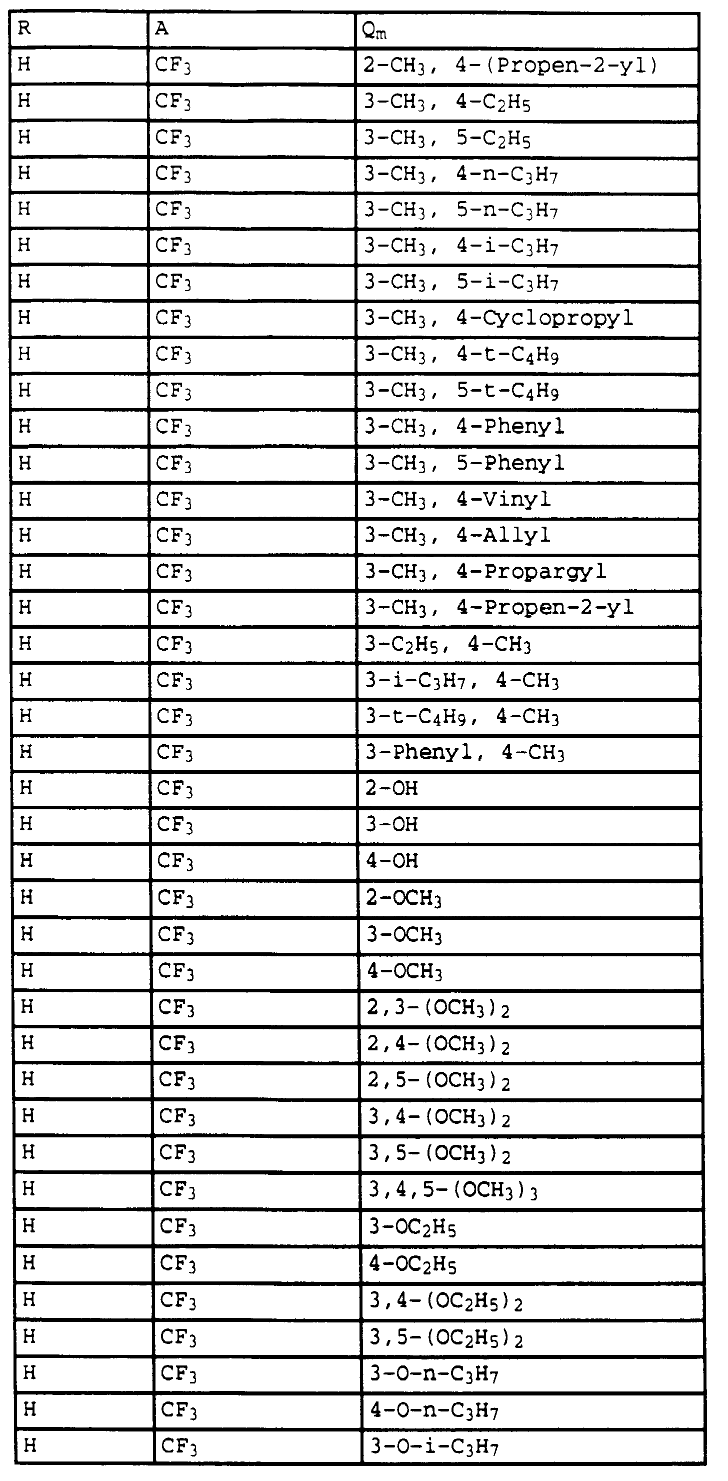

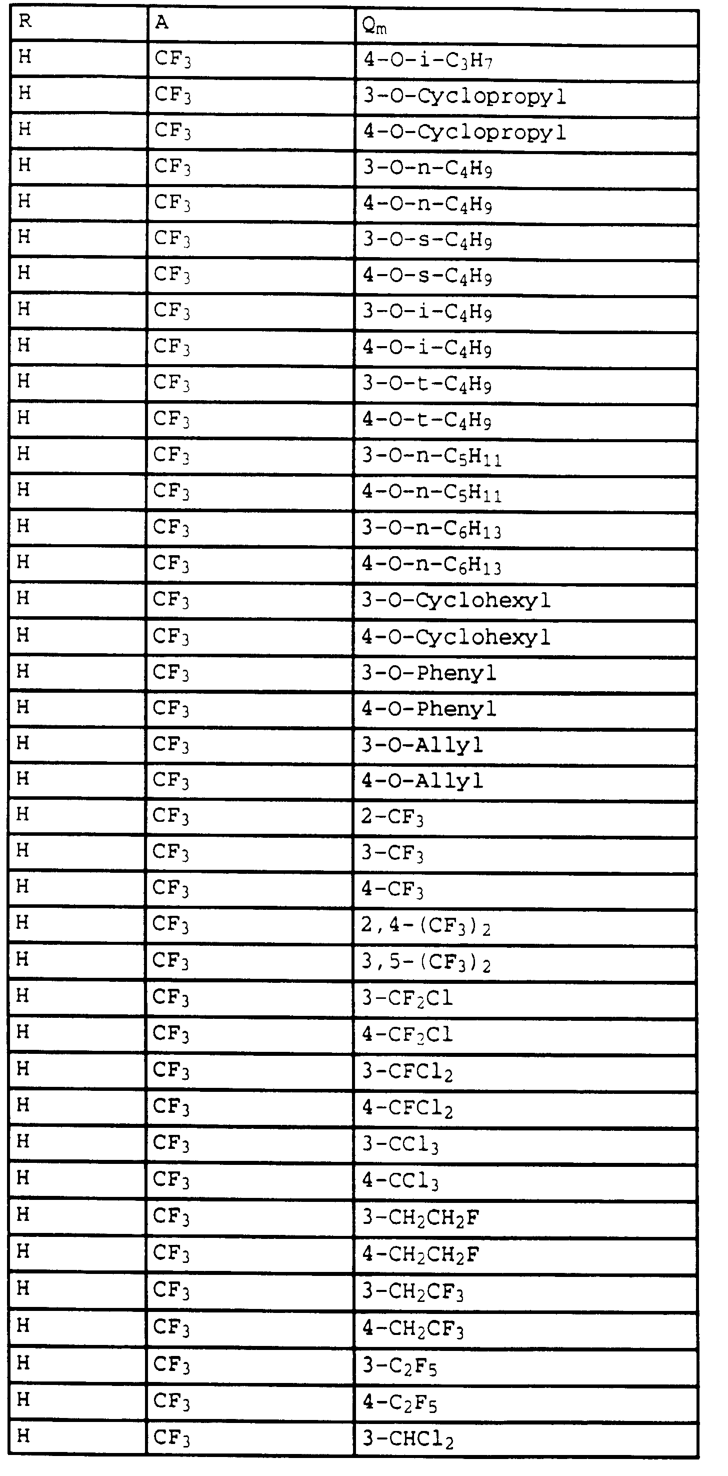

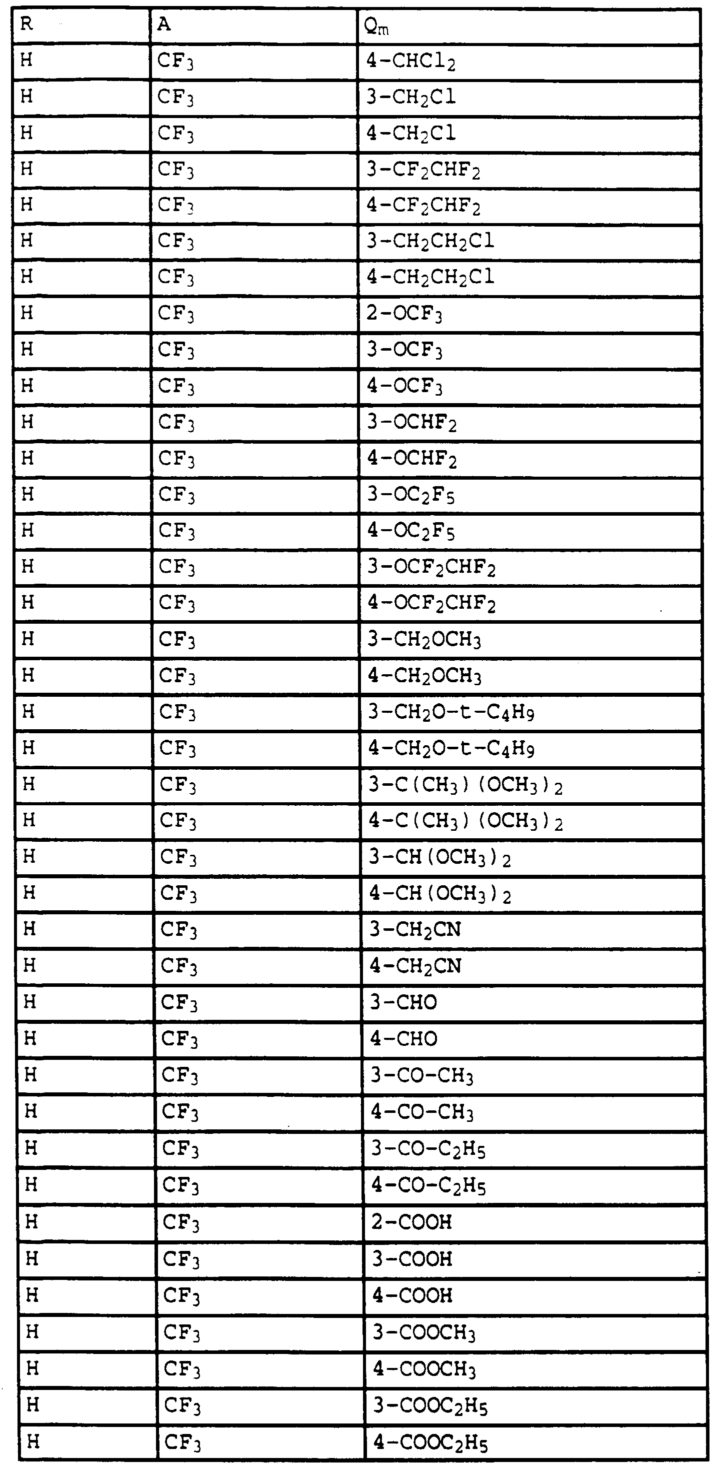

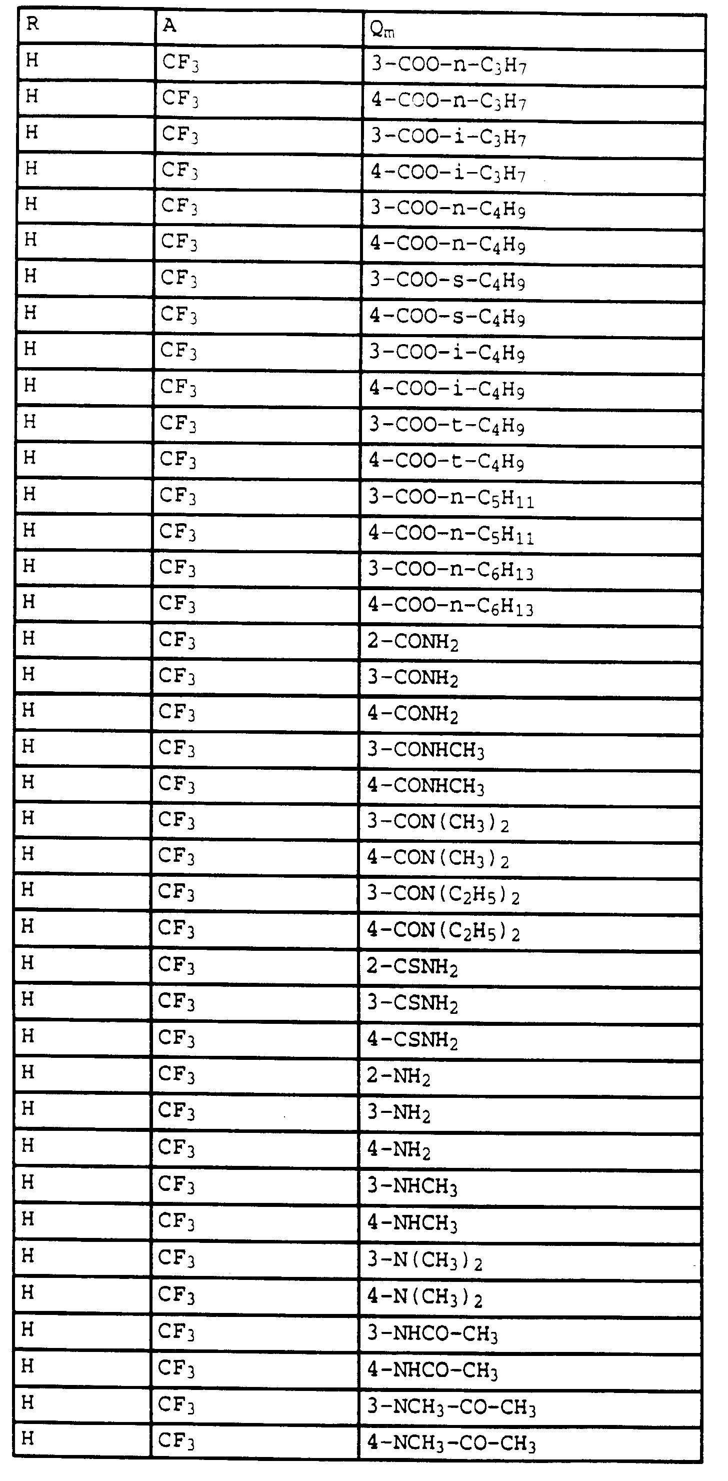

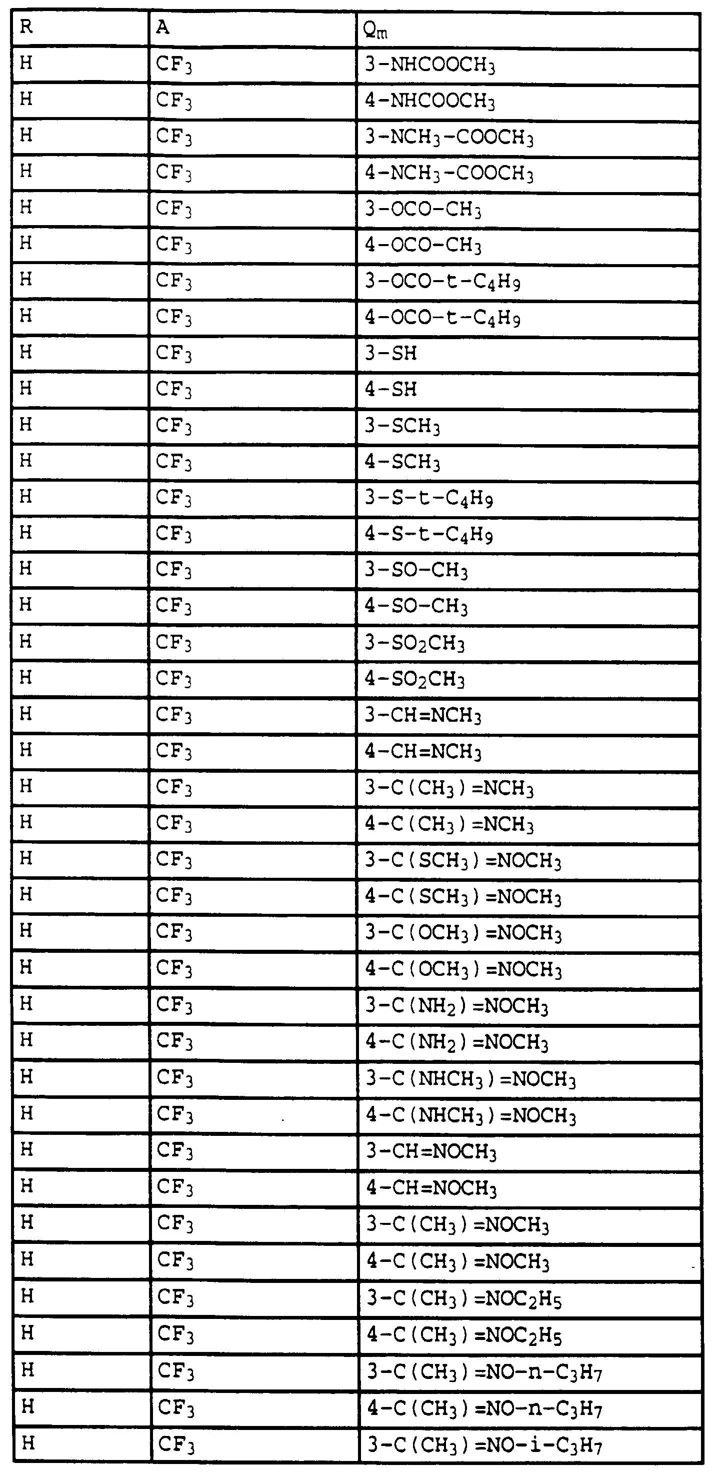

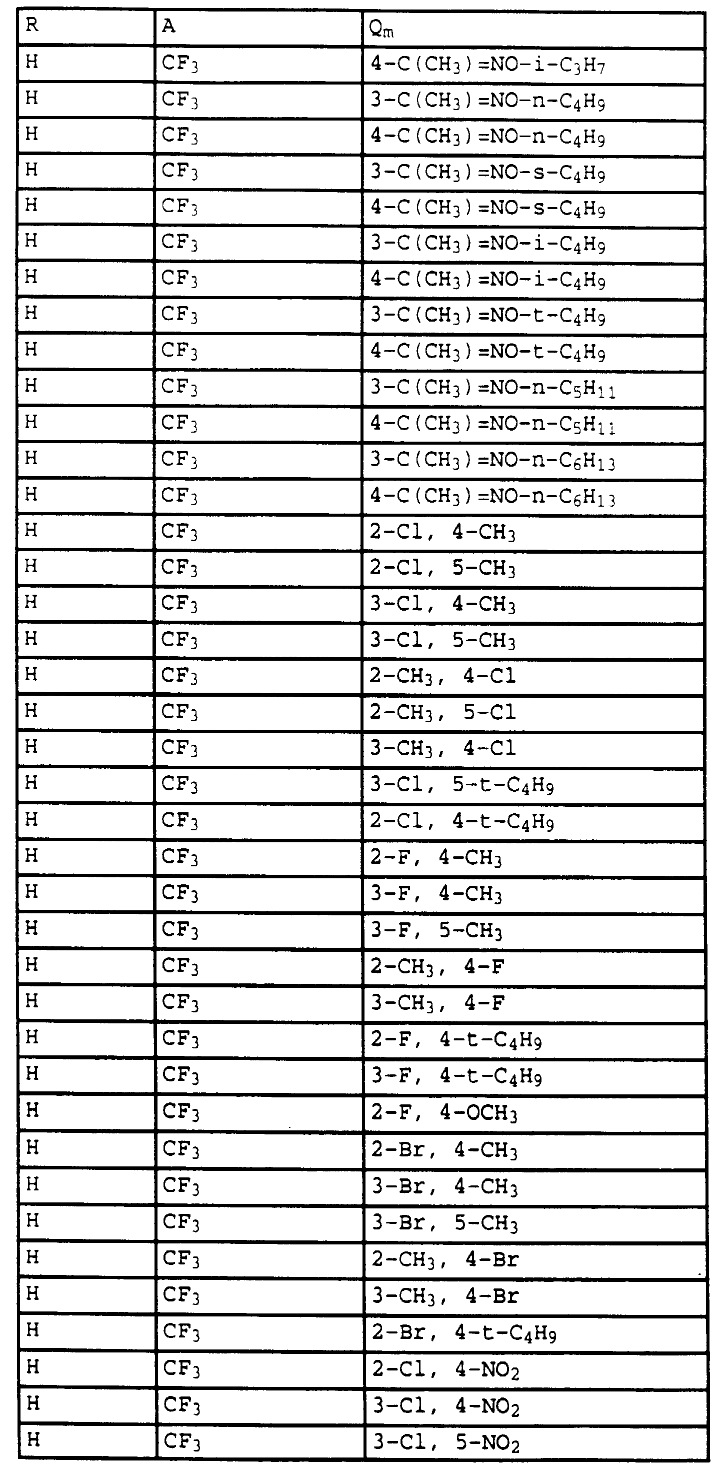

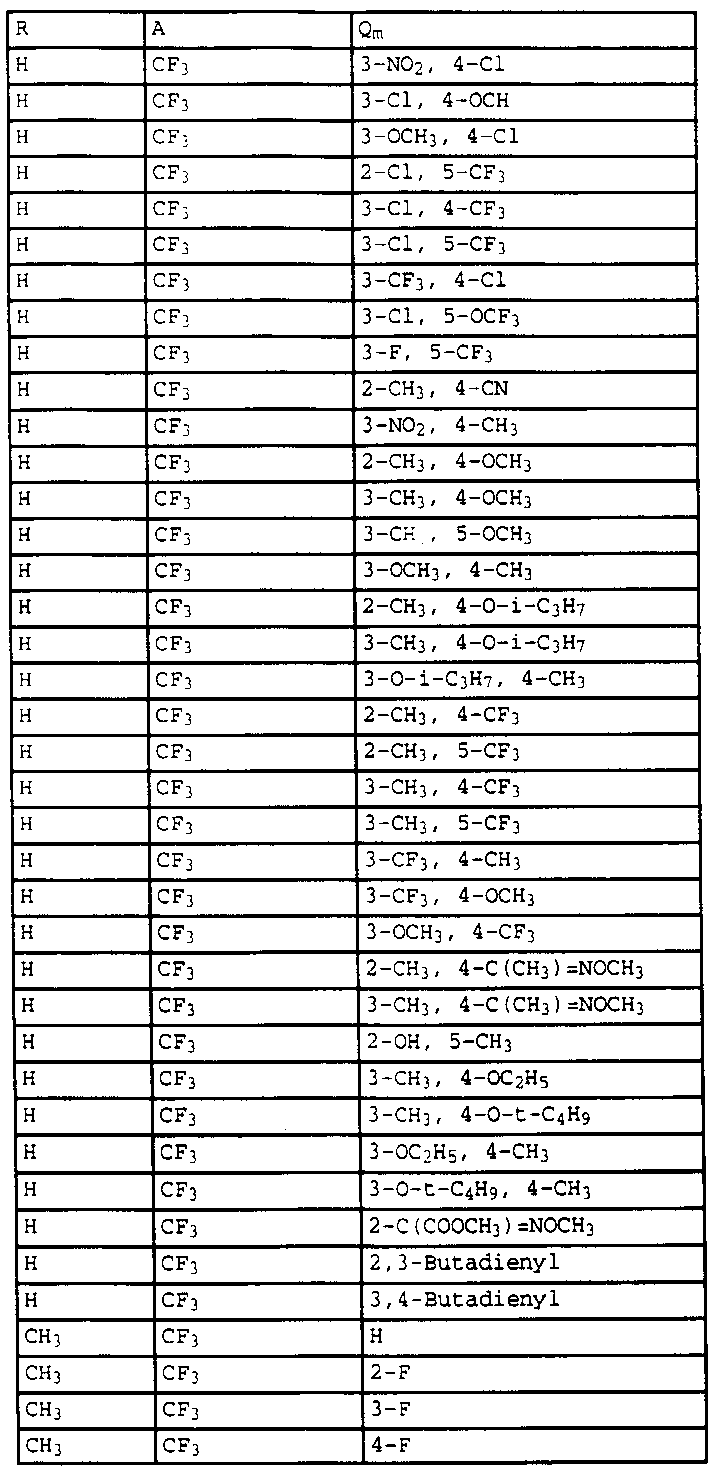

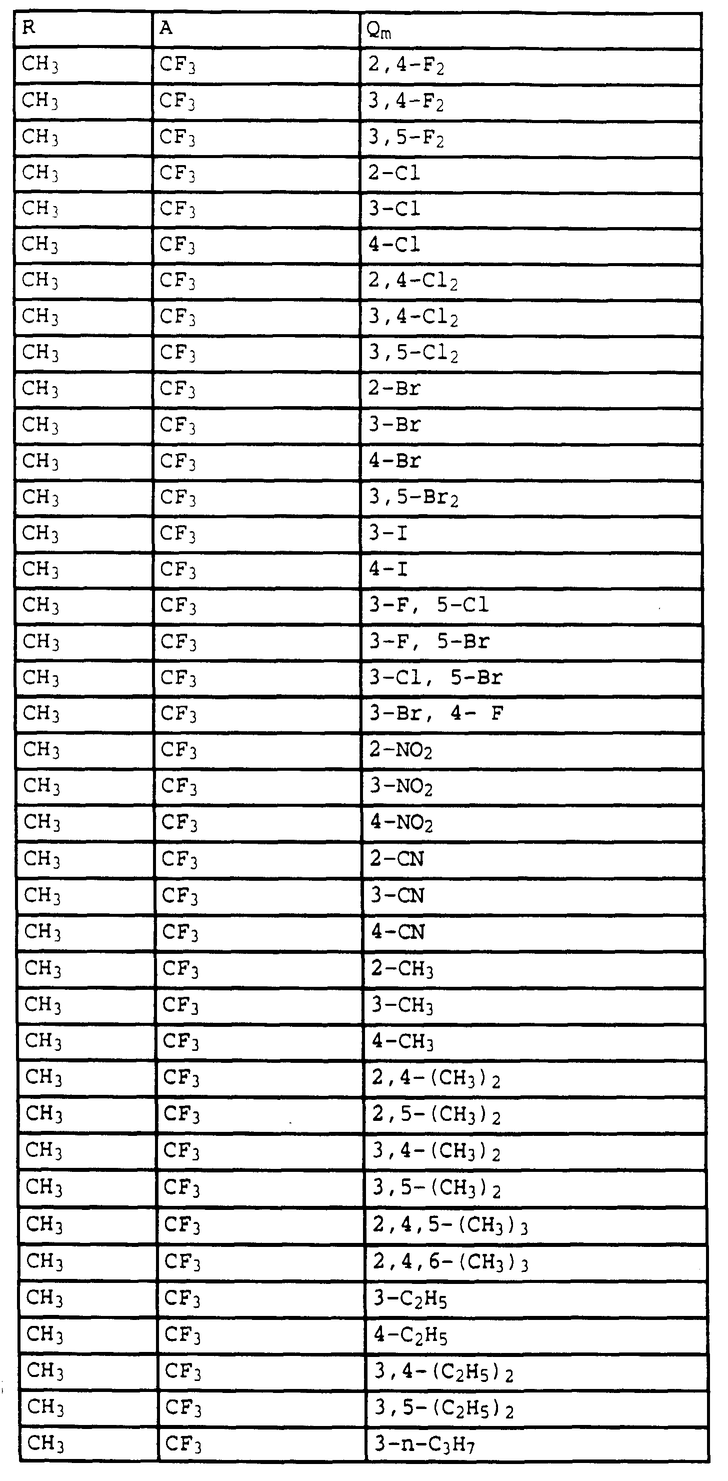

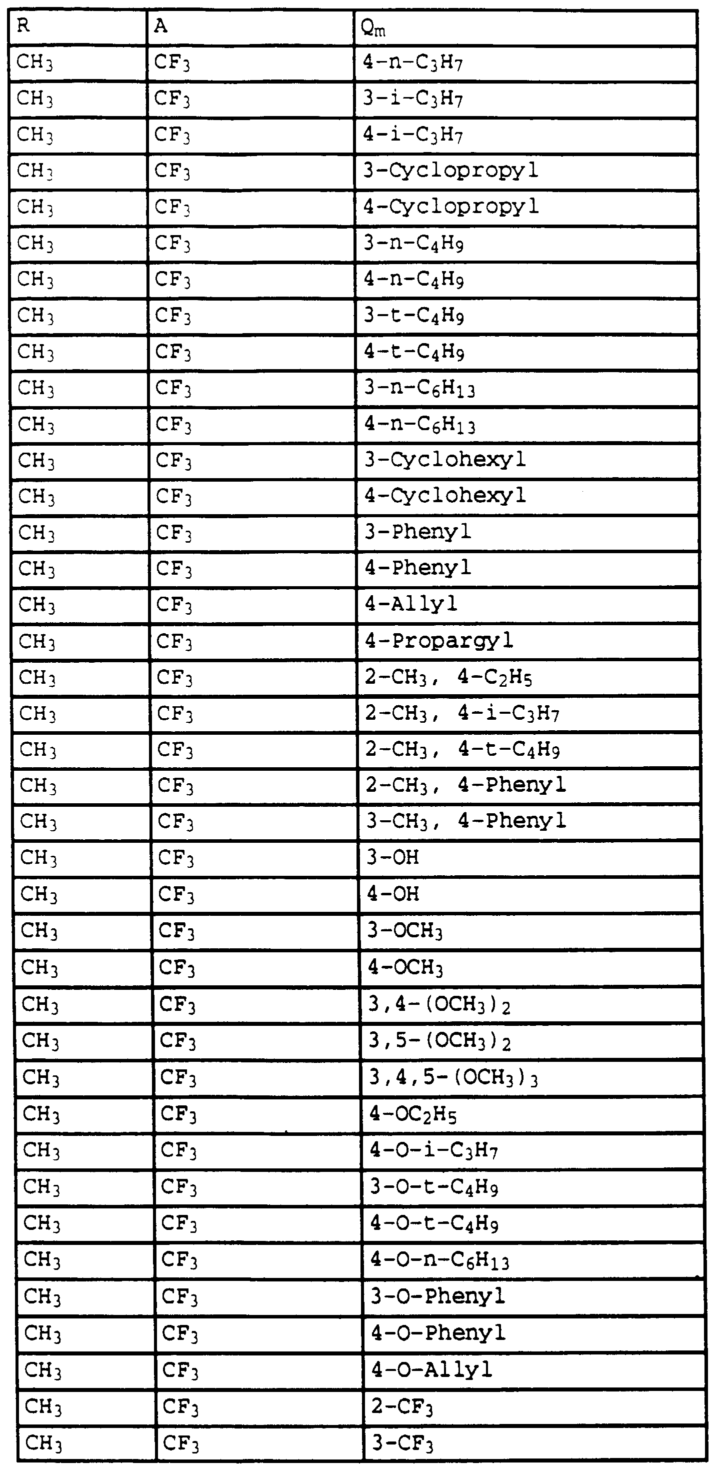

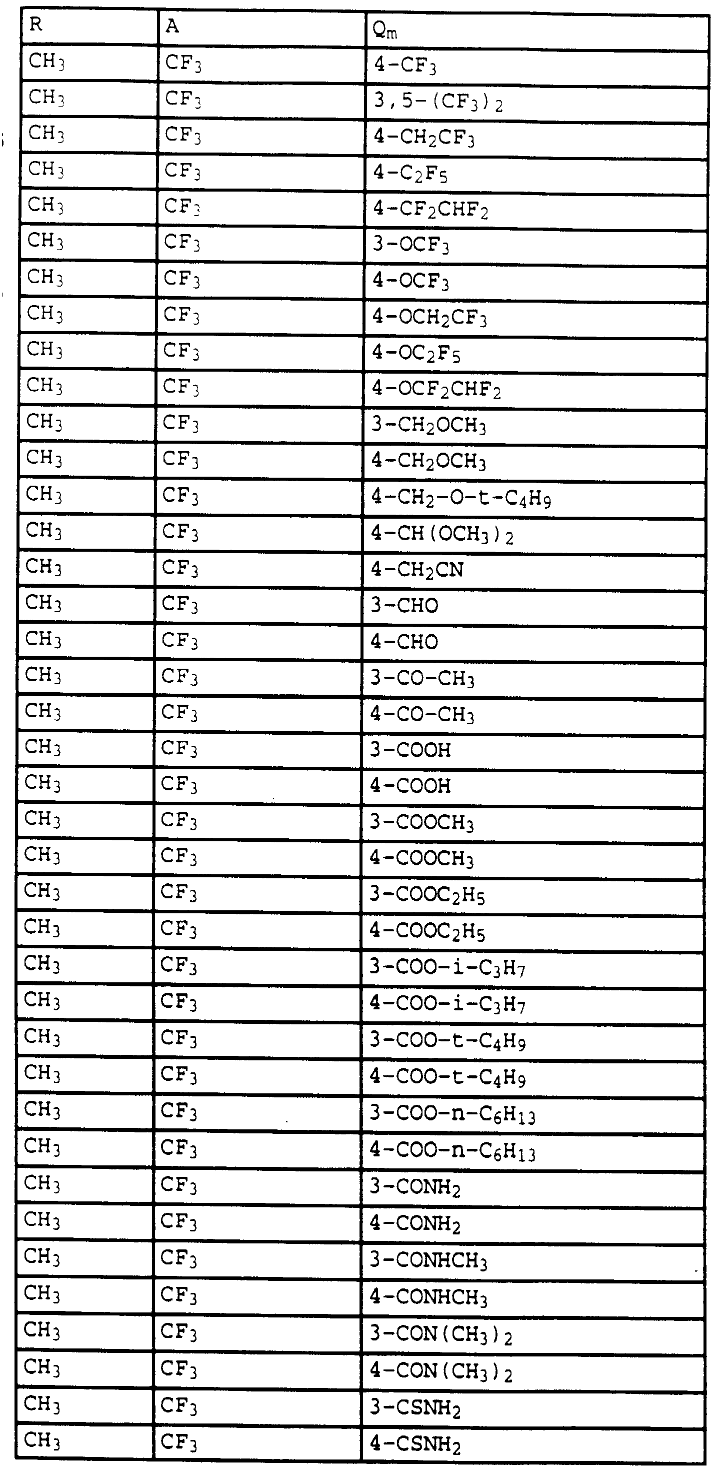

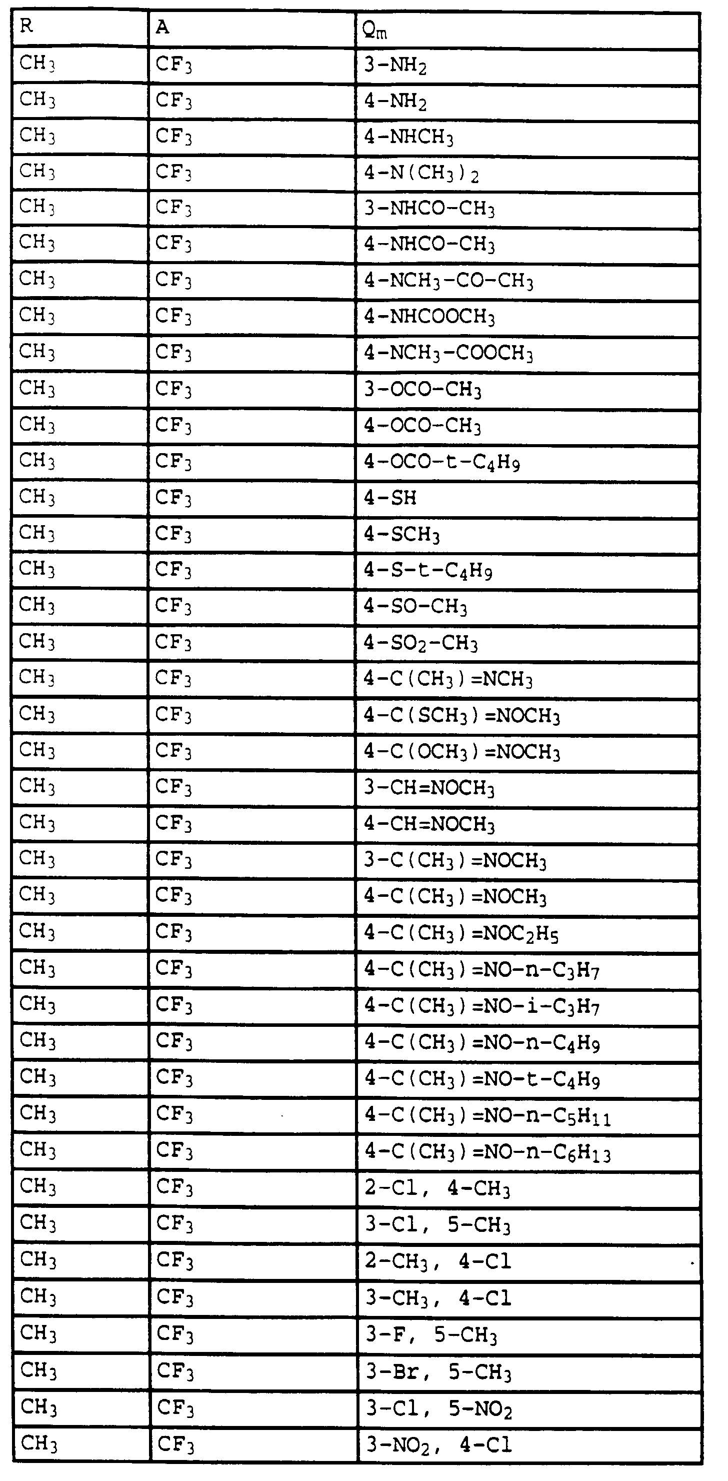

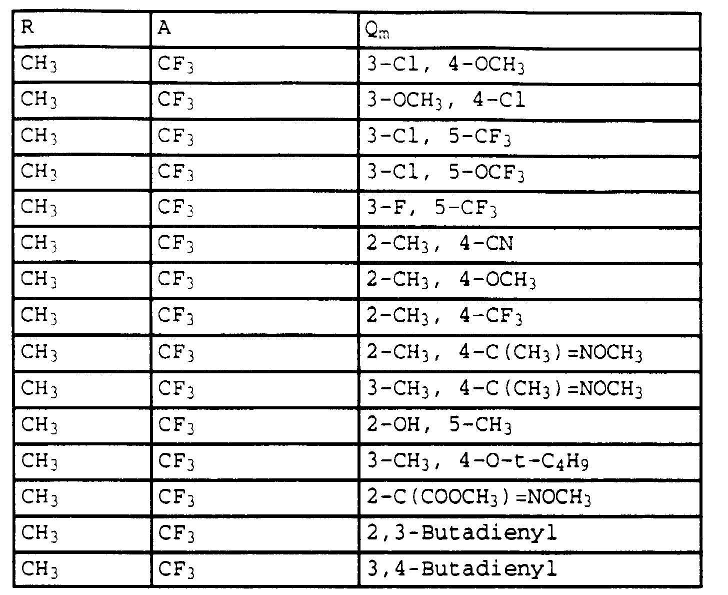

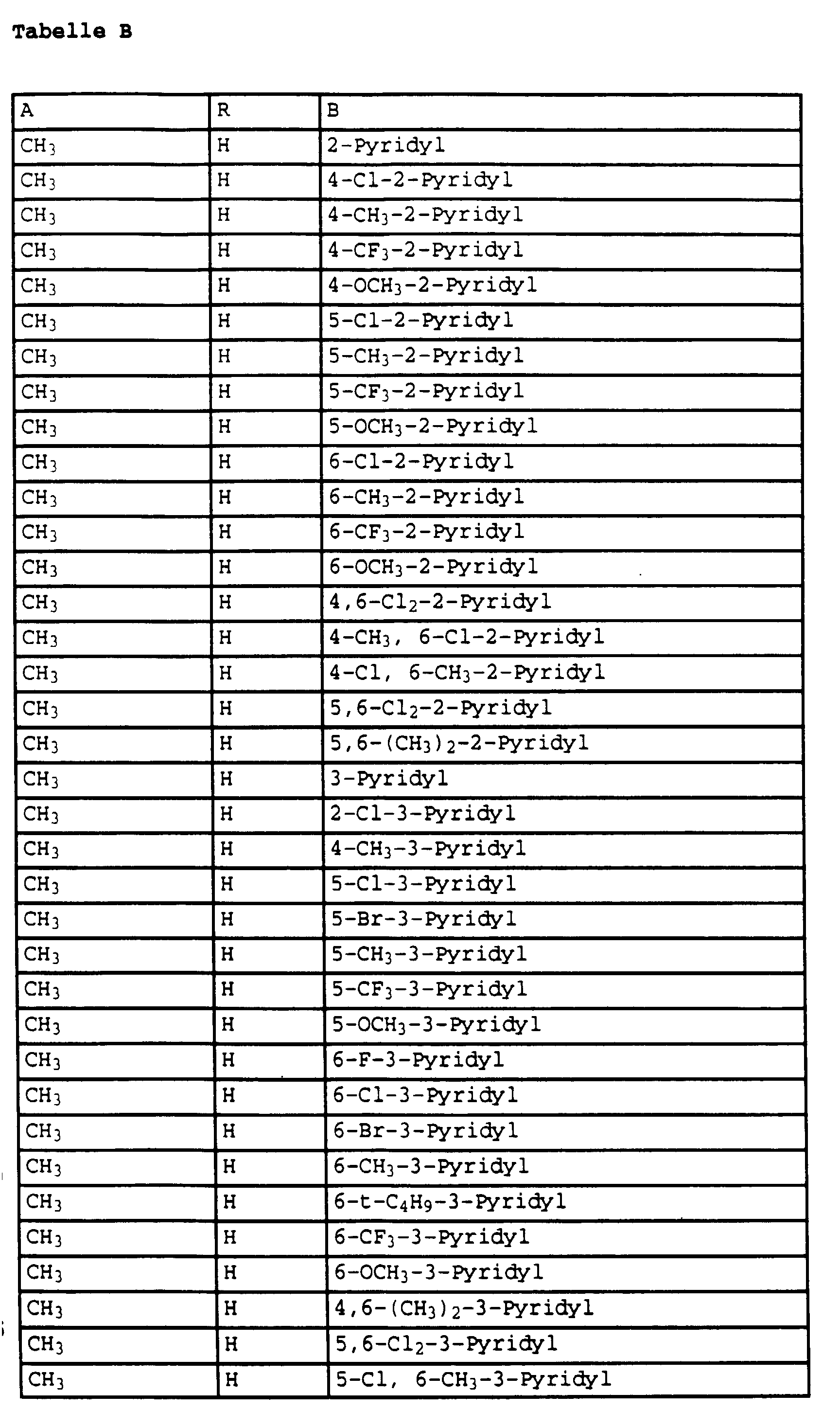

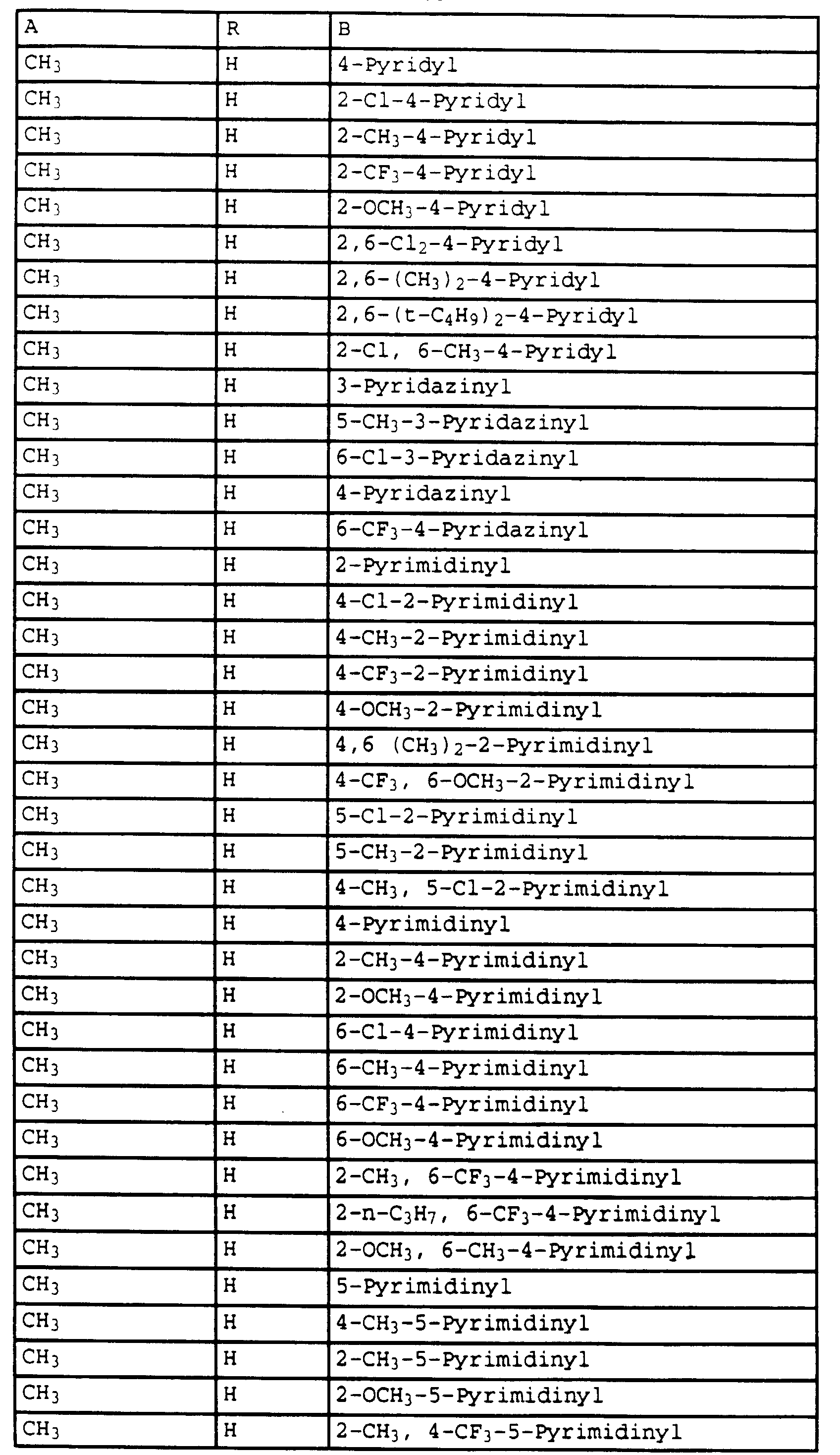

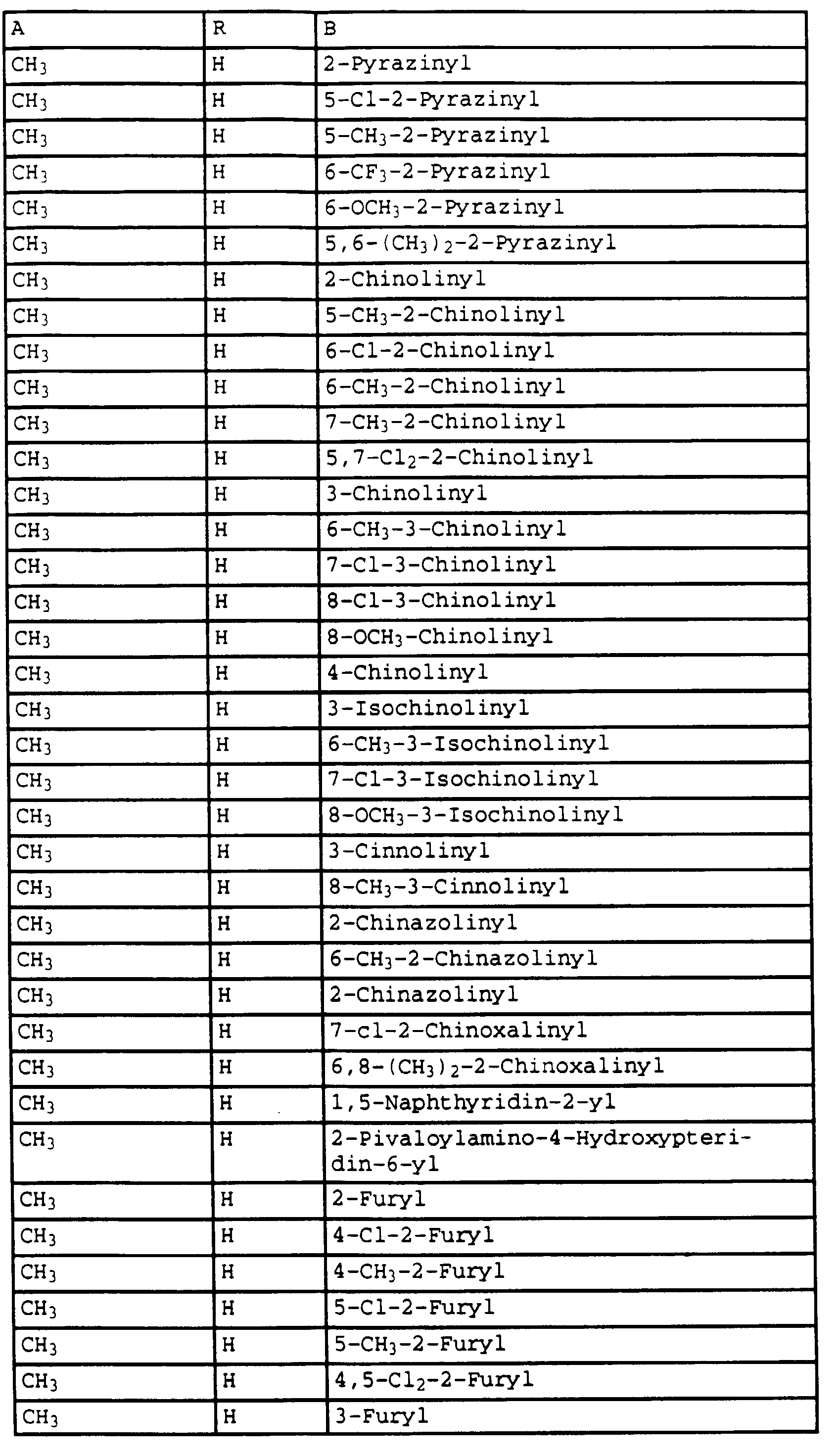

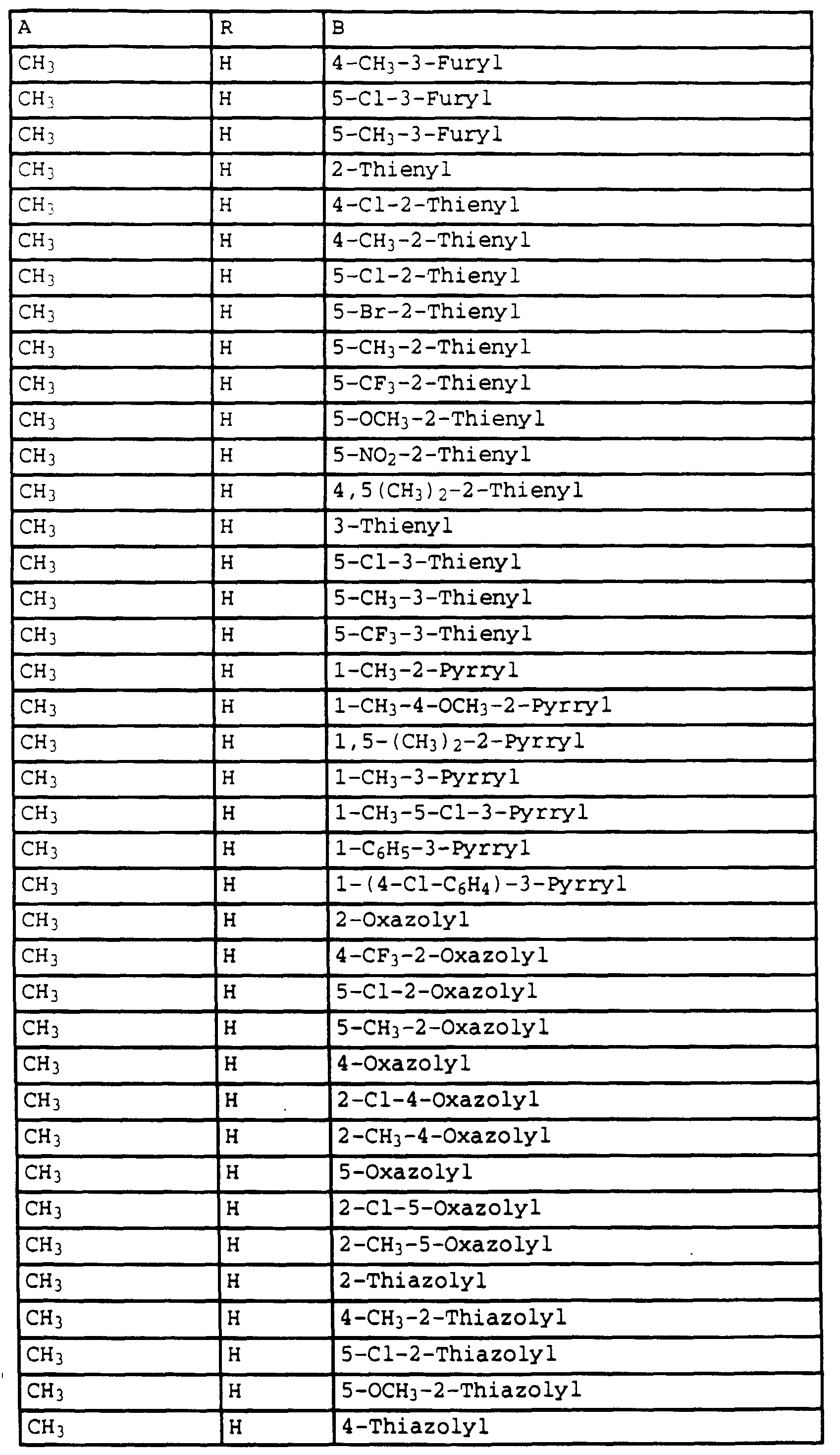

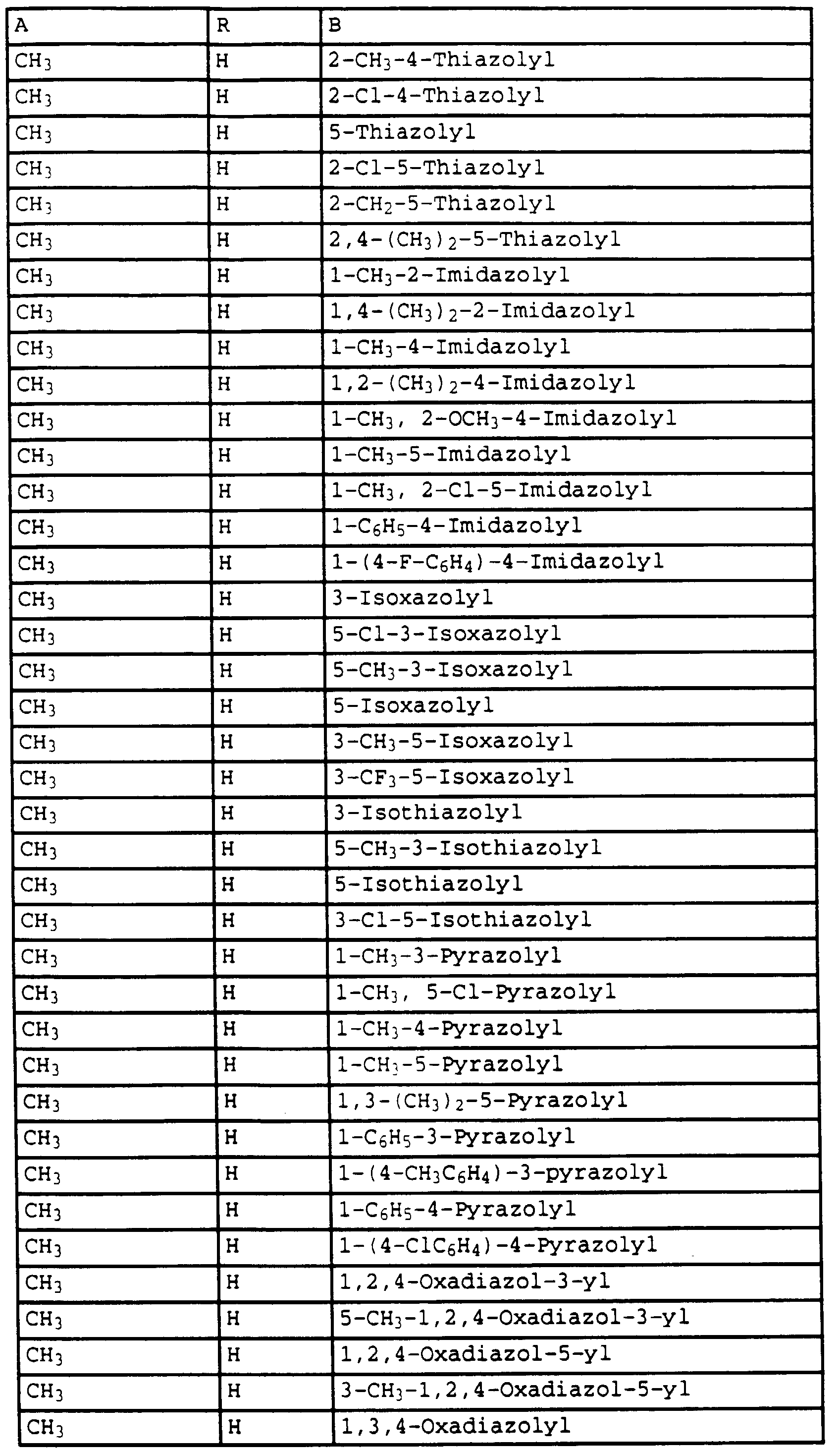

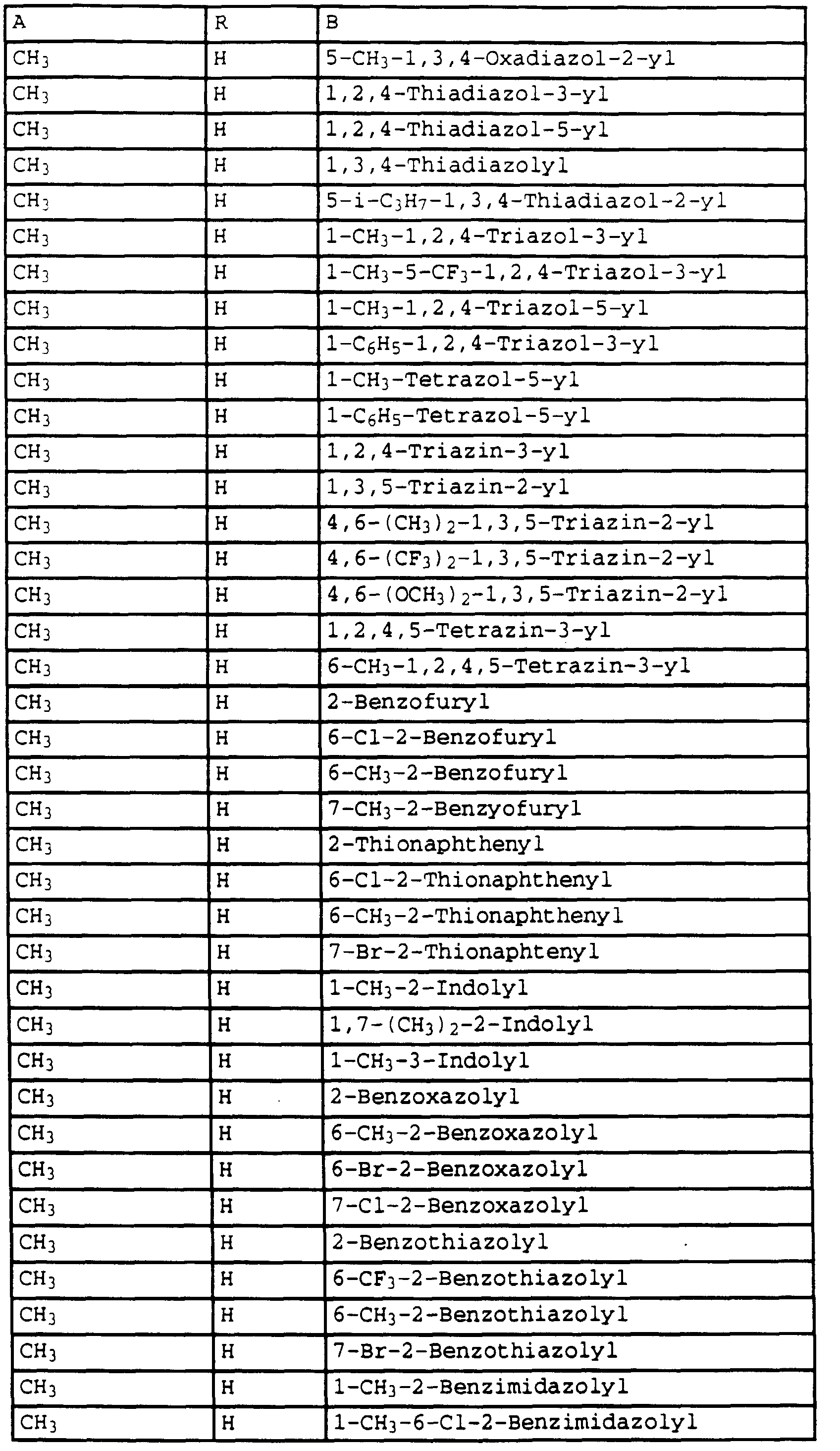

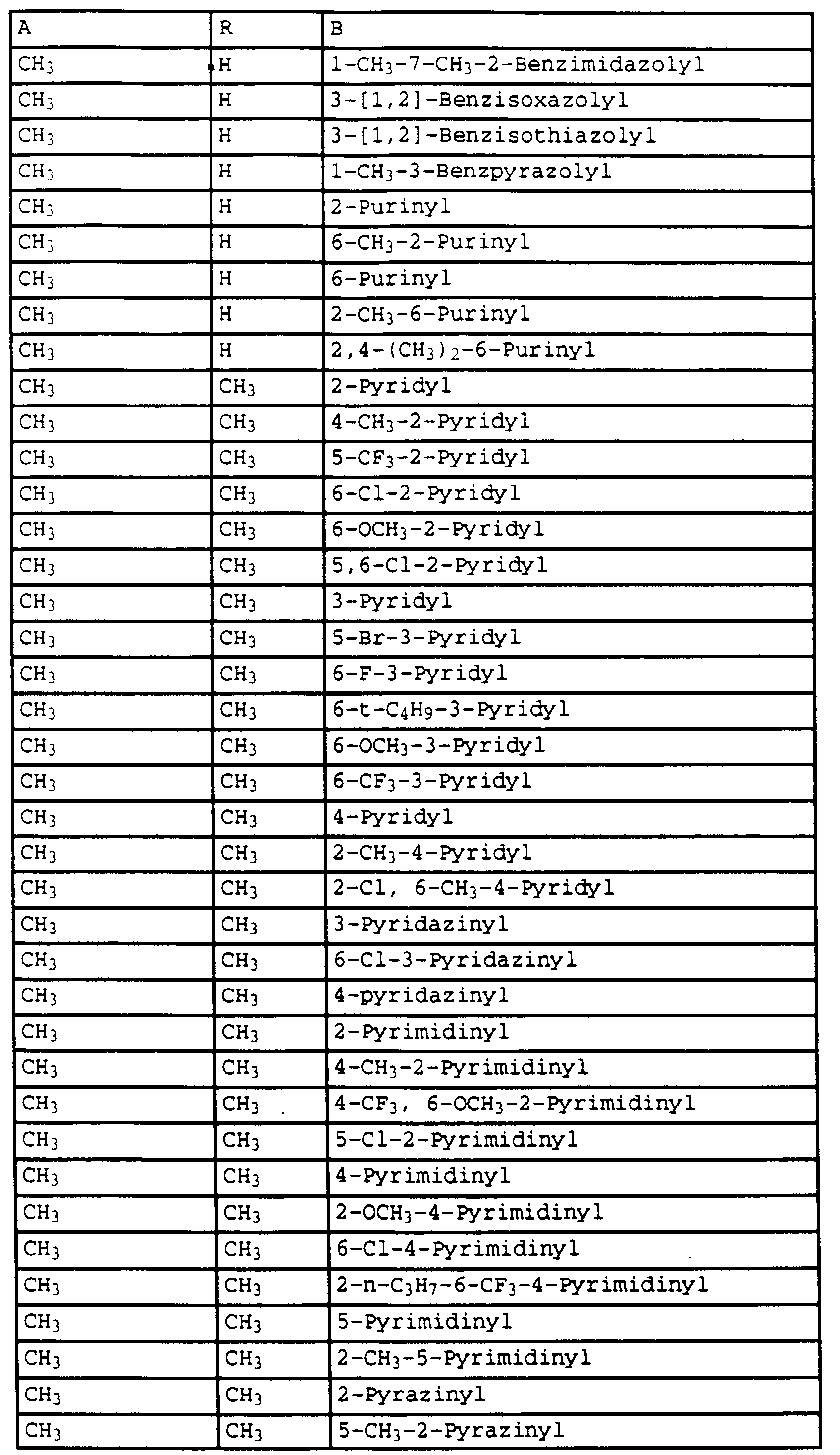

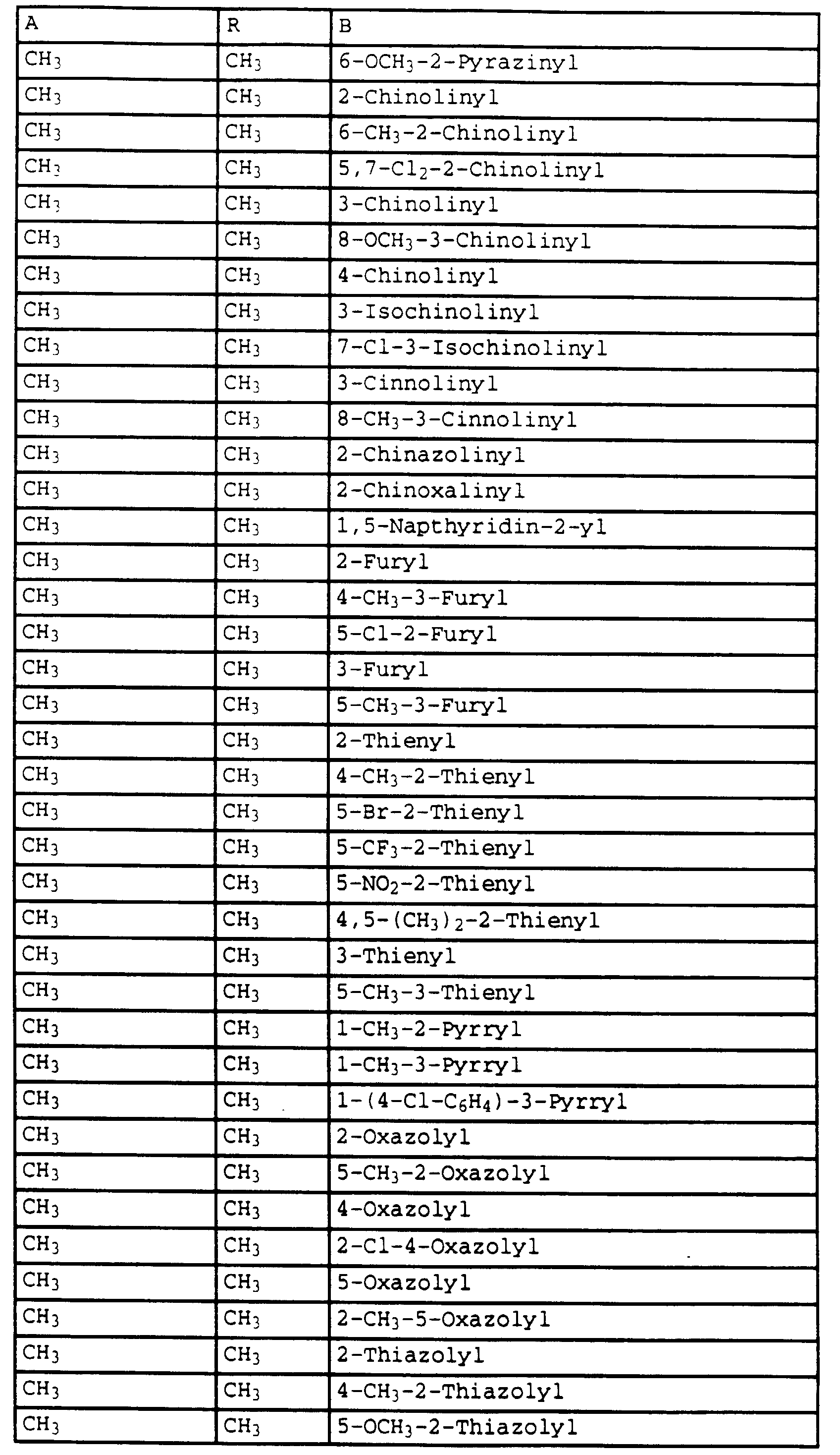

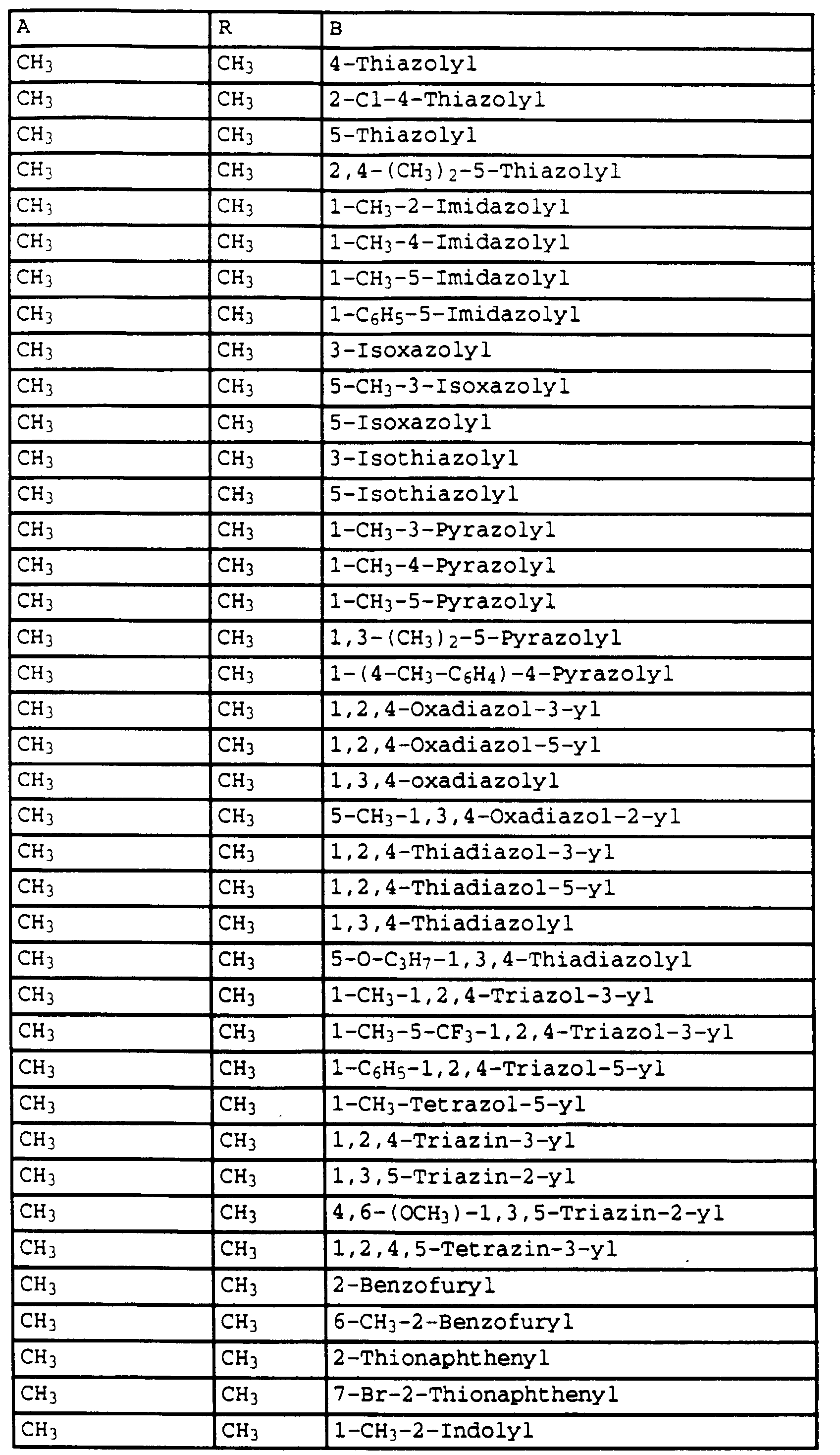

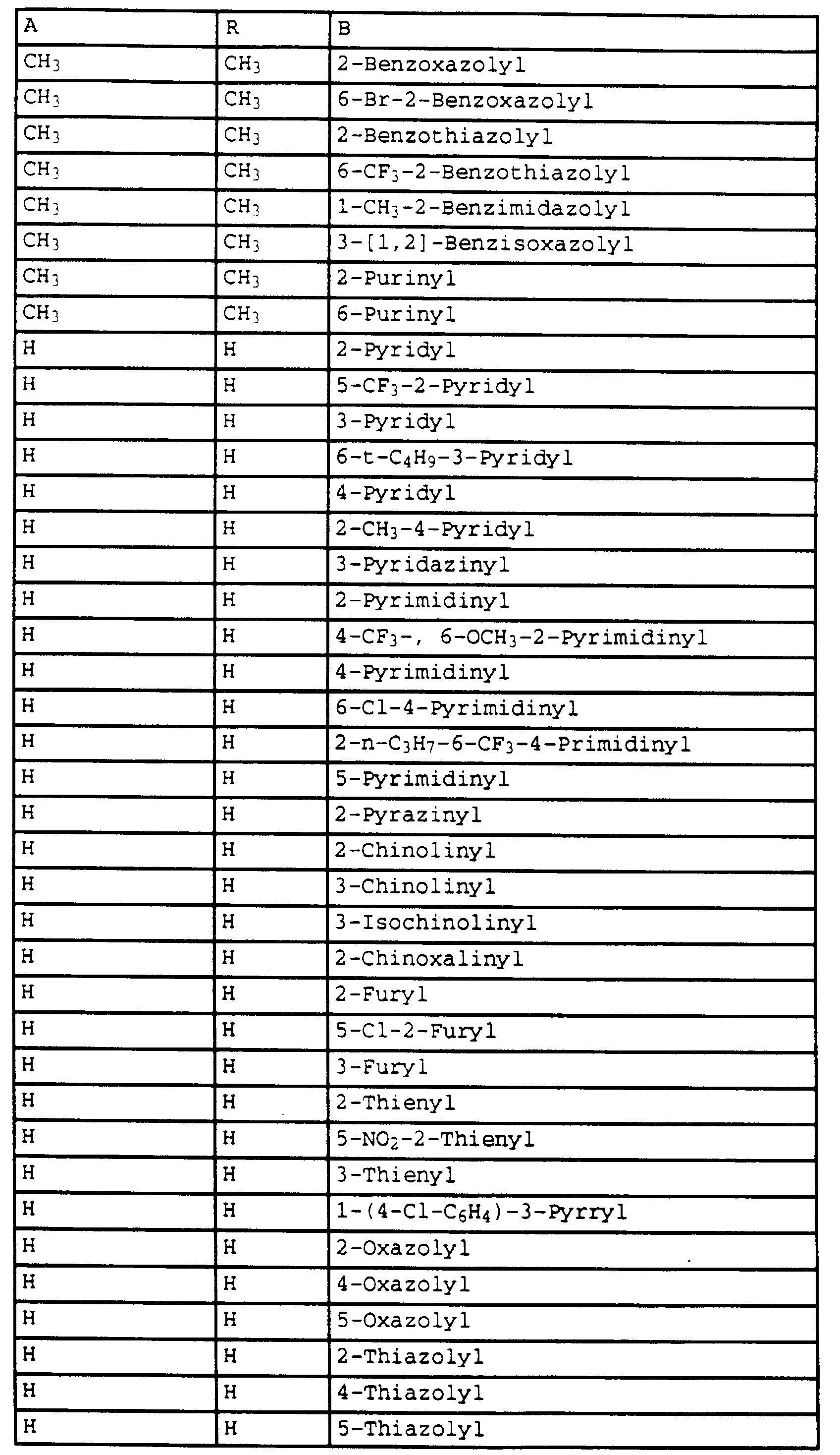

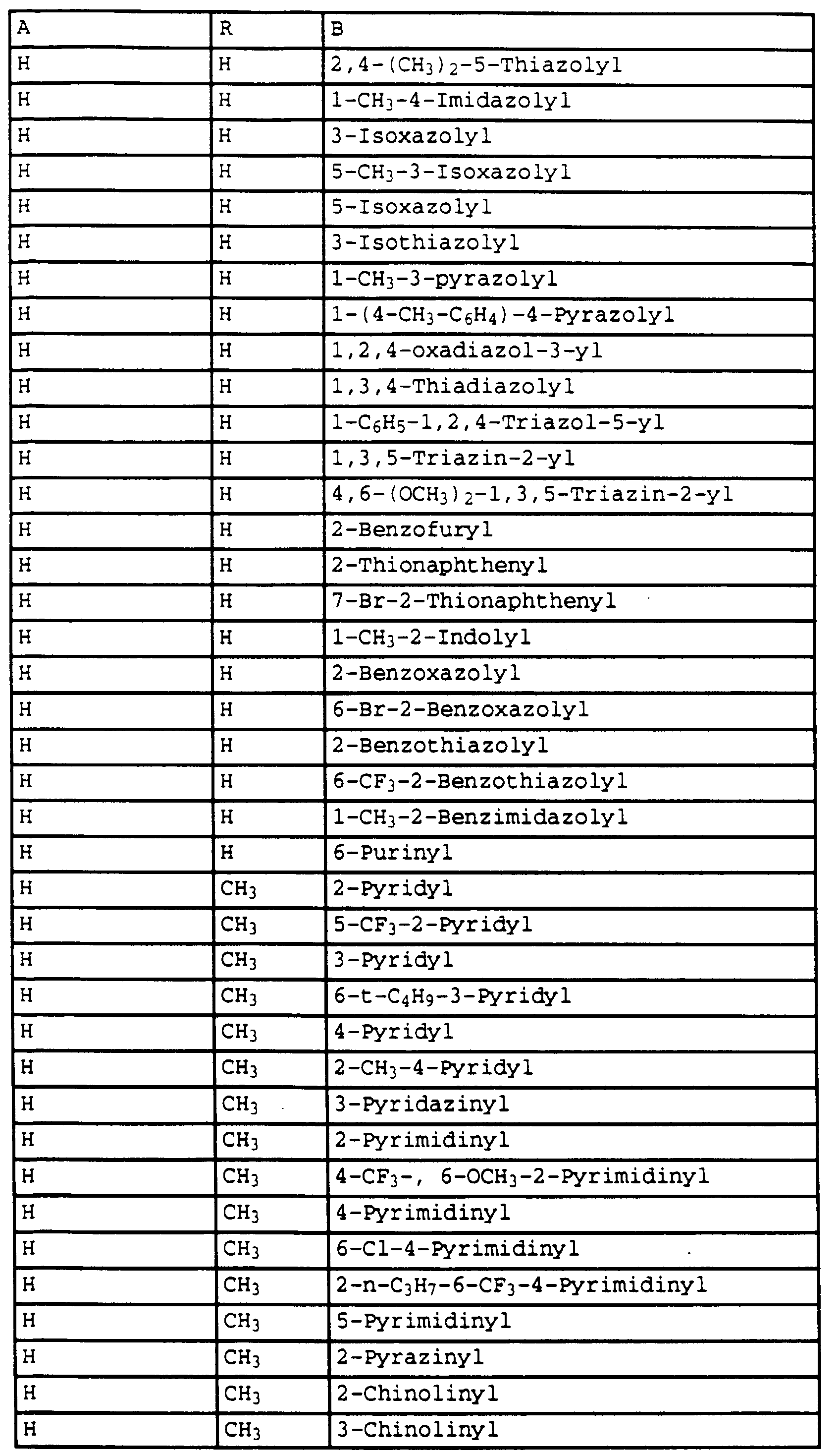

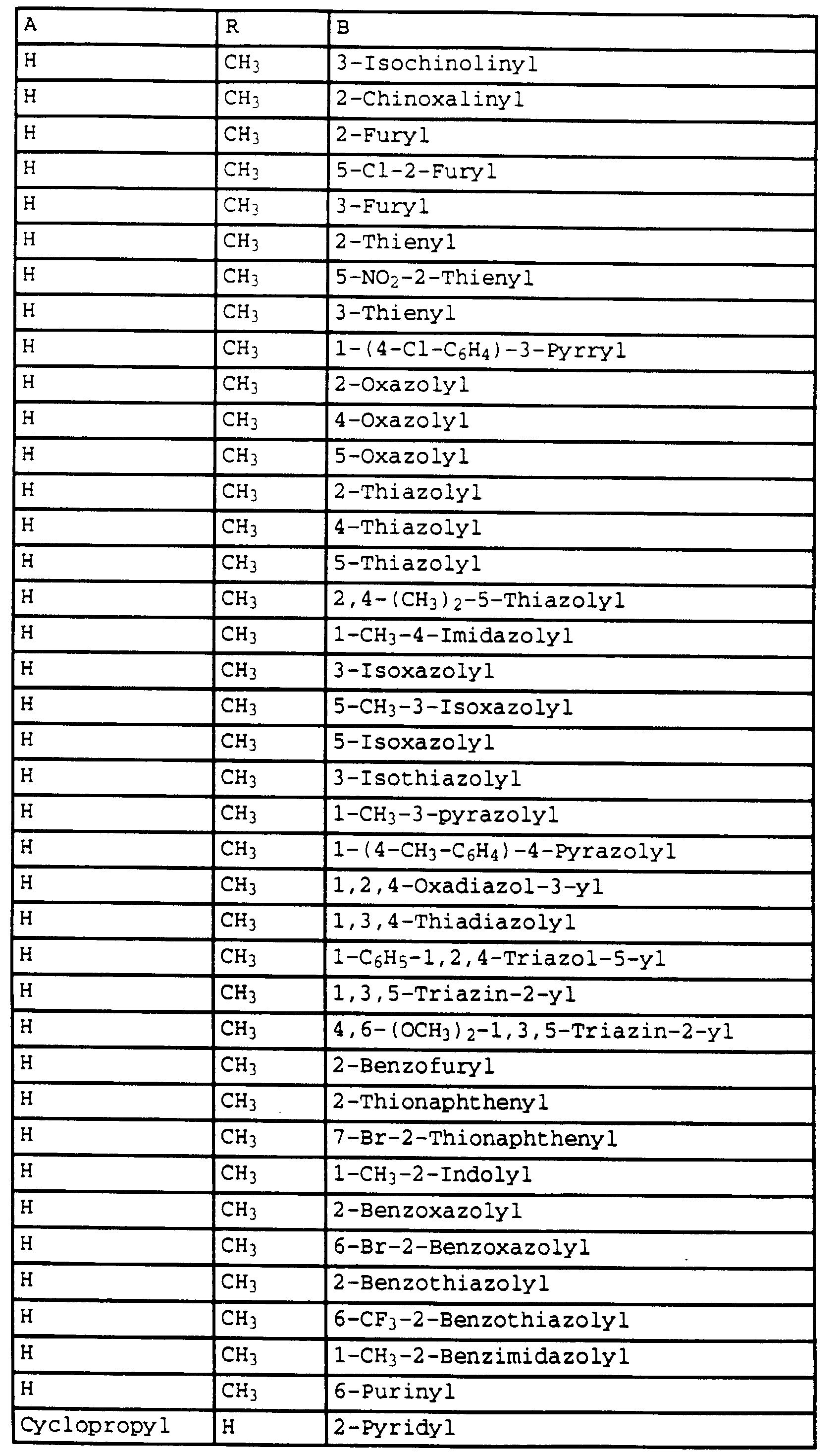

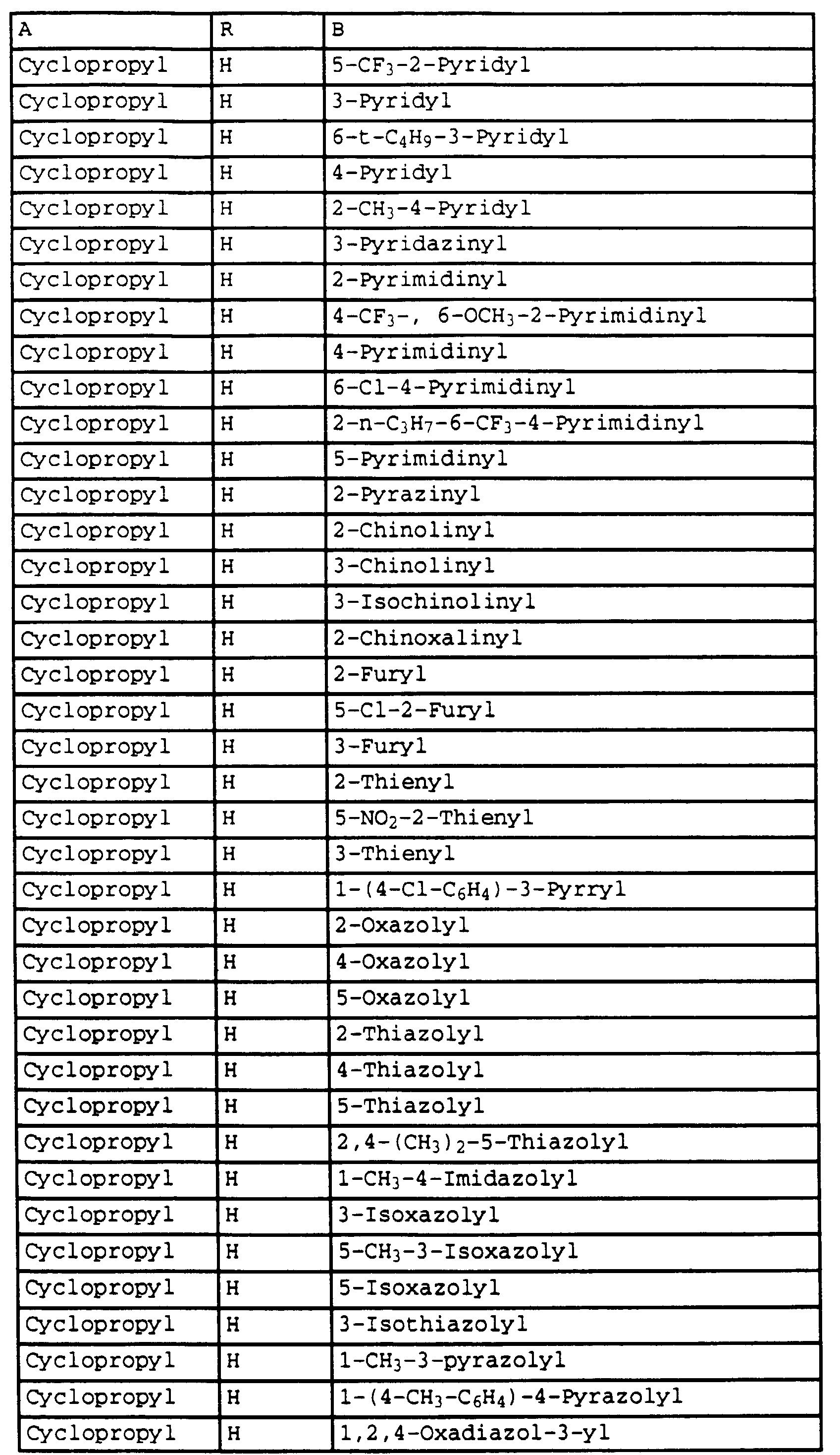

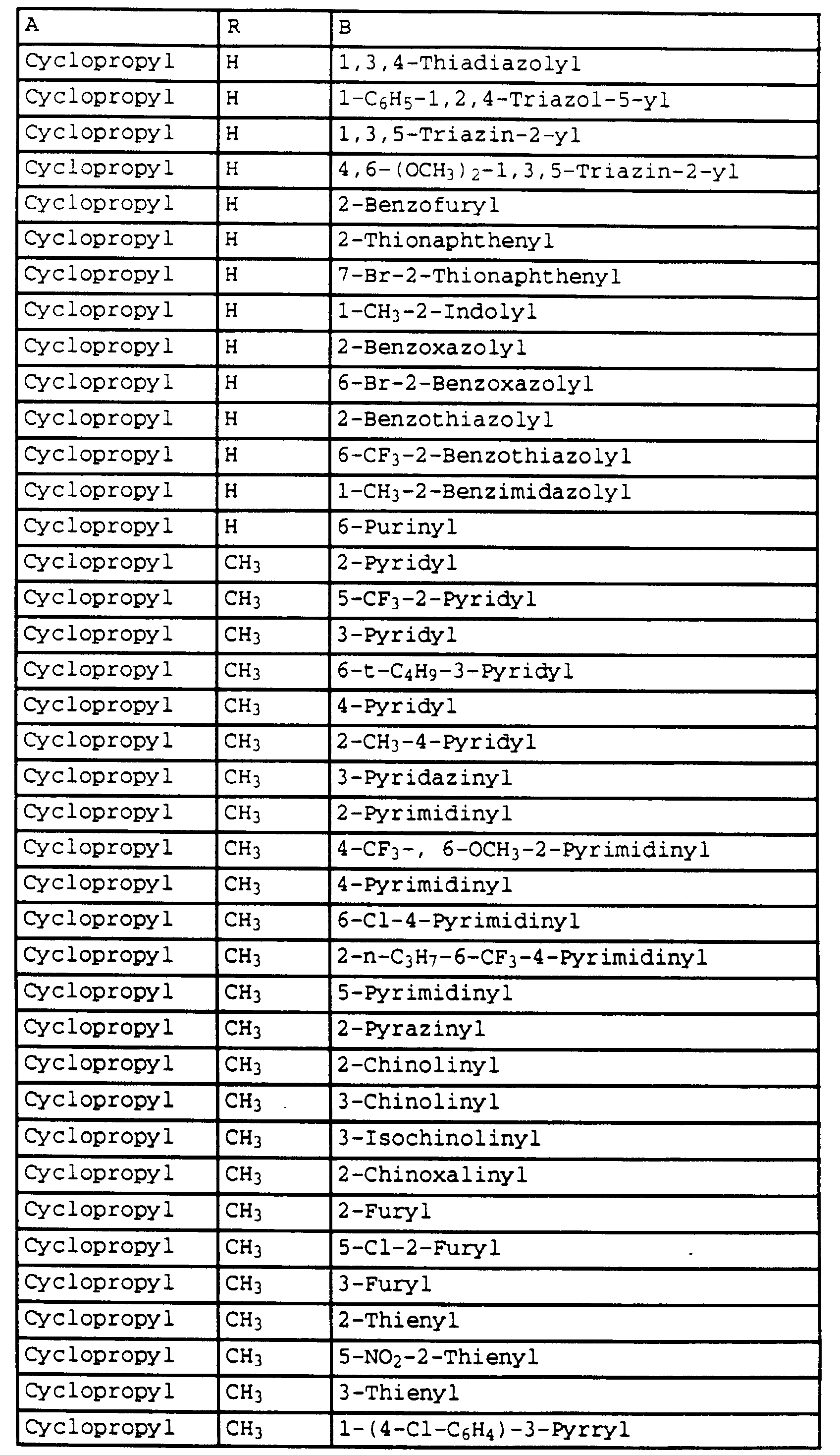

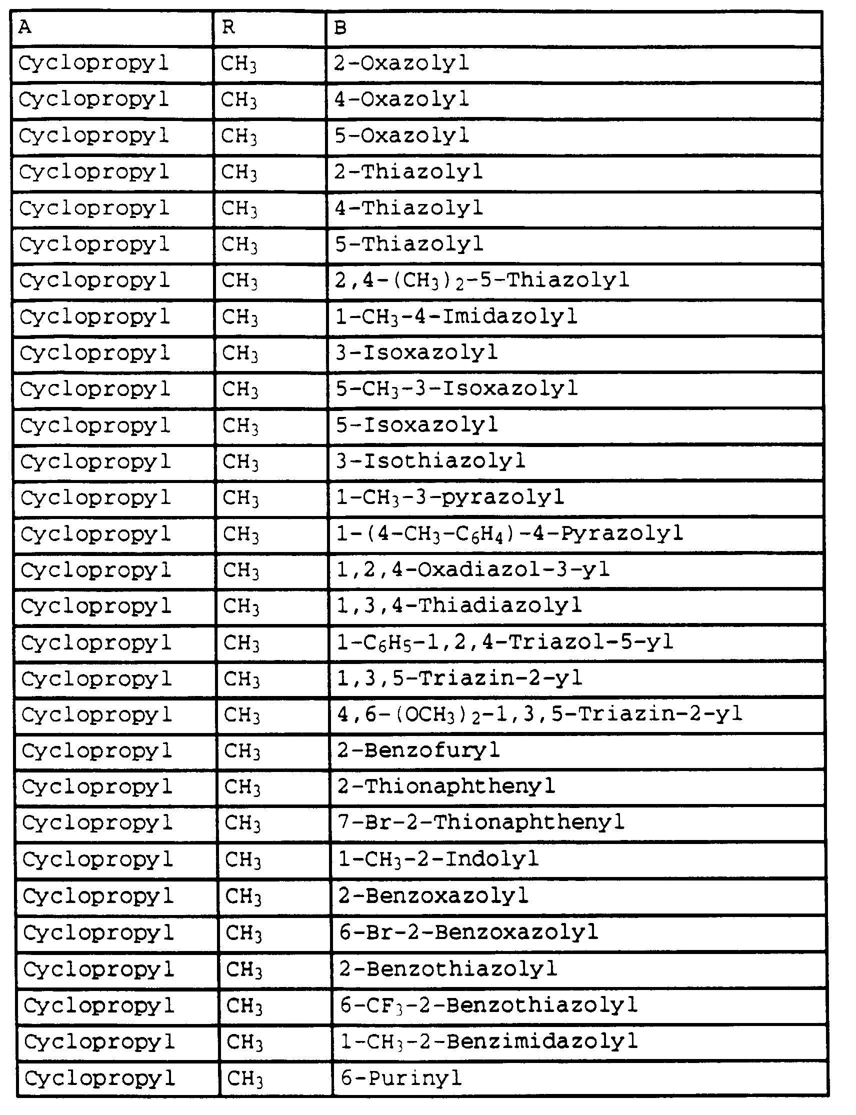

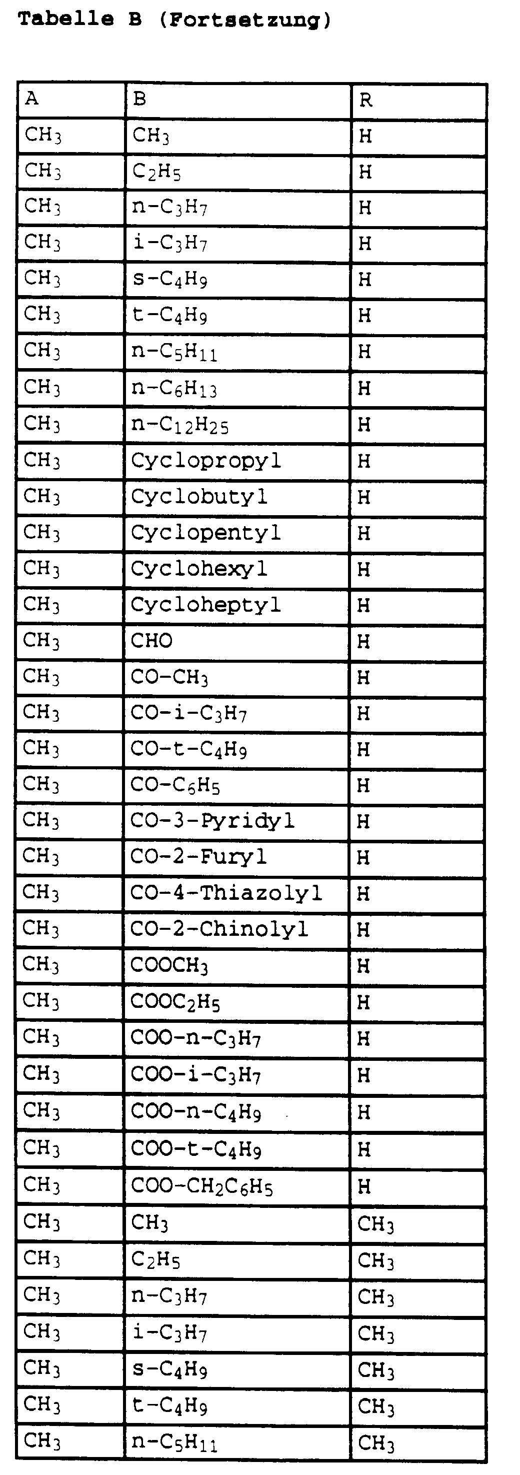

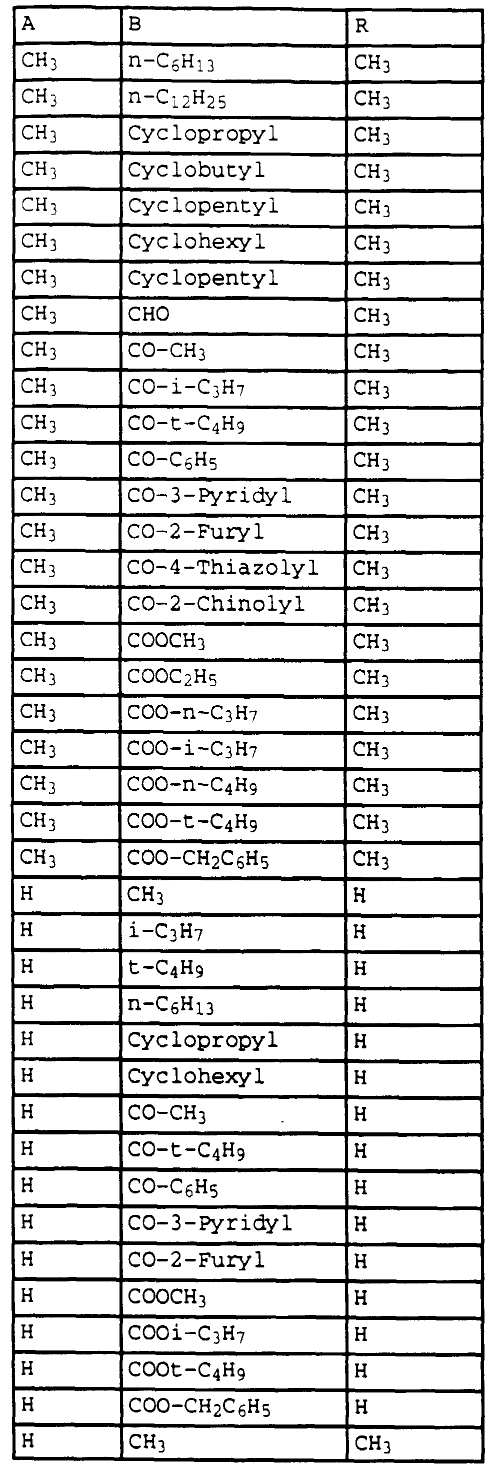

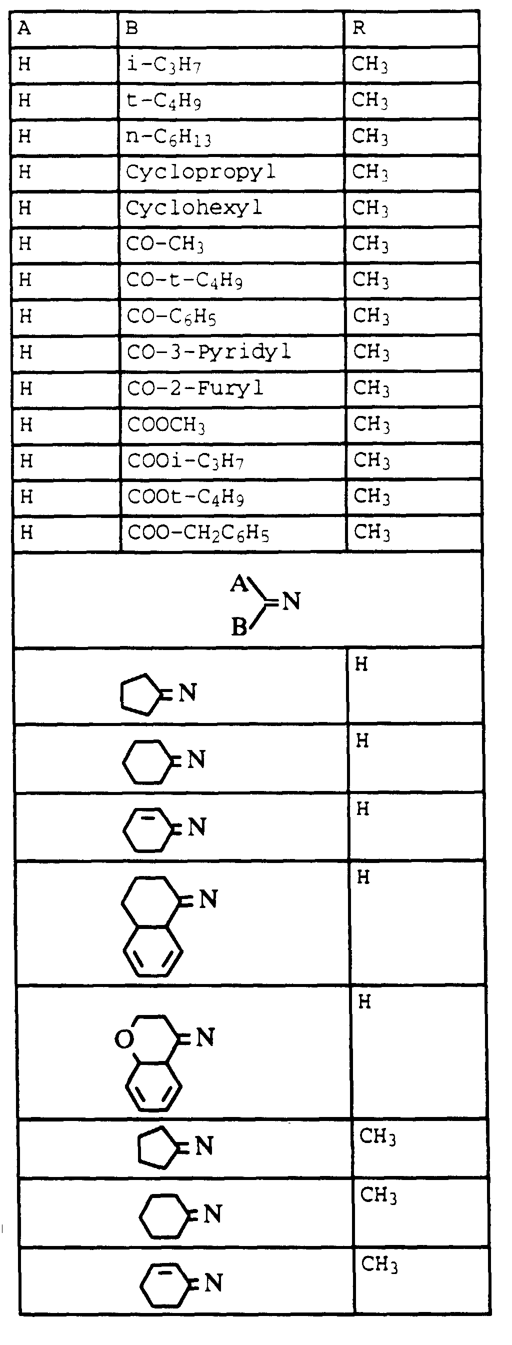

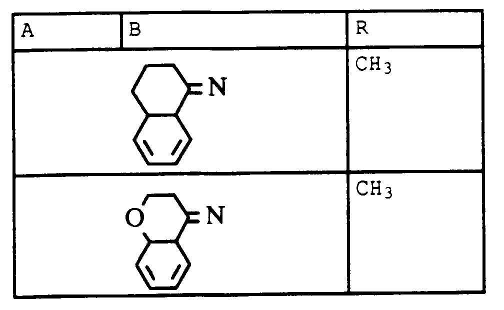

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung des ortho-substituierten Benzylesters einer Cyclopropancarbonsäure gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
Nitroderivate, wie Dinitro-(l-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-l-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diyl-bis-(1-(2,2,2-trichlor-ethyl)-formamid, 1-(3,4-Dichloranilino)-l-formylamino-2,2,2-trichlorethan, 2,6-Dimethyl-N-tridecyl-morpholin bzw. dessen Salze, 2,6-Dimethyl-N-cyclododecylmorpholin bzw. dessen Salze, N-[3-(p-tert.-Butylphenyl)-2-methylpropyl]-cis-2,6-dimethyl- morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-lH- 1,2,4-triazol, l-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl- harnstoff, l-(4-Chlorphenoxy)-3,3-dimethyl-1-(1H-1,2,4-triazol-l-yl)-2- butanon, 1-(4-Chlorphenoxy)-3,3-dimethyl-1-(1H-1,2,4-triazol-l-yl)-2- butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-l,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimins longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
I. 5 Gew.-Teile eines Wirkstoffs werden mit 95 Gew.-Teilen feinteiligem Kaolin innig vermischt. Man erhält auf diese Weise ein Stäubemittel, das 5 Gew.% des Wirkstoffs enthält.
II. 30 Gew.-Teile eines Wirkstoffs werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
III. 10 Gew.-Teile eines Wirkstoffs werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
IV. 20 Gew.-Teile eines Wirkstoffs werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
V. 80 Gew.-Teile eines Wirkstoffs werden mit 3 Gew.-Teilen des Natriumsalzes der Diisobutylnaphthalin-alpha-sinlfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
VI. Man vermischt 90 Gew.-Teile eines Wirkstoffs mit 10 Gew.-Teilen N-Methyl-α-pyrrolidon und erhält eine Lösung, die zur Anwendung in Form kleinster Tropfen geeignet ist (Wirkstoffgehalt 90 Gew.%).
VII. 20 Gew.-Teile eines Wirkstoffs werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
VIII. 20 Gew.-Teile eines Wirkstoffs werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle C mit physikalischen Angaben aufgeführt.

Die folgenden Beispiele sollen die Herstellung der neuen Wirkstoffe erläutern.

### Beispiel 1

### Darstellung von [2-(α-Methoxyimino)-methoxycarbonylmethyl]-benzaldazin (Nr. I.11)

2,2 g (10 mmol) α-Methoxyimino-(2-formylphenyl)-essigsäuremethylester werden in 50 ml Eisessig gelöst und bei Raumtemperatur (RT) 0,25 g (5 mmol) Hydrazinhydrat zugegeben. Die Farbe der Reaktionsmischung wird heller, und nach 5 min Rühren bildet sich ein hellgelber Niederschlag. Man rührt noch 25 min bei RT weiter, saugt den Niederschlag ab und wäscht mit Pentan nach. Man erhält die Titelverbindung als hellgelbes Pulver vom Smp. 207-209°C; Ausbeute 1,6 g (73% d.Th.)

¹H-NMR (CDCl₃, δ-Skala): 3,86 (s, 3 H, COOCH₃); 4,04 (s, 3 H, NOCH₃); 7,29 (mc, 1 H), 7,52 (mc, 2 H) und 7,91 (mc, 1 H; Aromatenprotonen); 8,47 (s, 1 H, CH=N)

### Beispiel 2

### Darstellung von 1-Methyl-1(4'-methylphenyl)-4[2'(α-methoxyimino)-methoxycarbonylmethylen]-phenyl-2,3-diazabutadien (Nr. I.04)

Die zweiphasige Mischung von 10,1 g (200 mmol) 100%igem Hydrazinhydrat, 2,5 ml Wasser und 13,4 g (100 mmol) 4-Methylacetophenon werden 10 h auf 60°C erhitzt. Man läßt abkühlen, versetzt mit Wasser, extrahiert 3 x mit Ether, trocknet die organischen Phasen über Natriumsulfat und engt im Vakuum ein. Man erhält 12,5 g (85 % d.Th.) 4-Methylacetophenon-hydrazon als fast farbloses Öl.

1,6 g des wie oben beschrieben hergestellten 4-Methylacetophenonhydrazons werden in 100 ml Tetrahydrofuran gelöst und bei RT zu einer Lösung von 2,2 g (10 mmol) α-Methoxyimino(2-formylphenyl)-essigsäuremethylester in 30 ml Eisessig getropft. Man rührt 30 min, neutralisiert unter leichter Eiskühlung mit gesättigter Natriumcarbonatlösung, extrahiert mit Diethylether, trocknet über Natriumsulfat, dem eine Spatelspitze Natriumhydrogencarbonat zugesetzt wurde und engt im Vakuum ein. Nach Abtrennung der als Nebenprodukte anfallenden symmetrischen Azine (vgl. Schema 1) durch Säulenchromatographie (Essigester/Cyclohexan 96:4 bis 80:20, Kieselgel 60) erhält man die Titelverbindung als gelbe Kristalle, Smp. 127-129°C, in 1,7 g (48% d.Th.) Ausbeute.

¹H-NMR (CDCl₃, δ-Skala): 2,41 und 2,46 (2 s, je 3 H, Ar-CH₃ und N=C-CH₃); 3,82 (s, 3 H, COOCH₃); 4,04 (s, 3 H, NOCH₃); 7,1-7,3 (m, 3 H), 7,48 (mc, 2 H), 7,78 (d, 2 H) und 7,85 (mc, 1 H; Aromatenprotonen); 8,38 (s, 1 H, CH=N).

### Beispiel 3

### Darstellung von 1-Methyl-1-(4'-methylphenyl)-4[2'-(α-methoxyimino)-methylaminocarbonylmethylen]-phenyl-2,3-diazabutadien (Nr. I.09)

1,0 g (2,9 mmol) 1-Methyl-1-(4'-methylphenyl)-4-[2'-(α-methoxyimino)-methoxycarbonylmethylen[-phenyl-2,3-diazabutadien (Produkt aus Beispiel 2) werden in 20 ml Tetrahydrofuran (THF) gelöst, 1,3 ml 40%iger wäßriger Methylaminlösung (14,5 mmol) zugesetzt und die Reaktionsmischung bei RT über Nacht gerührt; eine geringe Menge eines feinteiligen Feststoffs fällt aus. Man saugt von diesem Niederschlag ab und wäscht mit Pentan nach, wobei im Filtrat die Titelverbindung ausfällt. Durch Zugabe weiteren Pentans wird die Fällung vervollständigt. Der Niederschlag wird abgesaugt, mit Pentan gewaschen und durch Stehenlassen an Luft getrocknet.Blaßgelbe Kristalle, Smp. 182-185°C, Ausbeute 0,8 g (80% d.Th.).

¹H-NMR (CDCl₃, δ-Skala): 2,42 und 2,47 (2 s, je 3 H, Ar-CH₃ und N=C-CH₃); 2,93 (d, 3 H, CONHCH₃); 3,96 (s, 3 H, NOCH₃); 6,87 (br, 1 H, CONH); 7,24 (mc, 3 H), 7,50 (mc, 2 H), 7,80 (d, 2 H) und 8,03 (mc, 1 H; Aromatenprotonen); 8,27 (s, 1 H, CH=N).

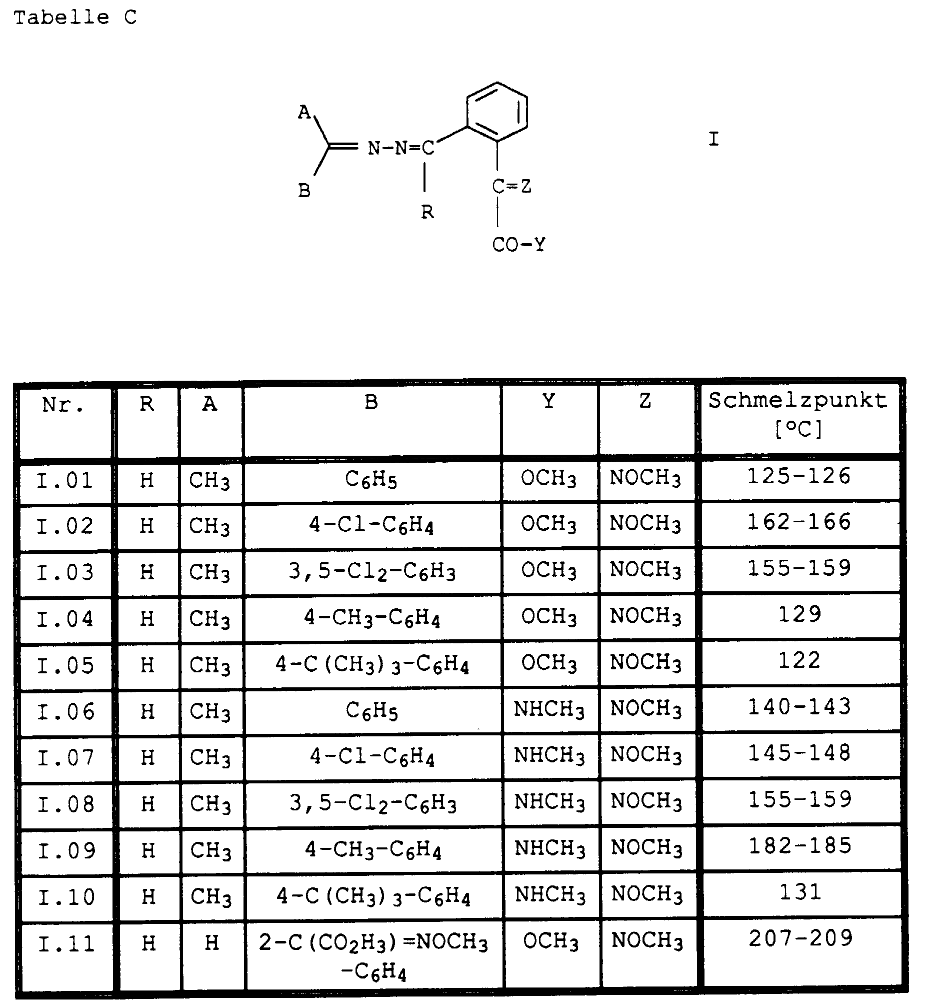

### Beispiele für die Wirkung gegen Schadpilze

Die fungizide Wirkung der Verbindungen der allgemeinen Formel I ließ sich durch die folgenden Versuche zeigen:
Die Wirkstoffe wurden als 20%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### A. Botrytis cinerea

Scheiben von grünen Paprikaschoten wurden mit der Wirkstoffaufbereitung tropfnaß gespritzt. Nach dem Abtrocknen wurden die Fruchtscheiben mit einer Sporensuspension des Pilzes Botrytis cinerea (1,7 x 10⁶ Sporen pro ml einer 2%-igen Biomalzlösung) besprüht und 4 Tage bei 18°C bei hoher Luftfeuchtigkeit bewahrt. Die Auswertung erfolgte visuell.

In diesem Test zeigten die unbehandelten Kontrollen einen Befall von 90% während die jeweils mit 500 ppm-haltiger Wirkstoffaufbereitung der Verbindungen I.01, I.04, I.06 und I.08 behandelten Fruchtscheiben maximal zu 15% befallen waren.

### B. Plasmopara viticola

Topfreben (Sorte: "Müller Thurgau") wurden mit der Wirkstoffaufbereitung tropfnaß gespritzt. Nach 8 Tagen wurden die Pflanzen mit einer Zoosporenaufschwemmung des Pilzes Plasmopara viticola besprüht und 5 Tage bei 20-30°C bei hoher Luftfeuchtigkeit bewahrt. Vor der Beurteilung wurden die Pflanzen danach für 16 h bei hoher Luftfeuchtigkeit bewahrt. Die Auswertung erfolgte visuell.

In diesem Test zeigten die unbehandelten Kontrollen einen Befall von 70% während die jeweils mit 63 ppm-haltiger Wirkstoffaufbereitung der Verbindungen I.01, I.03, I.04, I.06, I.07, I.08 und I.09 behandelten Pflanzen maximal zu 15% befallen waren.

### Beispiele für die Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a. als 0,1%-ige Lösung in Aceton oder
b. als 10%-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a. bzw. mit Wasser im Fall von b. verdünnt.

Nach Abschluß der Versuche wurde die jeweils niedrigste Konzentration ermittelt, bei der die Verbindungen im Vergleich zu unbehandelten Kontrollversuchen noch eine 80 bis 100%-ige Hemmung bzw. Mortalität hervorriefen (Wirkschwelle bzw. Minimalkonzentration).

## Patentansprüche

1. Verbindungen der Formel I, 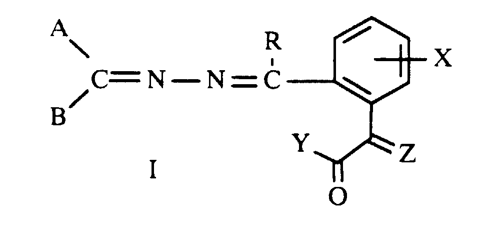 in der die Substituenten die folgende Bedeutung haben:
X Wasserstoff, Cyano, Nitro, Trifluormethyl, Halogen, C₁-C₆-Alkyl oder C₁-C₆-Alkoxy;
Y Methoxy, Amino, Methylamino oder Dimethylamino;
Z CHCH₃, CHOCH₃ oder NOCH₃;
R Wasserstoff oder C₁-C₆-Alkyl;
A Wasserstoff, C₁-C₆-Alkyl, C₁-C₆-Haloalkyl oder C₃-C₇-Cycloalkyl:
B gegebenenfalls substituiertes C₁-C₆-Alkyl, C₂-C₆-Alkenyl, C₂-C₆-Alkinyl, C₃-C₇-Cycloalkyl, C₅-C₇-Cycloalkenyl;
gegebenenfalls substituiertes gesättigtes oder partiell ungesättigtes Heterocyclyl;
gegebenenfalls substituiertes, gegebenenfalls kondensiertes Aryl;
gegebenenfalls substituiertes, gegebenenfalls benzo- oder heterokondensiertes 5- oder 6-gliedriges Heteroaryl;
gegebenenfalls substituiertes Aryl-C₁-C₆-Alkyl oder Heteroaryl-C₁-C₆-Alkyl, oder C(=O)W;
wobei
W Wasserstoff,
gegebenenfalls substituiertes C₁-C₆-Alkyl,
C₁-C₆-Alkoxy, C₁-C₆-Alkylthio, C₁-C₆-Alkylamino oder Di-C₁-C₆-alkylamino,
gegebenenfalls substituiertes Aryl, Heteroaryl, Aryloxy, Arylthio, Arylamino, Aryl-N-(C₁-C₆-Alkyl)-amino, Heteroaryloxy, Heteroarylthio, Heteroarylamino oder Heteroaryl-N-(C₁-C₆-Alkyl)amino;
oder
A und B zusammen mit dem C-Atom, dessen Substituenten sie sind, einen gegebenenfalls substituierten, gegebenenfalls annellierten, gesättigten oder ungesättigten 3- bis 7-gliedrigen isocyclischen oder 5-bis 7-gliedrigen heterocyclischen Ring, der auch chinonoid sein kann.

2. Verfahren zur Herstellung der Verbindungen der allgemeinen Formel **I** gemäß Anspruch 1, dadurch gekennzeichnet, daß man eine Carbonylverbindung der allgemeinen Formel **II** 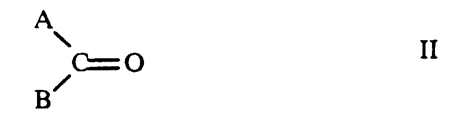 mit einer Carbonylverbindung der allgemeinen Formel **III** 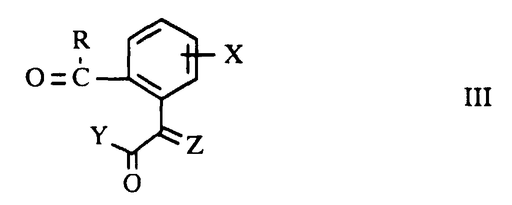 und mit Hydrazin, einem seiner Säureadditionssalze oder seinem Hydrat umsetzt.

3. Verfahren zur Herstellung der Verbindungen der allgemeinen Formel **I** gemäß Anspruch 1, dadurch gekennzeichnet, daß man eine Carbonylverbindung der allgemeinen Formel **III** gemäß Anspruch 2 mit einem Hydrazon **IV** 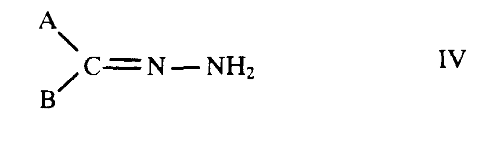 oder einem seiner Säureadditionssalze umsetzt.

4. Zur Bekämpfung von Schädlingen oder Schadpilzen geeignetes Mittel, enthaltend einen festen oder flüssigen Trägerstoff und eine Verbindung der allgemeinen Formel I gemäß Anspruch 1.

5. Verwendung der Verbindungen I gemäß Anspruch 1 zur Herstellung eines zur Bekämpfung von Schädlingen oder Schadpilzen geeigneten Mittels.

6. Verfahren zur Bekämpfung von Schadpilzen, dadurch gekennzeichnet, daß man die Pilze oder die vor Pilzbefall zu schützenden Materialien, Pflanzen, Boden oder Saatgüter mit einer wirksamen Menge einer Verbindung der allgemeinen Formel I gemäß Anspruch 1 behandelt.

7. Verfahren zur Bekämpfung von Schädlingen, dadurch gekennzeichnet, daß man die Schädlingen oder die von ihnen zu schützenden Materialien, Pflanzen, Boden oder Saatgüter mit einer wirksamen Menge einer Verbindung der allgemeinen Formel I gemäß Anspruch 1 behandelt.

## Claims

1. A compound of the formula **I** 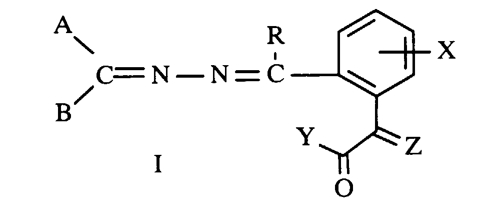 where the substituents have the following meanings
X is hydrogen, cyano, nitro, trifluoromethyl, halogen, C₁-C₆-alkyl or C₁-C₆-alkoxy;
Y is methoxy, amino, methylamino or dimethylamino;
Z is CHCH₃, CHOCH₃ or NOCH₃;
R is hydrogen or C₁-C₆-alkyl;
A is hydrogen, C₁-C₆-alkyl, C₁-C₆-haloalkyl or C₃-C₇-cycloalkyl;
B is unsubstituted or substituted C₁-C₆-alkyl, C₂-C₆-alkenyl, C₂-C₆-alkynyl, C₃-C₇-cycloalkyl, C₅-C₇-cycloalkenyl,
unsubstituted or substituted, saturated or partially unsaturated heterocyclyl,
unsubstituted or substituted aryl, which may be fused,
unsubstituted or substituted 5- or 6-membered heteroaryl, which may be benzo- or hetero-fused,
unsubstituted or substituted aryl-C₁-C₆-alkyl or heteroaryl-C₁-C₆-alkyl, or
C(=O)W;
where
W is hydrogen,
unsubstituted or substituted C₁-C₆-alkyl,
C₁-C₆-alkoxy, C₁-C₆-alkylthio, C₁-C₆-alkylamino or di-C₁-C₆-alkylamino,
unsubstituted or substituted aryl, heteroaryl, aryloxy, arylthio, arylamino, aryl-N-(C₁-C₆-alkyl)amino, heteroaryloxy, heteroarylthio, heteroarylamino or heteroaryl-N-(C₁-C₆-alkyl)amino;
or
A and B, together with the C atom whose substituents they are, are an unsubstituted or substituted, saturated or unsaturated 3- to 7-membered isocyclic ring or 5- to 7-membered heterocyclic ring, which may be fused and which may also be quinonoid.

2. A process for preparing compounds of the general formula I as claimed in claim 1, which comprises reacting a carbonyl compound of the general formula II 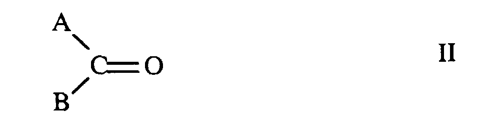 with a carbonyl compound of the general formula **III** 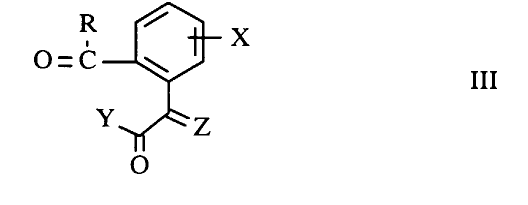 and with hydrazine, one of its acid addition salts or its hydrate.

3. A process for preparing compounds of the general formula **I** as claimed in claim 1, wherein a carbonyl compound of the general formula **III** as in claim 2 is reacted with a hydrazone **IV** 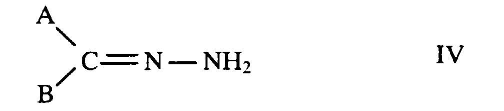 or one of its acid addition salts.

4. A composition suitable for the control of pests or harmful fungi, containing a solid or liquid carrier and a compound of the general formula I as claimed in claim 1.

5. The use of the compounds I as claimed in claim 1 for preparing a composition suitable for the control of pests or harmful fungi.

6. A method for controlling harmful fungi, which comprises treating the fungi or the materials, plants, soil or seeds to be protected from fungal attack with an effective amount of a compound of the general formula I as claimed in claim 1.

7. A method for controlling pests, which comprises treating the pests or the materials, plants, soil or seeds to be protected from them with an effective amount of a compound of the general formula I as claimed in claim 1.

## Revendications

1. Composé de formule I 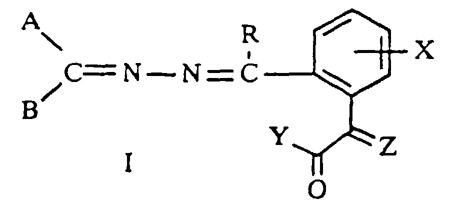 dans laquelle les symboles ont les significations suivantes :
X : l'hydrogène, un groupe cyano, nitro, trifluorométhyle, un halogène, un groupe alkyle en C1-C6 ou alcoxy en C1-C6 ;
Y : un groupe méthoxy, amino, méthylamino ou diméthylamino ;
Z : un groupe CHCH₃, CHOCH₃ ou NOCH₃ ;
R : l'hydrogène ou un groupe alkyle en CI-C6 ;
A : l'hydrogène, un groupe alkyle en C1-C6, halogénoalkyle en C1-C6 ou cycloalkyle en C3-C6 ;
B : un groupe alkyle en C1-C6, alcényle en C2-C6, alcynyle en C2-C6, cycloalkyle en C3-C7, cycloalcényle en C5-C7 éventuellement substitué ;
un groupe hétérocyclyle saturé ou partiellement insaturé et éventuellement substitué ;
un groupe aryle éventuellement substitué et éventuellement condensé ;
un groupe hétéroaryle à cinq ou six chaînons, éventuellement substitué et éventuellement condensé sur un cycle benzénique ou sur sur un hétérocycle ;
un groupe aryl-alkyle en C1-C6 ou hétéroaryl-alkyle en C1-C6 éventuellement substitué, ou C(=O)W, dans lequel
W représente
l'hydrogène,
un groupe alkyle en C1-C6 éventuellement substitué,
un groupe alcoxy en C1-C6, alkylthio en C1-C6, alkylamino en C1-C6, ou di-(alkyle en C1-C6)amino,
un groupe aryle éventuellement substitué, hétéroaryle, arylthio, arylamino, aryl-N-(alkyle en C1-C6)amino, hétéroaryloxy, hétéroarylthio, hétéroarylamino ou hétéroaryl-N-(alkyle en C1-C6)amino ;
ou bien
A et B représentent ensemble et avec l'atome de carbone dont ils sont substituants
un noyau isocyclique de trois à sept chaînons ou hétérocyclique de cinq à sept chaînons, saturé ou instauré, éventuellement condensé, et qui peut également être quinoïde.

2. Procédé de préparation des composés de formule générale I selon revendication 1, caractérisé par le fait que l'on fait réagir un dérivé carbonylé de formule générale II 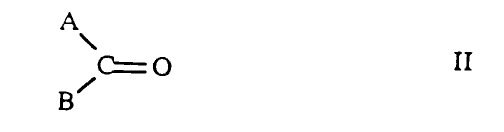 avec un dérivé carbonylé de formule générale III 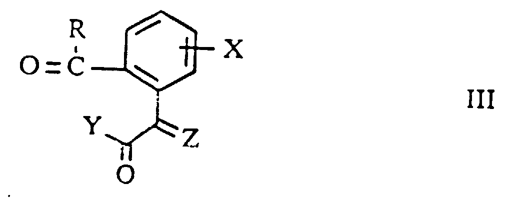 et avec l'hydrazine, l'un de sels formés par addition avec un acide ou son hydrate.

3. Procédé de préparation des composés de formule générale I selon la revendication 1, caractérisé par le fait que l'on fait réagir un dérivé carbonylé de formule générale III selon la revendication 2 avec une hydrazone IV 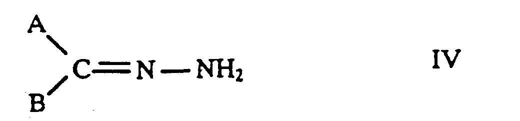 ou avec un de ses sels formés par addition avec un acide.

4. Produit approprié à la lutte contre les parasites ou les mycètes nuisibles, qui contient un véhicule solide ou liquide et un composé de formule générale I selon revendication 1.

5. Utilisation des composés I selon revendication 1 pour la préparation d'un produit apte à la lutte contre les parasites ou contre les mycètes nuisibles.

6. Procédé pour combattre les mycètes nuisibles, caractérisé par le fait que l'on traite les mycètes ou les matières, végétaux, sols ou semences à protéger contre les mycètes, par une quantité efficace d'un composé de formule générale I selon revendication 1.

7. Procédé pour combattre les parasites, caractérisé par le fait que l'on traite les parasites ou les matières, végétaux, sols ou semences à protéger contre les parasites, par une quantité efficace d'un composé de formule générale I selon revendication 1.
